# EUROPEAN PATENT SPECIFICATION

(11) **EP 3 320 914 B1**
(45) Date of publication and mention of the grant of the patent: **30.12.2020**
(21) Application number: 17209271.0
(22) Date of filing: 10.09.2013
(51) Int. Cl.: A61K 39/00, A61K 38/21, A61K 35/17, A61K 45/06, C12N 5/00, C07K 16/28, C12N 15/117, A61P 25/28, C12N 5/0783

(54) **T-HELPER 1 ADJUVANT FOR TREATING AMYOTROPHIC LATERAL SCLEROSIS**
TH1-TYP ADJUVANS ZUR BEHANDLUNG VON AMYOTROPHER LATERALSKLEROSE
ADJUVANT T-HELPER 1 POUR LE TRAITEMENT DE LA SCLÉROSE LATÉRALE AMYOTROPHIQUE

(30) Priority: 10.09.2012 US 201261699071 P; 13.03.2013 US 201361779440 P
(43) Date of publication of application: 16.05.2018
(62) Document number(s) of the earlier application(s) in accordance with Art. 76 EPC: 13836112.6 / 2 892 353
(73) Proprietor: Yeda Research And Development Co. Ltd. At The Weizmann Institute Of Science, 7610002 Rehovot (IL)
(72) Inventor: EISENBACH-SCHWARTZ, Michal, 7610002 Rehovot (IL); FRIEDMAN, Nir, 7610002 Rehovot (IL); BARUCH, Kuti, 7610002 Rehovot (IL); KUNIS, Gilad, 7610002 Rehovot (IL); CAHALON, Liora, 7610002 Rehovot (IL); ROSENZWEIG, Neta, 7610002 Rehovot (IL); DECZKOWSKA, Aleksandra, 7610002 Rehovot (IL)
(74) Representative: Lavoix

(56) References cited:
- WO-A1-2006/080596
- WO-A2-2006/109195
- US-A1- 2009 010 873
- BUTCHI NIRANJAN B ET AL: "TLR7 and TLR9 Trigger Distinct Neuroinflammatory Responses in the CNS", AMERICAN JOURNAL OF PATHOLOGY, vol. 179, no. 2, 17 June 2011 (2011-06-17) , pages 783-794, XP002757119,
- MURUGESAN NIVETHA ET AL: "Active induction of experimental autoimmune encephalomyelitis by MOG35-55 peptide immunization is associated with differential responses in separate compartments of the choroid plexus.", FLUIDS AND BARRIERS OF THE CNS 2012, vol. 9, no. 1, 7 September 2012 (2012-09-07), page 15, XP021123091, ISSN: 2045-8118
- RON-HAREL N ET AL: "Immune senescence and brain aging: can rejuvenation of immunity reverse memory loss?", TRENDS IN NEUROSCIENCES 2009 ELSEVIER LTD GBR, vol. 32, no. 7, July 2009 (2009-07), pages 367-375, XP002779071, ISSN: 0166-2236

## Description

### FIELD OF THE INVENTION

The present invention relates in general to methods of treating disease, disorder, condition or injury of the Central Nervous System (CNS) by activation of the choroid plexus.

### BACKGROUND OF THE INVENTION

Existing methods for treatment of CNS disease, such as multiples sclerosis or Alzheimer's disease, are in general unsatisfactory, basically due to the incomplete understanding of the inherent mechanisms regulating maintenance and repair of the CNS.

The brain is generally viewed as an "organ behind walls", shielded by barriers from the peripheral immune system. Nevertheless, circulating immune cells have been repeatedly shown to be essential for central nervous system (CNS) maintenance (Moalem G, et al. (1999); Wolf SA, et al. (2009); Ziv Y, et al. (2006)). Specifically, T cells that recognize CNS antigens were reported to contribute to the functional integrity of the CNS under both normal and pathological conditions (Moalem G, et al. (1999); Ziv Y, et al. (2006); Olsson T et al. (2003)). Such CNS-specific T cells, or cytokines derived from them, were shown to support several aspects of adult brain plasticity, including hippocampus-dependent learning and memory, adult neurogenesis, and neurotrophic factor production (Ziv Y, et al. (2006)). Interestingly, T cells are rarely found in the healthy CNS parenchyma, raising several key questions as to how, where and when these cells exert their effects on the healthy CNS.

Under physiological conditions, T cells are mainly found at the borders of the CNS - the choroid plexus (choroid plexus) of the brain's ventricles, comprising the blood-cerebrospinal- fluid barrier (BCSFB), the meningeal spaces and the cerebrospinal fluid (CSF) (Engelhardt B & Ransohoff RM (2005)). Strategically positioned at the lining between the CNS and the immune system, the choroid plexus, in addition to its role in generating the CSF, can enable bi-directional communication between the CNS parenchyma and the blood circulation (Emerich DF et al. (2005)) needed for brain maintenance and repair.

This picture of the mechanisms governing the maintenance and repair of the CNS is still incomplete and therefore the existing means for treating diseases of the brain leave a considerable need for improvement.

### SUMMARY OF INVENTION

In one aspect, the present invention provides a vaccine for use in the treatment of ALS comprising a non-encephalitogenic Th1 adjuvant as a sole active ingredient, wherein the vaccine is to be administered according to a regimen to thereby confer reduction and maintenance of the level of immunosuppression in the circulation of said mammal relative to a reference and induction towards a Th1-type immune response, wherein said reduced level of immunosuppression and Th1-type immune response in the circulation indicates and ensures activation of the choroid plexus of said mammal and maintenance of said activation thus allowing either anti-inflammatory immune cells or immune cells which acquire a healing phenotype at the cerebrospinal fluid to pass through the choroid plexus and accumulate at a site of damage in the CNS caused by said ALS, and the non-encephalitogenic Th1 adjuvant is selected from an agonist of TLR3, 4, 5, 7, 8 and 9.

In another aspect the present invention provides a vaccine for use in the treatment of ALS comprising a non-encephalitogenic Th1 adjuvant as a sole active ingredient, wherein the vaccine is to be administered according to a regimen to thereby confer reduction and maintenance of the level of immunosuppression in the circulation of said mammal relative to a reference and induction towards a Th1-type immune response, wherein said reduced level of immunosuppression and Th1-type immune response in the circulation indicates and ensures activation of the choroid plexus of said mammal and maintenance of said activation thus allowing either anti-inflammatory immune cells or immune cells which acquire a healing phenotype at the cerebrospinal fluid to pass through the choroid plexus and accumulate at a site of damage in the CNS caused by said ALS, and the non-encephalitogenic Th1 adjuvant is selected from an agonist of TLR3, 4, 5, 7, 8 and 9.

### BRIEF DESCRIPTION OF DRAWINGS

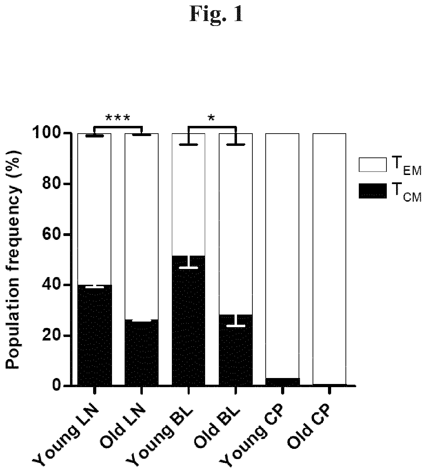

**Fig. 1** shows that the choroid plexus is populated by effector memory CD4+ T cells. Quantitative analysis of FACS plots for Young (3 months) and Old (22 months) mouse effector and central memory CD4⁺ T cell frequencies in inguinal lymph nodes (LN), blood (BL) and choroid plexus (CP) tissues. Data shown are representative of two to three independent experiments. Bar graphs throughout the figure show mean ± SE of each group (*n* = 4-5 mice per group; **P* < 0.05; ****P* < 0.001; Student's *t* test) T_{EM} - effector memory T cells (white bars); T_{CM} - central memory T cells (black bars).

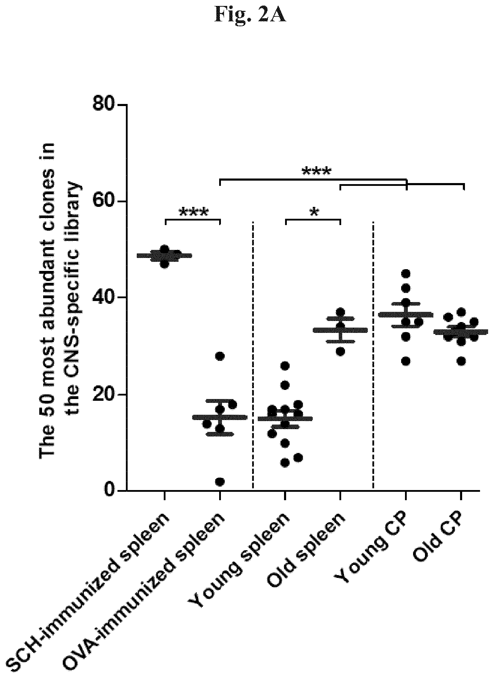

**Figs. 2A-B** show that the choroid plexus CD4⁺ TCR repertoire is enriched with CNS-specific clones. (A) A library of CNS specific clones was compiled from TCRβ amino acid sequences measured from all spleen SCH-immunized mice. The library contains clones that were significantly expanded compared with the group of naive (young) mouse spleen. The 50 most abundant clones from each tissue (SCH-immunized spleen, OVA-immunized spleen, young spleen, old spleen, young CP and old CP) were examined for their presence in this library, lines represent average ± SEM (n = 3-12 mice per group; **P* < 0.05; ****P<* 0.001; One-way ANOVA, followed by Newman-Keuls *post hoc* analysis). **(B)** Quantitative comparison between the fractions of clones present in the CNS-specific library using different cutoffs (TOP10-TOP150) for the most abundant clones of each tissue (SCH-immunized spleen (filled circles), OVA-immunized spleen (empty circles), naive spleen (crossed circles), young CP (filled squares), old CP (empty squares) and SCH immunized CP (half filled squares)) (*P* < 0.05; Two-way ANOVA).

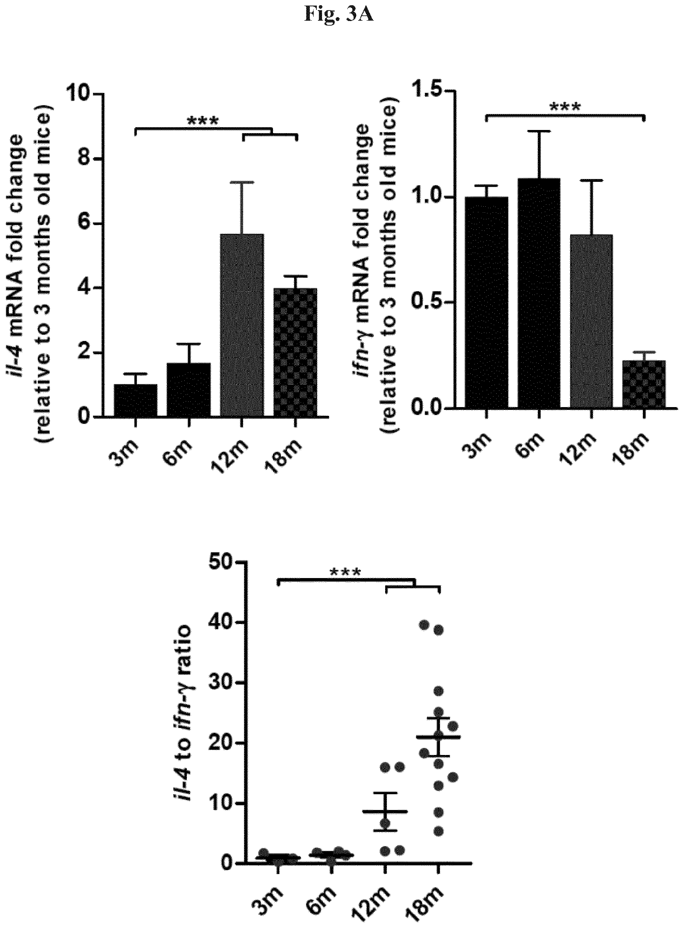

**Figs. 3A-I**. show Th2-mediated inflammation of the CP epithelium during the aging process. **A**) Changes in mRNA transcript levels, measured by real-time qPCR, of *il-4* (left panel), *ifn-γ* (middle panel) and their ratio (right panel) in the CP of mice at the ages of 3, 6, 12 and 18 months relative to 3 months old mice (*n* = 8-10 mice per group; ****P* < 0.001; one-way ANOVA, followed by Newman-Keuls *post hoc* analysis). **(B)** *ccl11* mRNA levels in the CP of young (3 months) and old (20 months) mice *(n* = 5-6 mice per group; **P* < 0.05; Student's *t* test). (C) CCL11 protein levels (pg/mg tissue) in the CP of young (3 months) and old (20-22 months) mice *(n* = 16 mice per group; **P* < 0.05; Student's *t* test) **(D)** *ccl11* mRNA levels 24 hours after the addition of IL-4 (untreated, 0.12ng/ml, 0.37ng/ml, 1.1ng/ml, 3.3ng/ml, 10ng/ml, 30ng/ml), of cultured CP epithelial cells (*n* = 3 per group; one-way ANOVA, Newman-Keuls *post hoc;* one representative experiment shown out of three independently performed). **(E)** Changes in *ccl11* mRNA levels, measured by real-time qPCR, of cultured CP epithelial cells from young (3 months) mice untreated, or 24 hours after the addition of the cytokines IL-4 (10ng/ml), IFN-γ (100ng/ml), or their combination (*n* = 8 mice per group; ****P* < 0.001; one-way ANOVA, followed by Newman-Keuls *post hoc* analysis; one representative experiment shown out of three independently performed). **(F)** Changes in *ccl11* mRNA levels, measured by real-time qPCR, of cultured CP epithelial cells from young (3 months) untreated, and old (22 months) mice untreated, and 24 hours after the addition of the cytokines IL-4 (10ng/ml) or IFN-γ (100ng/ml) (*n* = 8 mice per group; **P* < 0.05; one-way ANOVA, followed by Newman-Keuls *post hoc* analysis). **(G)** *bdnf* mRNA levels 24 hours after the addition of IL-4 (untreated, 0.12ng/ml, 0.37ng/ml, 1.1ng/ml, 3.3ng/ml, 10ng/ml, 30ng/ml), measured by qPCR, of cultured CP epithelial cells (*n* = 3 per group; one-way ANOVA, Newman-Keuls *post hoc;* one representative experiment shown out of three independently performed). **(H)** Changes in *arg1* mRNA levels, measured by real-time qPCR, in the CP of young (3 months) and old (22 months) mice (*n* = 5-6 mice per group; ****P* < 0.001; Student's *t* test). **(I)** Nissl stainings were used to measure average cross-sectional area of epithelial cells in µm² (****P* < 0.001; Student's *t* test), and to assess epithelial hypertrophy in young and old mice.

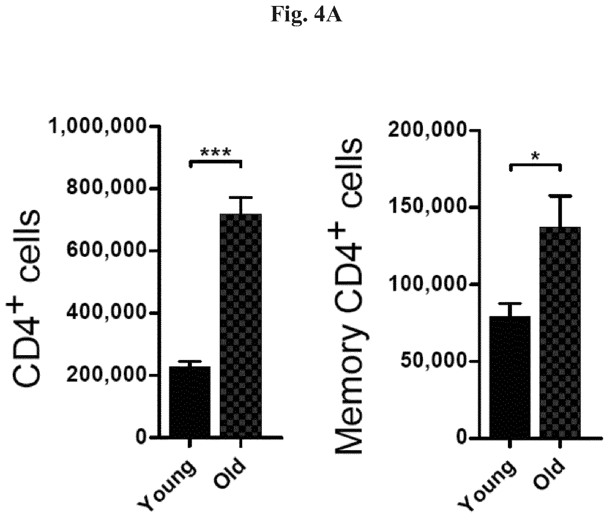

**Figs. 4A-G** depict syngeneic homeostatic-driven proliferation effects on the CP and hippocampus. **(A)** A histogram quantifying FACS histograms and quantitative analysis for CD4⁺ T cells (left) and CD4⁺/CD44^{high} memory T cells (right) in the bone marrow of young (3 months) and old (22 months) mice. Cells were pre-gated by CD45⁺ and TCRβ (*n* = 4-5 mice per group; **P* < 0.05; ****P* < 0.001; Student's *t* test). **(B)** The 100 most abundant TCRβ clones from the SCH-immunized spleen, naive spleen and bone marrow of young (3 months) and old (22 months) mice were examined for their presence in the CNS-specific clonal library (*n* = 8 mice per group; **P* < 0.05; ****P* < 0.001; one-way ANOVA, followed by Newman-Keuls *post hoc* analysis). **(C)** Representative flow cytometry plots demonstrating resting (Carboxyfluorescein succinimidyl ester(CFSE)^{high}) and dividing (CFSE^{low}) T cells (TCRβ⁺) in the donor bone marrow (BM) on the day of transplantation (d0), in the spleen of young recipients on days 3 and 5 post transplantation (top), and in the spleen and cervical lymph nodes of the aged recipients on day 5 post transplantation (bottom). **(D)** Morris Water Maze (MWM) test of young (3 months old), untreated old (22 months old), and aged littermates after homeostatic-driven proliferation (HDP) achieved by their irradiation and syngeneic bone marrow transplantation at the age of 18 months ("Old-HDP", 22 months old). Mice were divided into intact or impaired memory groups according to their MWM score, where an average latency time of above 60 seconds on the fourth day of acquisition was classified as "impaired memory" (white bars), and a latency of less than 60 seconds was defined as "intact memory" (black bars) (**P* < 0.05; ****P* < 0.001; χ² test; *n* = 10-14 mice per group). **(E)** Changes in *il-4* and *ifn*-*γ* mRNA levels and their ratio, measured by real-time qPCR, in the CP of young (3 months), old (22 months), and old-HDP (22 months) mice (**P* < 0.05; ***P* < 0.01; ****P* < 0.001; One-way ANOVA, followed by Newman-Keuls *post hoc* analysis). **(F)** Correlation analysis between the CP mRNA levels of *il-4*/*ifn-γ* ratio and *ccl11* (*P <* 0.05; Pearson r²=0.2294; *n* = 17 mice). **(G)** Changes in *bdnfl, ho1* and *pgc1 α* mRNA levels, measured by real-time qPCR, in the hippocampus of young (3 months), old (22 months), and old-HDP (22 months) mice (**P* < 0.05; ***P* < 0.01; ****P* < 0.001; One-way ANOVA, followed by Newman-Keuls *post hoc* analysis).

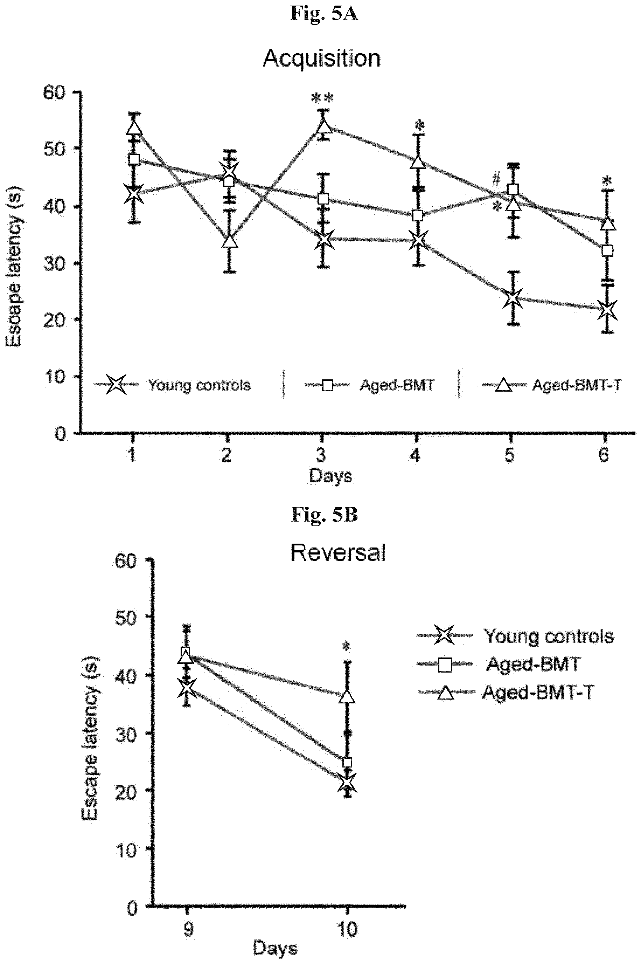

**Figs. 5A-C** shows that cognitive ability induced by homeostatic-driven proliferation of pseudo-autologous BM is T-cell dependent. Aged mice with memory impairments were transplanted with either complete BM, derived from donor mice of identical age to the recipients, or with T cell depleted BM, from the same donors. The mice were tested in the Morris Water Maze (MWM) 8 weeks following transplantation, together with non-treated young mice of the same genetic background. (A) Acquisition phase, escape latency in seconds is presented (repeated measure ANOVA: groups: *P* = 0.005, days: *P* = 0.0005, groups x days: *P* = 0.02). **(B)** Reversal phase, escape latency in seconds is presented (analysis of covariance, comparing groups regarding the differences between performance of each mouse in the 2 days of the test (day2-day1), with the day 1 measure as the covariant: *P* = 0.0002, **P* < 0.05 (young vs. aged-BMT-T)). **(C)** Reversal phase, path length to platform in centimeters (cm) is presented (performance in the reversal phase according to swimming path length to the platform (*P* < 0.0001, *#P* < 0.05 (aged-BMT-T vs. aged-BMT), **P* < 0.05 (aged-BMT-T vs. young). Crosses: young mice (control); empty squares: aged mice transplanted with whole BM (aged-BMT); empty triangles - aged mice transplanted with aged-BM depleted of T-cells (aged-BMT-T).

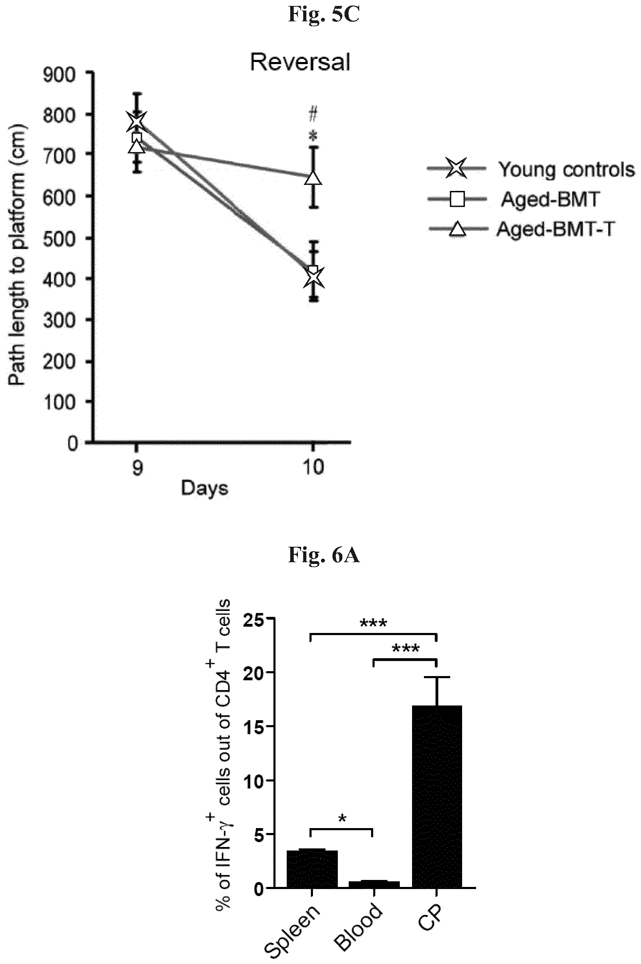

**Figs. 6A-C** show characterization of the naive CP for its resident T-cell populations. (A) CD4⁺ T cells from spleen, blood or CP of WT mice were activated with PMA and ionomycin for 6 h, treated with Brefeldin-A for the last 4 h, and then stained for intracellular IFN-γ and analyzed by flow cytometry. **(B)** CD4⁺ T cells from blood, spleen or CP that express IL-4 were quantified using the IL-4-IRES-eGFP reporter mouse model. (C) CD4⁺ Tregs from spleen, blood or CP were quantified by intracellular staining for the transcription factor FoxP3. Mean frequencies ± SEM are indicated at the gated regions. Bar graphs represent mean ± SEM; *P < 0.05; ***P < 0.001; One-way ANOVA, followed by Student's t-test post hoc analysis (n=4-8 per group).

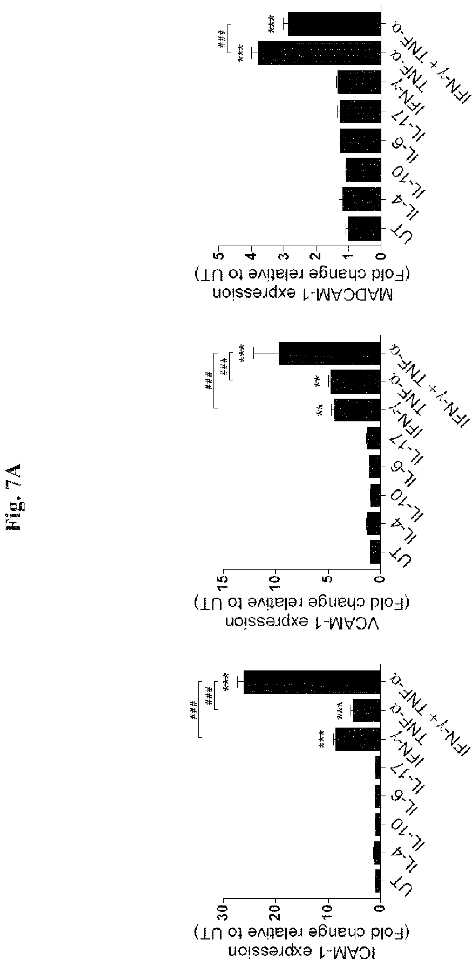

**Figs. 7A-H** show that choroid plexus epithelial cells upregulate trafficking molecules in response to a specific cytokine milieu. **(A-E)** mRNA levels of trafficking molecules measured by real time qPCR, of cultured CP cells from WT mice 24 h after the addition of the cytokines IL-4 (10 ng/ml), IL-10 (10 ng/ml), IL-6 (10 ng/ml), IL-17 (100 ng/ml), IFN-γ (100 ng/ml), TNF-α (100 ng/ml) or the combination of IFN-γ and TNF-α, relative to un-treated (UT) cells. Bars represent mean ± SEM; *P < 0.05; ** P<0.01; ***P < 0.001 vs. UT cells; ^{#}P < 0.05; ^{##} P<0.01; ^{###}P < 0.001 between indicated groups by one-way ANOVA followed by Tukey's HSD post hoc analysis (n=3 per group). Results of one of three independently performed experiments are presented. Trafficking molecules were ICAM-1, VCAM-1 and MADCAM-1 **(A),** CCL5, CXCL9, CXCL10 and MHC-II **(B),** CCL2, Fractalkine and M-CSF (C), Arg1 (**D**) and CCL20 **(E). (F-H)** Quantitative analysis of the intensity (corrected total cell fluorescence (CTCF)) of staining per cell in arbitrary units in cultured CP cells from WT mice 3 days after the addition of IFN-γ (100 ng/ml) immunostained for the epithelial marker, cytokeratin, and for ICAM-1 **(F)** or VCAM-1 **(G).** IL-4 (10 ng/ml) treated cells were stained for Arg1 **(H).** Data are expressed as mean ± SEM; *P < 0.05; ***P < 0.001; Student's t-test (n=3 per group).

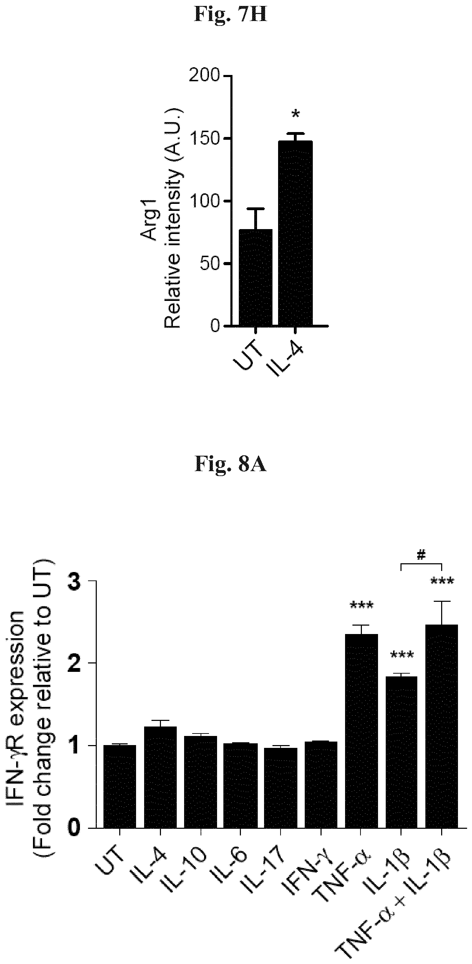

**Figs. 8A-C** show that TNF-α, and INF-γ reciprocally control the expression of their receptors by the CP. **(A)** Cultured CP cells were treated with either IL-4 (10 ng/ml), IL-10 (10 ng/ml), IL-6 (10 ng/ml), IL-17 (100 ng/ml), IFN-γ (100 n-g/ml), TNF-α, (100 ng/ml), IL-1β (100 ng/ml) or their combination for 24 h, and mRNA levels of IFN-γR were measured by real time qPCR. Bars represent mean ± SEM; *P < 0.05, ***P < 0.001 vs. un-treated (UT) cells; ^{#}P < 0.05 between indicated groups by one-way ANOVA followed by Tukey's HSD post hoc analysis (n=3 per group). **(B)** Cultured epithelial cells were treated with neutralizing antibodies against either TNF-R1 or TNF-R2, 1 h prior to the addition of TNF-α, (100 ng/ml) for 24 h, and mRNA levels of IFN-γR were measured by real time qPCR. Bars represent mean ± SEM; *P < 0.05, ***P < 0.001 vs. un-treated cells; ^{###}P < 0.001 between indicated groups by one-way ANOVA followed by Tukey's HSD post hoc analysis (n=3 per group). **(C)** mRNA levels of TNF-R1 were measured by real time qPCR in cultured CP cells from WT mice 24 h after the addition of the cytokines IL-4 (10 ng/ml), IL-10 (10 ng/ml), IL-6 (10 ng/ml), IL-17 (100 ng/ml), IFN-γ (100 ng/ml), TNF-α, (100 ng/ml) or the combination of IFN-γ and TNF-α, and were compared to untreated (UT) cells. Bars represent mean ± SEM; ** P<0.01; ***P < 0.001 vs. UT cells; ^{##} P<0.01 between indicated groups by one-way ANOVA followed by Tukey's HSD post hoc analysis (n=3 per group). One representative experiment is presented out of three independent repetitions.

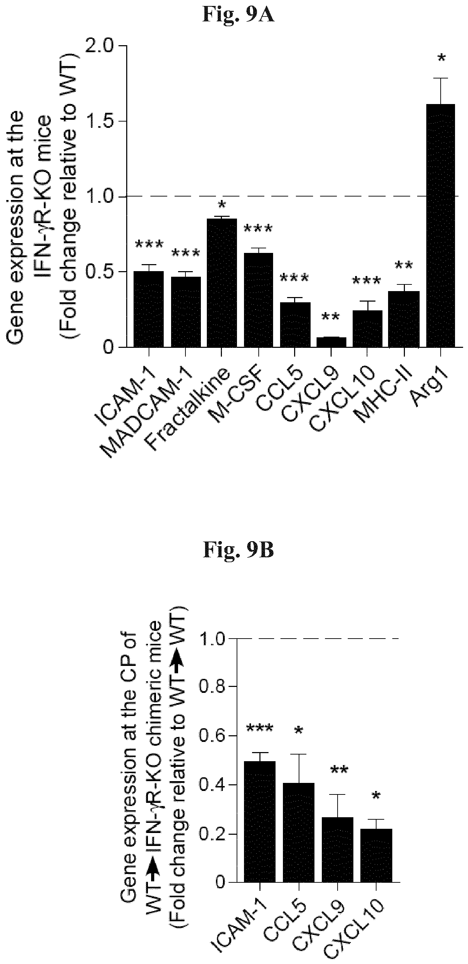

**Figs. 9A-D** show that IFN-γ signaling is needed to maintain immune surveillance of the healthy CNS. **(A)** mRNA levels of various adhesion molecules, chemokines and immunoregulatory molecules (ICAM-1, MADCAM-1, Fractalkine, M-CSF, CCL5, CXCL9, CXCL10, MHC-II and Arg1) measured by real time qPCR in the CP of IFN-γR-KO mice. Bars represent mean ± SEM; *P < 0.05; **P < 0.01; ***P < 0.001 vs. WT; Student's t-test (n=5-6 per group). **(B)** Chimeric IFN-γR-KO or WT mice were reconstituted with WT BM cells. mRNA levels of ICAM-1, CCL5, CXCL9 and CXCL10 were measured by real time qPCR in their CP. Bars represent mean ± SEM; *P < 0.05; **P < 0.01; ***P < 0.001; Student's t-test (n=5 per group). **(C)** Chimeric WT mice were reconstituted with either WT or IFN-γ-KO BM cells. mRNA levels of ICAM-1, CCL5, CXCL9 and CXCL10 were measured by real time qPCR in their CP. Bars represent mean ± SEM; *P < 0.05; **P < 0.01; one-way ANOVA, followed by Student's t-test post hoc analysis (n=5 per group). **(D)** The number of CD4⁺ T cells in the spleen (upper left panel), blood (upper right panel), CP (lower left panel) and CSF (lower right panel) of WT and IFN-γR-KO mice was quantified using flow cytometry. Bars represent mean ± SEM; *P < 0.05 by Student's t-test (n=5-7 per group).

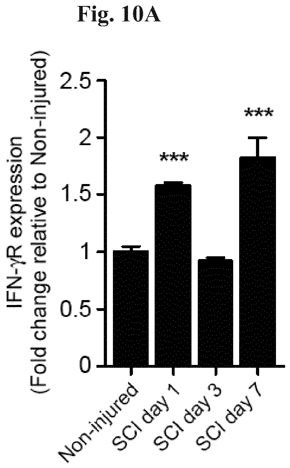

**Figs. 10A-G** show that IFN-γR-KO mice have impaired recovery following spinal cord injury (SCI), which is associated with failure of CP activation for leukocyte trafficking. **(A)** WT mice were subjected to SCI and mRNA levels of IFN-γR in their CP were measured by real time qPCR on days 1, 3 and 7 post-injury. Bars represent mean ± SEM, ***P < 0.001 vs. non-injured; one-way ANOVA, followed by Student's t-test post hoc analysis (n=4-6 per group). **(B)** IFN-γR-KO and WT mice were similarly injured and mRNA levels of ICAM-1, CXCL9 and CXCL10 in their CP were measured by real time qPCR at day 7 post-injury. Bars represent mean + SEM; *P < 0.05; **P < 0.01; ***P < 0.001 vs. WT mice; ^{#}P < 0.05; ^{##} P<0.01; ^{###}P < 0.001 between indicated groups by one-way ANOVA followed by Student's t-test post hoc analysis (n=5-12 per group). (C) IFN-γR-KO (circles) and WT (diamonds) mice were subjected to a well calibrated SCI, and were followed for hind-limb locomotor activity assessed according to the Basso Mouse Scale (BMS). Bars represent mean ± SEM; *P < 0.05; **P < 0.01; repeated measures ANOVA, followed by Student's t-test post hoc analysis (n=11-12 per group). **(D)** mRNA levels of TNF-α, IL-1β and IL-6 at the site of injury were measured by real time qPCR 24 hours after the injury in IFN-γR-KO and WT mice. Bars represent mean ± SEM; **P < 0.01; ***P < 0.001 vs. WT mice by one-way ANOVA, followed by Student's t-test post hoc analysis (n=6 per group). **(E)** The numbers of CD4⁺ T cells (left panel) and monocytes (CD11b⁺)(right panel) in the CSF of WT and IFN-γR-KO mice were quantified using flow cytometry at 1 day post SCI, and were compared to non-injured WT and IFN-γR-KO mice. Bars represent mean ± SEM; *P < 0.05; **P < 0.01; ***P < 0.001 vs. WT mice; ^{##} P<0.01; ^{###}P < 0.001 between indicated groups by one-way ANOVA, followed by Student's t-test post hoc analysis (n=6-8 per group). **(F)** Quantitative analysis of the number of T cells at the epicenter of the site of injury as measured by immunohistochemistry, in WT and IFN-γR-KO mice 7 days post injury showing significant reduction in the numbers of T cells in the IFN-γR-KO mice. Bars represent mean ± SEM; **P < 0.01 by Student's t-test (n=5-6 "per group). **(G)** The numbers of CD4⁺ T cells and monocytes (CD11b⁺/CD45.2^{high}) in the spinal cord of WT and IFN-γR-KO mice were quantified using flow cytometry at day 7 post SCI. Bars represent mean + SEM; *P < 0.05; ***P < 0.001 by Student's t-test (n=6-7 per group).

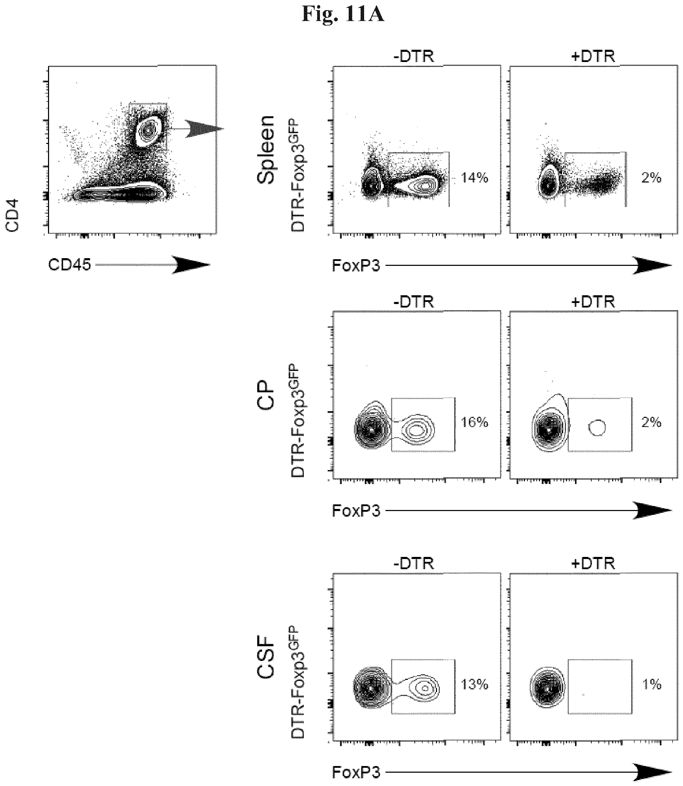

**Figs. 11A-B** show that Foxp3⁺ Tregs depletion activates the CP for trafficking. **(A)** Foxp3-DTR^{-/-} and Foxp3-DTR^{-/+} were injected daily with diphtheria toxin (DTX) for 4 constitutive days. On the fifth day mice were perfused with phosphate buffered saline (PBS) and their spleen, CP and CSF were analyzed by flow cytometry for their cellular composition. (n=6 per group). **(B)** Following the same depletion protocol, mRNA expression levels of selected genes (from upper left: ICAM1, CXCL9, CXCL10, CXCL11, ARG1, MCP1, MCSF, IGF1, IGF2, BDNF-5) was examined in the CP of both Foxp3-DTR^{-/-} and Foxp3-DTR^{-/+} mice.

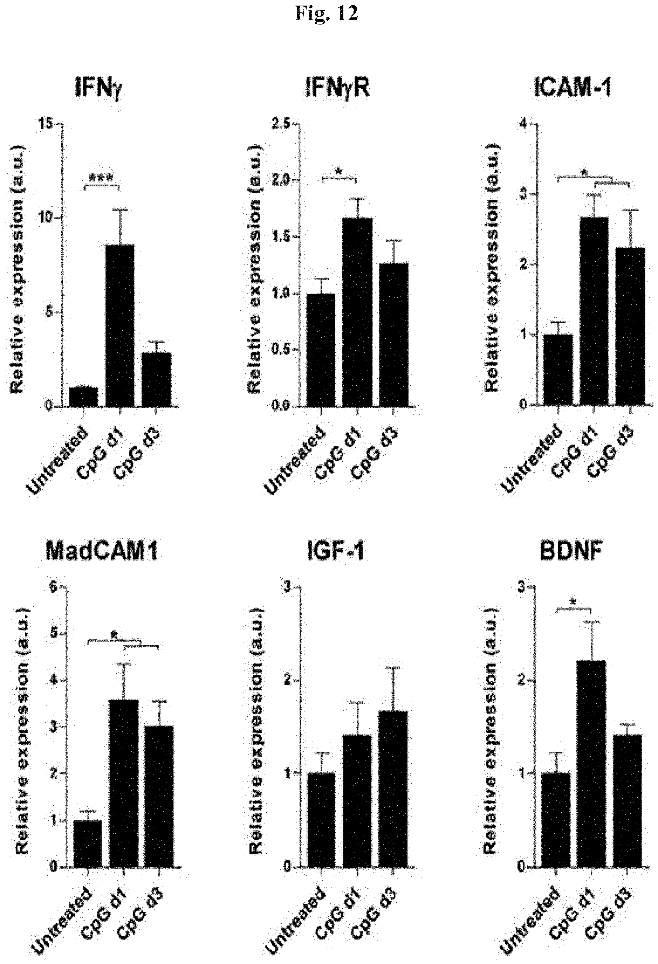

**Fig. 12** shows that peripheral immunomodulation by CpG affects the choroid plexus epithelium. CpG (20ug) was injected i.p, and choroid plexus tissues were isolated one (d1) or three (d3) days after the injection. Changes in mRNA transcript levels, by real-time qPCR, of IFN-γ, IFN-γ receptor, ICAM-1, MadCAM-1, IGF-1 and BDNF (from upper left) in the CP of C57BL6/J mice (n = 6 mice per group; ****P* < 0.001, **P* < 0.05; one-way ANOVA, followed by Newman-Keuls *post hoc* analysis) are shown.

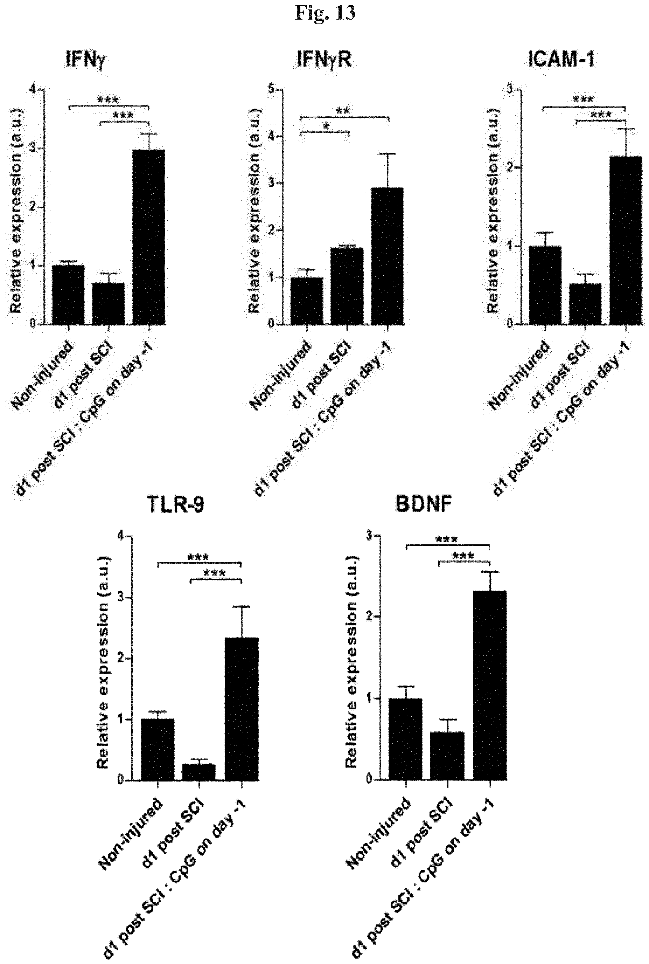

**Fig. 13** shows that preconditioning by CpG affects the choroid plexus epithelium day 1 post spinal cord injury. CpG (20ug) was injected i.p. 1 day prior to spinal cord injury (day -1). Changes in mRNA transcript levels, measured by real-time qPCR, are shown for IFN-γ, IFN-γ receptor, ICAM-1, TLR-9 and BDNF (from left) in the CP of C57BL6/J mice (n = 6 mice per group; ****P* < 0.001; ***P* < 0.01; **P* < 0.05; one-way ANOVA, followed by Newman-Keuls *post hoc* analysis).

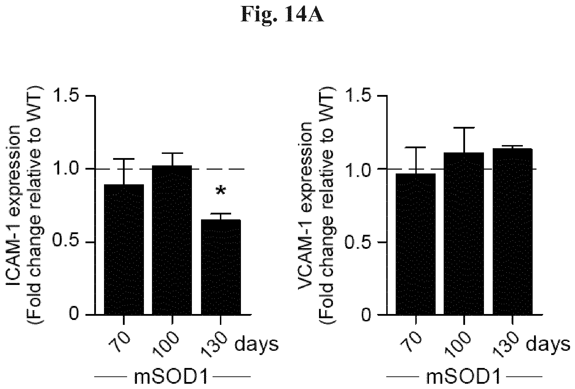

**Figs. 14A-D** show that the choroid plexus of mSOD1 mice is not spontaneously activated to enable leukocyte trafficking. **(A)** mRNA levels of the intercellular adhesion molecule 1 (ICAM-1) and vascular cell adhesion molecule 1 (VCAM-1) in the choroid plexus (CP) of mSOD1 mice were measured by quantitative PCT (qPCR) at age 70, 100 and 130 days (n=6-7 per group; bars represent mean ± s.e.m. *p< 0.05, vs. WT littermates by Student's t-test) and presented as fold change relative to wild-type (WT). **(B)** mRNA levels of the chemokines CCL2, M-CSF and Fractalkine in the CP of mSOD1 mice, measured by qPCR at age 70, 100 and 130 days (n=5-7 per group) (bars represent mean ± s.e.m.; *p< 0.05, vs. WT littermates by Student's t-test), and presented as fold change relative to WT. **(C)** Protein levels of the chemokines CCL2 and M-CSF in the CP of WT and mSOD1 mice in pg/mg protein, measured by Multiplex ELISA at age 70, 100 and 130 days (n=2-4 per group; bars represent mean ± s.e.m.; *p< 0.05, vs. WT by ANOVA, followed by Student's t-test post hoc analysis). **(D)** Protein levels of the inflammatory cytokines TNF-α, IL-1β and IL-6 in the spinal cord of WT and mSOD1 mice in pg/mg protein were measured by Multiplex ELISA at age 70, 100 and 130 days (n=2 per group; bars represent mean ± s.e.m.; *p< 0.05, **p< 0.01, vs. WT by ANOVA, followed by Student's t-test post hoc analysis).

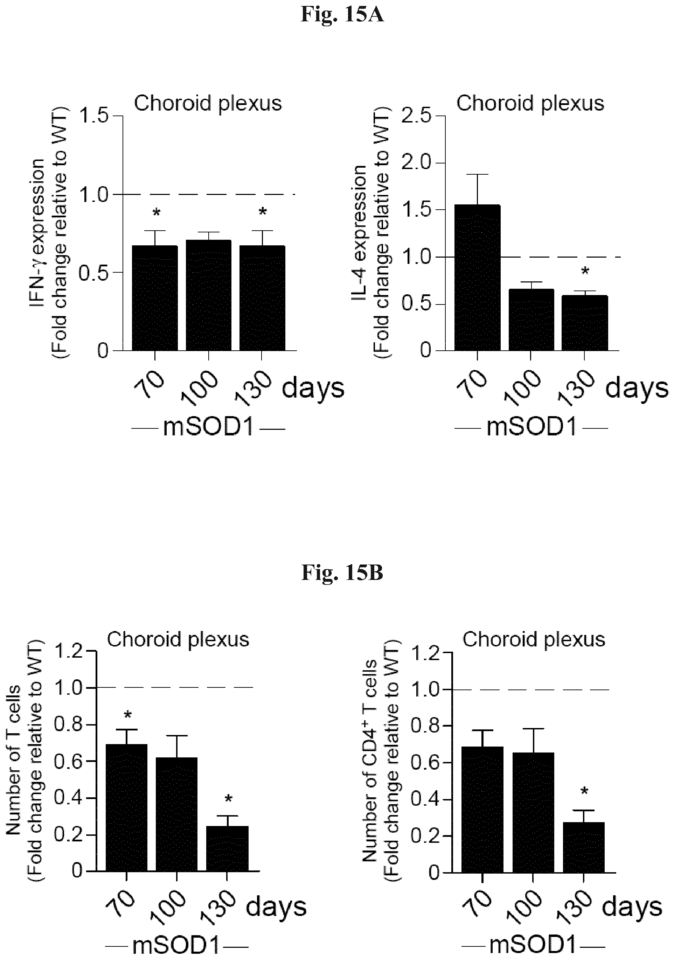

**Figs. 15A-D** show IFN-γ-dependent activation of the choroid plexus in mSOD1 mice. (A) mRNA levels of IFN-γ and IL-4 in the CP of mSOD1 mice were measured by qPCR at age 70, 100 and 130 days (n=6-7 per group; bars represent mean ± s.e.m.; *p< 0.05, vs. WT littermates by Student's t-test) and presented as fold change relative to wild-type. **(B)** Quantitative analysis by flow cytometry of the numbers of total T cells (left panel) and CD4⁺ T cells (right panel)(presented as fold change relative to WT littermates) in the CP of mSOD1 mice at age 70, 100 and 130 days (n=4-6 per group; bars represent mean ± s.e.m.; *p< 0.05, vs. WT littermates by Student's t-test). **(C)** Quantitative analysis by flow cytometry of the numbers of total T cells (left panel) and CD4⁺ T cells (right panel) in the CSF of 70 day old WT and mSOD1 mice (n=10 per group; graphs show number of cells in individual mice and mean ± s.e.m.; **p< 0.01, ***p< 0.001, by Student's t-test). **(D)** CP epithelial cells from 70 day old WT (black bars) and mSOD1 (white bars) mice were cultured *in vitro* and were either treated with IFN-γ (100ng/ml), IL-4 (10 ng/ml), or left untreated (UT). After 24 h in culture, mRNA levels of the adhesion molecules ICAM-1 (upper left panel) and VCAM-1 (upper right panel), and the chemokines CCL5 (lower left panel) and CXCL10 (lower right panel) in the cultured cells were measured by qPCR (n=3 per group; bars represent mean ± s.e.m.; **p< 0.01, ***p< 0.001, vs. untreated cells by one-way ANOVA followed by Tukey's HSD post hoc analysis) A.U. - arbitrary units.

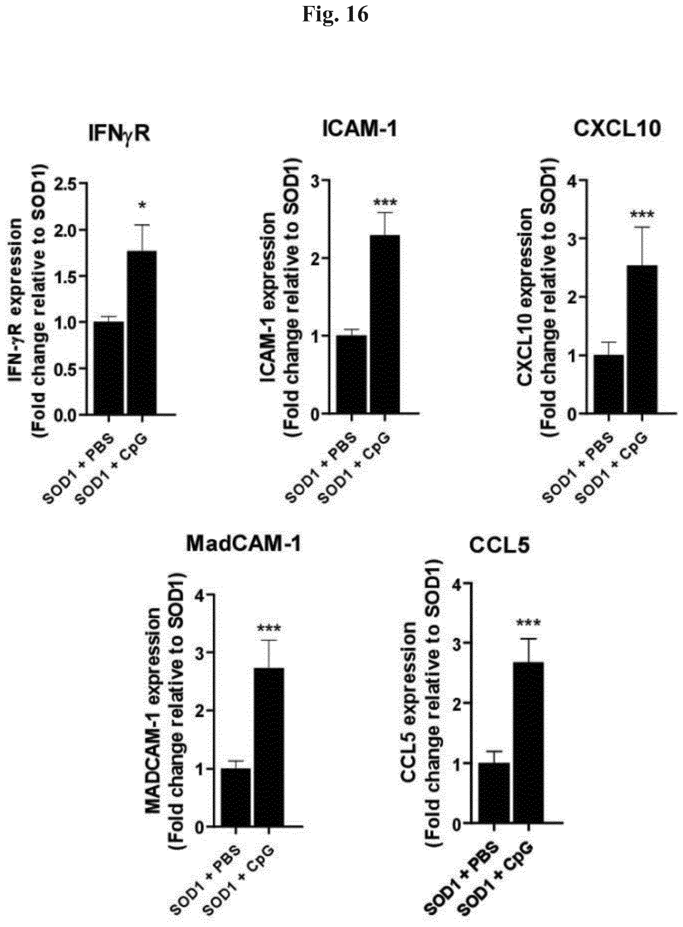

**Fig. 16** shows that CpG injections affect the choroid plexus epithelium of mSOD1 mice. CpG was injected i.p. on days 80 and 83 to mSOD1 mice. Changes in mRNA transcript levels, measured by real-time qPCR, are shown for IFN-γ receptor, ICAM-1, CXCL-10, MADCAM-1 and CCL5 (from upper left) in the CP of untreated (SOD1+PBS) and treated (SOD1+CpG) day 87 old mSOD1 mice (n = 6 mice per group; ****P* < 0.001; ***P* < 0.01; **P <* 0.05; one-way ANOVA, followed by Newman-Keuls *post hoc* analysis).

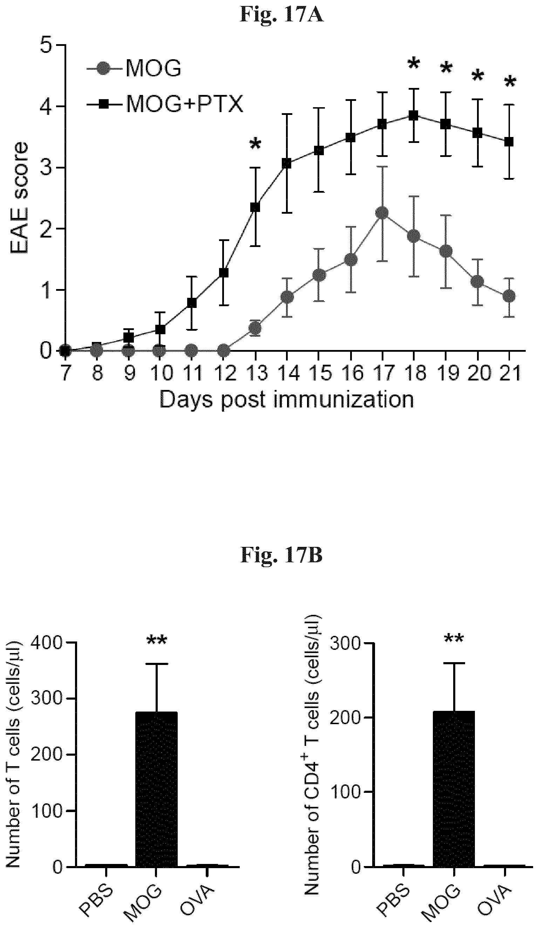

**Figs. 17A-B** show that induction of transient autoimmune encephalomyelitis activates the choroid plexus of WT mice for leukocytes trafficking. **(A)** WT mice were immunized with MOG₃₅₋₅₅ emulsified in CFA containing 0.5mg/ml *m. tuberculosis* (MOG, circles), to induce monophasic experimental autoimmune encephalomyelitis (EAE), and their clinical symptoms were compared to WT mice immunized with MOG₃₅₋₅₅ emulsified in CFA containing 2.5mg/ml *m. tuberculosis,* and that received injections of 300ng pertussis toxin (PTX) at the day of immunization and 2 days later (MOG+PTX, squares), causing chronic EAE (n=4-6 per group; mean ± s.e.m.; *p< 0.05; F=6.05, P=0.036, by repeated measures ANOVA). **(B)** Numbers of total T cells and CD4⁺ T cells in the CSF of MOG immunized WT mice (MOG) were quantified by flow cytometry, 14 days post immunization, and were compared to the numbers in WT mice immunized with ovalbumin (OVA) or in PBS-injected (PBS) mice (bars represent mean ± s.e.m.; **p< 0.01, ***p< 0.001, vs. PBS by one-way ANOVA followed by Student's t-test post hoc analysis).

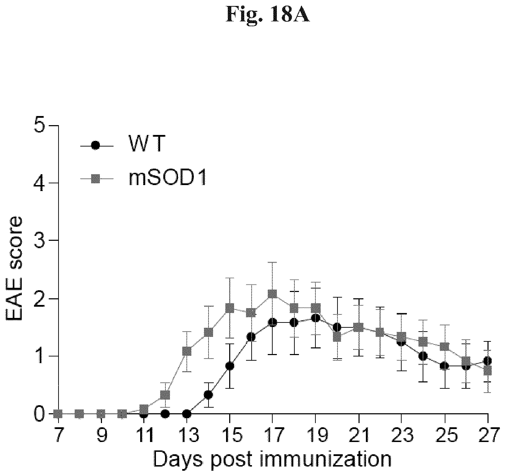

**Figs. 18A-E** show that induction of transient autoimmune encephalomyelitis in mSOD1 mice activates the choroid plexus for trafficking of leukocytes to the ventral horn gray matter. **(A)** mSOD1 mice (squares) were immunized with MOG, and their EAE symptoms were compared to those of MOG-immunized WT mice (circles) (n=6 per group; mean ± s.e.m.; F=0.41, P=0.535, by repeated measures ANOVA). **(B)** mRNA levels of the adhesion molecule ICAM-1 and the chemokines CCL5, CXCL9 and CXCL10 (from upper left) in the CP of MOG immunized WT (WT+MOG) and mSOD1 (mSOD1+MOG) mice, 14 days post immunization, were measured by real time qPCR and compared to the levels in non-immunized WT and mSOD1 mice (n=5-8 per group; bars represent mean ± s.e.m.; *p< 0.05, **p< 0.01, ***p< 0.001, vs. WT mice by one-way ANOVA followed by Student's t-test post hoc analysis) and presented as fold change relative to WT. (C) Quantitative analysis by flow cytometry of cell numbers in CSF showing elevation in total leukocytes (CD45.2⁺), T cells (CD45.2⁺TCRβ⁺), and CD4⁺ T cells (CD45.2⁺TCRβ⁺CD4⁺) in both immunized groups (n=6 per group; graphs show number of cells in individual mice and mean ± s.e.m.; *p< 0.05, **p< 0.01, ***p< 0.001, vs. WT mice by one-way ANOVA followed by Student's t-test post hoc analysis). X axis labels same as in **B. (D),** Number of CD4⁺ T cells was quantified by flow cytometry in the spinal cords of MOG-immunized WT and mSOD1 mice, 14 days post immunization (n=6 per group; bars represent mean ± s.e.m.; *p< 0.05, ***p< 0.001, vs. WT mice by one-way ANOVA followed by Student's t-test post hoc analysis). X axis labels same as in **B. (E)** Number of CD4⁺ T cells was quantified by flow cytometry in the spinal cords of MOG-immunized WT and mSOD1 mice, 28 days post immunization (n=6 per group; bars represent mean ± s.e.m.; ***p< 0.001, vs. WT mice by one-way ANOVA followed by Student's t-test post hoc analysis) X axis labels same as in **B.**

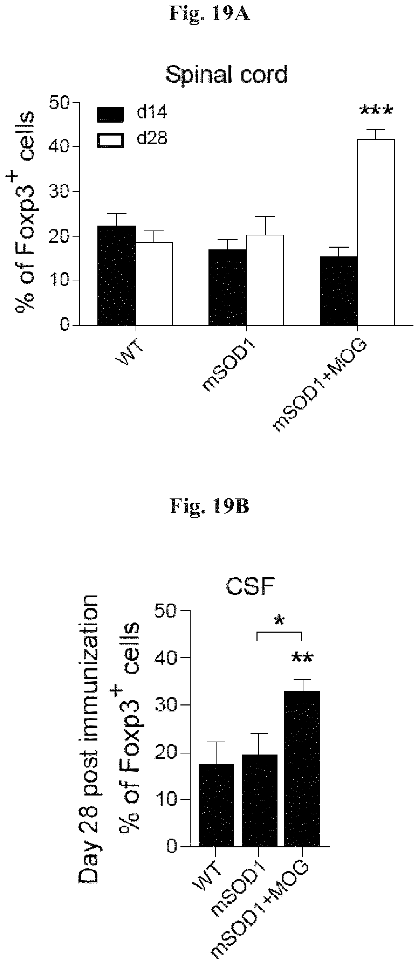

**Figs. 19A-G** show that induction of transient autoimmune encephalomyelitis in mSOD1 mice facilitates accumulation of Foxp3⁺ regulatory T cells in the spinal cord and increases life expectancy. (A-C), Quantitative analysis by flow cytometry of the percentage of Tregs (Foxp3⁺) out of CD4⁺ T cells (CD45.2⁺TCRβ⁺CD4⁺) in the spinal cords of WT, mSOD1 mice, and MOG-immunized mSOD1 mice at 14 (black bars) and 28 (white bars) days post immunization **(A)** and in the CSF **(B),** and blood **(C)** of the mice 28 days post immunization (n=6 per group; bars represent mean ± s.e.m.; *p< 0.05, **p< 0.01, ***p< 0.001, by one-way ANOVA followed by Student's t-test post hoc analysis). **(D)** mRNA levels of IL-10 in the spinal cords of MOG immunized mSOD1 mice (mSOD1+MOG), 28 days post immunization, were measured by real time qPCR (presented as fold change relative to WT) and compared to the levels in non-immunized WT and mSOD1 mice (n=4-6 per group; bars represent mean ± s.e.m.; *p< 0.05, vs. WT mice by one-way ANOVA followed by Student's t-test post hoc analysis). **(E)** mRNA levels of TGF-β1 in the spinal cords of MOG immunized mSOD1 mice (mSOD1+MOG), 28 days post immunization, were measured by real time qPCR and compared to the levels in non-immunized WT and mSOD1 mice (n=4-6 per group; bars represent mean ± s.e.m.; **p< 0.01, ***p< 0.001, vs. WT mice by one-way ANOVA followed by Student's t-test post hoc analysis), presented as fold change relative to WT. **(F-G)** MOG immunized mSOD1 mice (MOG) had significantly longer lifespan, compared to PBS injected mice (PBS) (n=10 per group) as shown by average survival age **(F)** (bars represents mean ± s.e.m.. **p< 0.01, versus PBS injected mSOD1 mice by student's t-test), and by Kaplan-Meier survival curves **(G)** (logrank. χ²=5.08, p=0.024). Percent survival corresponding to age. One representative graph out of two experiments is presented. PBS - solid line; MOG - dashed line.

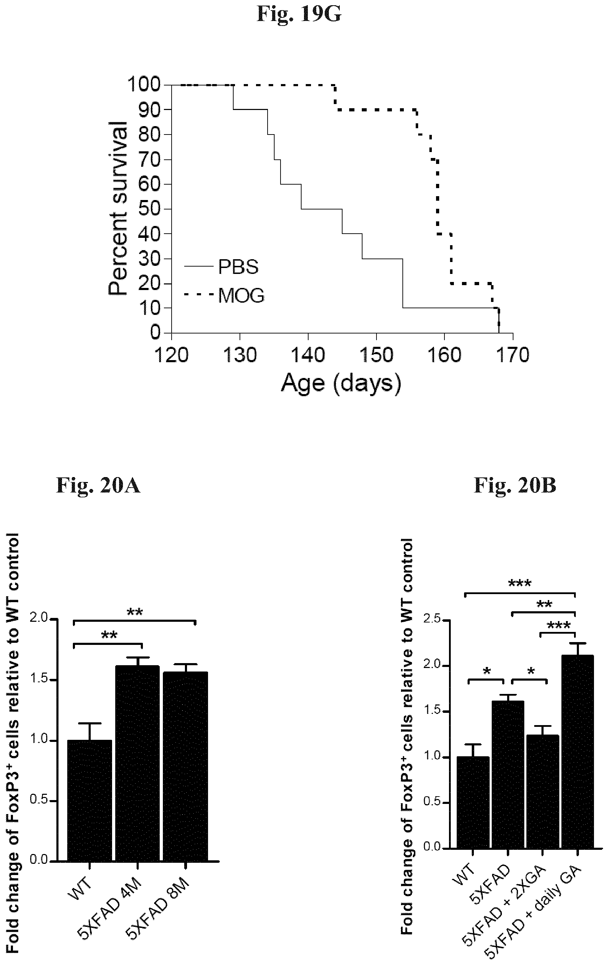

**Figs. 20A-D** show that infrequent vaccination with GA (Copolymer 1) rescues cognitive decline and reduces the incidence of peripheral FoxP3⁺ T cells (Tregs) in 5XFAD mice. The fold change in the frequency of Treg (FoxP3⁺) cells out of CD4⁺ T cells in the spleen of 5XFAD mice as compared to non-transgene (WT) controls was analyzed by FACS. (A). Histogram showing the fold change in the percentage of FoxP3⁺ cells gated from total CD4⁺TCRβ⁺ T cells, in 4 and 8 months old (4M and 8M, respectively) 5XFAD mice as compared to WT controls (WT:n=5; 5XFAD 4M:n=5; 5XFAD 8M:n=5; ***P* < 0.01). **(B)** Fold change in Tregs (FoxP3⁺) frequency relative to WT control in WT, 5XFAD mice, and in 5XFAD mice treated either by vaccinating twice, with the second injection being 3 days after the first (5XFAD+2XGA) or vaccinating every day for 28 days (5XFAD+daily GA) (WT:n=4; 5XFAD:n=5; 5XFAD+2XGA:n=5; 5XFAD+daily GA:n=6; *p< 0.05, **p< 0.01, ***p< 0.001). (C) Radial arm water maze (RAWM) was performed to 4 groups of 8 months old mice: non-transgene (WT, diamonds, dashed lines); 5XFAD (AD, squares, dotted lines); 5XFAD that were vaccinated twice in the first week and once a week for another 3 weeks (AD+GA weekly, triangles, solid line); and 5XFAD which were vaccinated daily for 28 days (AD+GA daily, circles, double line). Data is expressed as escape latency (seconds), each block represents three trials. Data is expressed as mean + SEM (WT:n=6; AD:n=6; AD+GA weekly:n=6; AD+GA daily:n=6). **(D)** Histogram showing the incidence of FoxP3⁺CD3⁺ T cells in the CP of three groups of mice: untreated WT, 5XFAD, and 5XFAD+GA (representing 5GA treatment) (WT:n=4; 5XFAD:n=6; 5XFAD+GA:n=7; ****P* < 0.001; one-way ANOVA, followed by Newman-Keuls *post hoc* analysis).

### DETAILED DESCRIPTION OF THE INVENTION

It has been found in accordance with the present invention that the choroid plexus epithelium, an active interface between the blood and the brain, is constantly populated by central nervous system (CNS)-specific effector memory type CD4⁺ T cells (Examples 1 and 2). It has further been found in accordance with the present invention that with aging, the antigenic specificity of such T cells is maintained, but their cytokine/chemokine balance changes towards a Th2-like epithelial inflammation, with detrimental consequences to the functioning brain (Example 3).

Our finding that the choroid plexus (CP), under physiological conditions, retains a broad repertoire of CD4⁺ T cell clones that recognize CNS antigens, together with the recent report that the choroid plexus contains specialized antigen presenting cells (APCs), Flt3⁺ dendritic cells, which can actively present self-antigens and stimulate T cells (Anandasabapathy N, et al. (2011)), supports the effector function of these T cells in this compartment. These T cells, after recognition and reactivation by their cognate antigens, either enter the CSF or remain in the CP. Focusing on circulating T cells that are retained in this compartment, we envisioned that a life-long crosstalk between these cells and the CNS, needed for maintaining cognitive ability, takes place at the blood-cerebrospinal- fluid barrier (BCSFB), with the choroid plexus epithelium as the mediator.

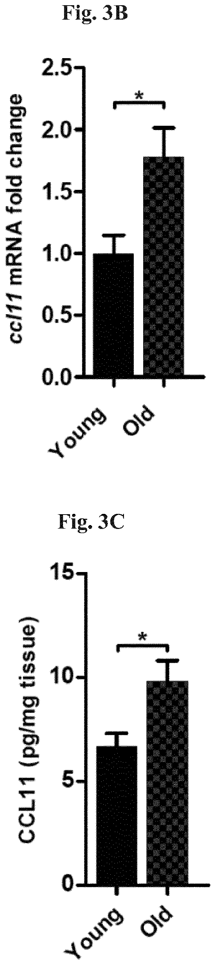

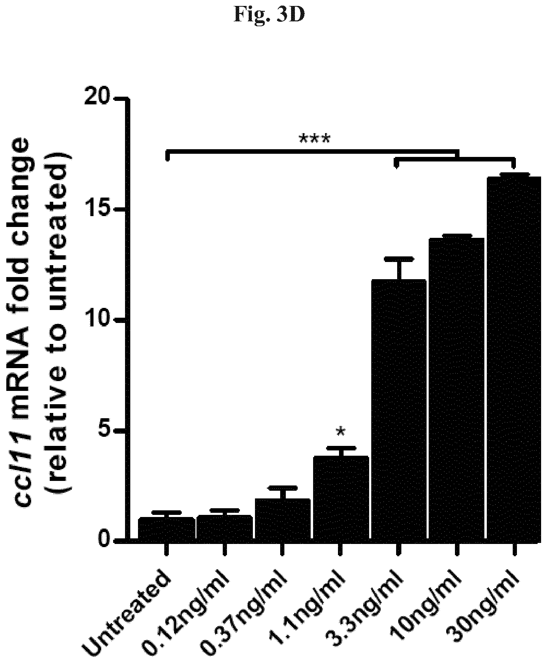

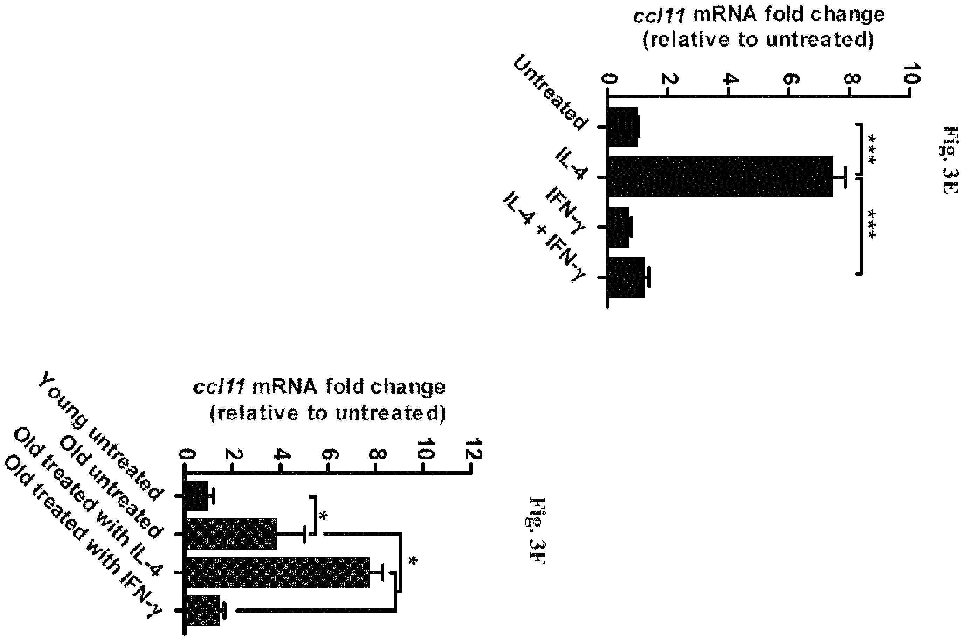

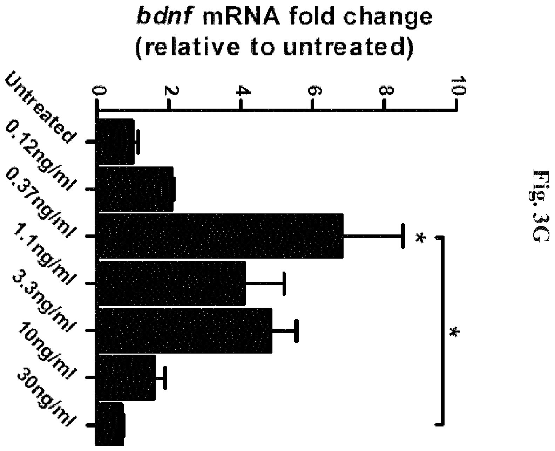

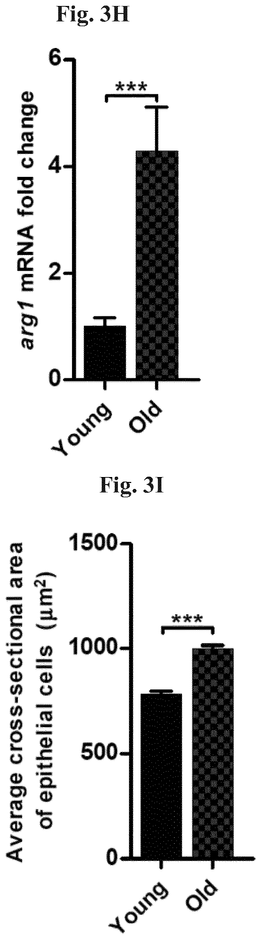

As shown herein below, examining the local effector cytokine balance in this epithelium revealed that while both IL-4 and IFN-γ were found in the choroid plexus throughout life, the ratio increased in favor of IL-4 during aging (Example 3, **Fig. 3**). Aging in general is associated with numerous functional changes in the immune system, also known as immune senescence, contributing to the dysfunction of the immune system. One of the most prominent features is that the aging process involves a shift towards a dominance of the helper Th2-related response. Accordingly, murine models of aging were shown to manifest age-associated dysregulation in cytokine production, specifically involving reduced production of IFN-γ, and generally increased production of IL-4. Thus, our findings describing a change in cytokine balance in the aged choroid plexus, appear to reflect the general condition of the immune system, further supporting the contention that the immune response within the choroid plexus is an integral part of systemic immunity, i.e. the immune response in the choroid plexus in terms of level of activation/suppression or Th-phenotype is a reflection of the immune response evident in the circulation, and that a life-long active dialogue takes place between the brain and the immune system in this compartment. Notably, as shown herein below, such a dialogue was also manifested by the CD4⁺ clonotypic enrichment of CNS-specific TCR in the choroid plexus of mice immunized with CNS antigens, but not of mice immunized with ovalbumin.

The change in local cytokine milieu at the choroid plexus of the aged mice was found here to critically affect this compartment. When not properly balanced by IFNγ, high levels of IL-4 induced the choroid plexus epithelium to produce CCL11, a chemokine that is associated with age-related cognitive impairments and accumulates at the aged CSF (Example 3). It was previously shown that cognitive tasks lead to accumulation of IL-4-producing T cells in the meningeal spaces of the brain (Derecki NC, et al. (2010)), and that CCL11 can interfere with IL-4 signaling (Stevenson NJ, et al. (2009)). The fact that CCL11 was found here to be produced by the choroid plexus epithelium, and specifically in response to IL-4 stimulation, coupled with its negative effects on brain functional plasticity, supports our hypothesis that the loss of cognitive ability in old mice is a reflection of epithelium-T cell crosstalk dysregulation at the choroid plexus. Such Th2-like inflammation is known to be associated with cancer or epithelial pathologies outside the CNS, such as the lung epithelium in asthma. Intriguingly, examining the aged choroid plexus epithelium in analogy to the asthmatic Th2-inflamed lung epithelium, revealed similarities in mechanistic and functional dysregulation that may reflect common disease-like processes amenable to immunomodulation.

It has further been found in accordance with the present invention that the choroid plexus epithelium acts as a bidirectional sensory organ of the CNS, receiving danger signals from within the parenchyma, and in response, regulating trafficking and maintenance of the CNS. CD4⁺ T cells expressing IFN-γ, IL-4, and the transcription factor FoxP3 were found in the choroid plexus under physiological conditions, as shown herein below (Example 5). By dissecting how the different effector cytokines affect this compartment, we found IFN-γ to be a key regulatory cytokine in activating the choroid plexus epithelium to express trafficking molecules and related chemokines (Example 6). Notably, this activation of the choroid plexus was found herein to be tightly regulated through the induction of IFN-γ receptor (IFN-γR) on the epithelium by a parenchyma-derived danger signal (Example 7). In contrast, IL-4 had no noticeable effect on trafficking molecules, yet it modulated the immune milieu by upregulating Arginase-1, potentially affecting the phenotype of immune cells in this compartment (Example 6).

As an immune privileged site, the CNS has a poor tolerance for immune cell infiltration to its parenchyma, and therefore much of what we know about immune infiltration to the CNS was studied in pathological states (Engelhardt and Ransohoff, 2005). Moreover, little attention has been devoted to the distinction between pathologies that are inflammatory in their etiology, manifested by pathogenic immune cell infiltration and for which immune suppression is beneficial, versus pathologies that are of non-inflammatory etiology, and in which transient or chronic local inflammation can potentially benefit from the infiltration of resolving immune cells (Banerjee et al., 2008; Beers et al., 2008; Derecki et al., 2012; Schwartz and Shechter, 2010; Simard et al., 2006).

In the present study, we focused on the blood-cerebrospinal- fluid barrier as the portal through which leukocytes can enter the CNS under physiological conditions, without the necessity of a breached blood-brain barrier (BBB). We assumed that the site at which the immune system should sense and respond to a threat coming from the parenchyma is the choroid plexus - an epithelial tissue that serves as a filter between the blood and the CNS and -as such-can receive signals from within the CNS and from immune cells residing within it. In order to cross this epithelial barrier, immune cells must first adhere to it and negotiate the intercellular junction. *In vitro* studies of the basal-to-apical T cell egression process through the lung epithelium showed that ICAM-1 expression is essential for the adhesion of T cells to the epithelial cells and for their consequent transmigration, and that ICAM-1 is upregulated when the epithelium is stimulated by IFN-γ, with or without TNF-α, (Miller and Butcher, 1998; Porter et al., 2008; Porter and Hall, 2009; Taguchi et al., 1998). In the present study, we found a synergistic effect between IFN-γ and TNF-α, on the expression of adhesion molecules, including ICAM-1 (Example 6). Our results are in line with the reported synergistic effect of TNF-α, and IFN-γ stimulation on the migratory properties of both lung (Barrett et al., 1998) and colon (Fish et al., 1999) epithelial cells. The finding in accordance with the present invention that TNF-α, could upregulate the expression of the receptor for IFN-γ on epithelial cells suggests that the synergistic effect of these two cytokines is mediated by increased sensitivity of the cells to IFN-γ due to the elevation in its receptor (Example 7). The fact that in healthy animals the choroid plexus epithelium hardly expresses IFN-γ receptor *in vivo,* and the observation that acute CNS injury can upregulate its expression, suggest that in order to fully activate expression of trafficking molecules in response to IFN-γ stimulation, the choroid plexus epithelium must be primed by danger signals coming from the injured CNS, possibly in the form of inflammatory mediators such as TNF-α. The danger signal may originate in any assault to the CNS, such as acute injury or an inflammatory or degenerative disease.

Crossing the epithelial monolayer involves the expression of chemokines on the apical side of these cells in order to create a chemical gradient. The expression of various chemokines on the apical surface of the epithelial barrier can also control the type of cells which infiltrate to the tissue. Thus, the infiltrating cells in most pulmonary diseases are memory T cells, which express high levels of CXCR3, the chemokine receptor for CXCL9, CXCL10 and CXCL11. Interestingly, memory T cells are the primary population of T cells in the CSF under normal conditions and were suggested to infiltrate through the choroid plexus (Kivisakk et al., 2003). In addition, monocyte infiltration following traumatic brain injury correlates with increased expression and presence of CCL2 in the choroid plexus and in the CSF (Szmydynger-Chodobska et al., 2012). Our findings thus suggest that IFN-γ is needed for activation of trafficking molecules but its receptor is hardly expressed on the choroid plexus epithelium unless a danger signal such as TNF-α, is sensed by it.

Epithelial cells are found in many tissues and play a role beyond trafficking. In the gut, it was shown that the M cells of the epithelial layer are responsible for transcytosis of antigens from the gut lumen to its associated immune follicles and that TGF-β and retinoic acid, which are constitutively expressed by the epithelial cells, are responsible for the generation of oral tolerance. As in the gut epithelium, the choroid plexus epithelium constitutively secretes transforming growth factor-β (TGF-β) to the CSF. Accordingly, CSF from both rodent and human is able to inhibit T cell proliferation and the secretion of pro-inflammatory cytokines, in part due the presence of TGF-β. It has been found according to the present invention that the choroid plexus is enriched with Tregs (Example 5), which can further regulate the activation of immune cells. Although TGF-β alone can mediate the induction of Tregs, its combination with IL-6 can determine Th17 differentiation (Zhou et al., 2008). Therefore, the differential local expression of IL-6 and TGF-β may prove important for the immunoregulatory properties of this compartment. The fact that neither IL-17, nor its induced cytokine IL-6, had any effect on expression of leukocyte trafficking molecules on the choroid plexus, argues that induction of molecules that can potentially activate the choroid plexus to facilitate trafficking through the blood-cerebrospinal- fluid barrier is not by itself an encephalitogenic process (Example 6). Moreover, our results disclosed herein below show that IFN-γ signaling is involved in facilitation of life-long immunosurveillance through the choroid plexus under physiological conditions. Knockout of IFN-γR resulted in reduced levels of homing determinants on the choroid plexus of naive animals relative to WT (Example 8) and impaired recovery after spinal cord injury (Example 9). In contrast, IL-17, which is known to be involved in encephalitogenic conditions of the CNS, such as experimental autoimmune encephalomyelitis (EAE), failed to activate the choroid plexus for trafficking. Altogether, these results substantiate our contention that the activation of the choroid plexus for trafficking is by itself not a sign of pathology. Nevertheless, although we found that IFN-γ supports non-encephalitogenic trafficking, it could lead to pathogenicity in the presence of Th-17 cells, which were barely detectable in the naive choroid plexus. Supporting this notion is the fact that IFN-γR-KO mice fail to develop classic EAE (Lees et al., 2008). These results are also in line with the reported CCR6⁺ cell entry through the choroid plexus, linked with the induction of EAE (Reboldi et al., 2009), and might suggest that prior to Th-17 entry, this compartment is activated by IFN-γ. Accordingly, the activation of trafficking through the choroid plexus, which can potentially be the beginning of an inflammatory disease, does not necessarily portend to it; such an opening can serve as a gate for opportunistic cells, which cannot infiltrate by themselves, but can use the opened port to enter the parenchyma as "Trojan horses", thus induce autoimmune disease.

Since our initial demonstration that T cell-mediated immunity supports hippocampus-dependent cognitive ability, we suspected that the significance of our findings would be in their potential relevance to aging (Ron-Harel N & Schwartz M (2009)). The present study provides evidence of a mechanism connecting aging of the immune system with aging of the CNS, and identifies the choroid plexus as a site of constant dialogue between CNS-specific T cells and the brain, demonstrating that this dialogue is altered with age. We further show that restoring the Th1/Th2 balance to that of young animals (having a Th1 phenotype) has the potential to partially restore memory function (Example 4, **Fig. 5**). It is therefore possible that targeting treatment for brain aging, based on immunomodulating Th2 inflammation at the blood-cerebrospinal- fluid barrier, may lead to hitherto unexplored approaches to reverse or arrest dementia, other age-related cognitive deficits or neurodegenerative diseases.

In addition, the results of this study provide experimental support for a dialogue that takes place between the circulating immune system and the choroid plexus epithelium. We found this epithelial-T cell crosstalk to facilitate the activation of the choroid plexus and to enable CNS maintenance and immune trafficking. Moreover, this study emphasizes that the mere activation of the choroid plexus for trafficking is not a sign of a beginning of a disease; the effector phenotype of the immune cells and the state of the parenchyma critically determine the outcome.

The present inventors also show herein that, even though CNS parenchyma of mSOD1 mice (a model for human amyotrophic lateral sclerosis) shows signs of local inflammation, their choroid plexus is not activated to enable immune cell trafficking; however, the choroid plexus is amenable to activation as inferred by the fact that IFN-γ treatment of choroid plexus cultures elevated the expression of trafficking molecules of the choroid plexus cultures, suggesting that the choroid plexus of mSOD1 mice has the ability to respond to effector T cell-derived cytokines (Examples 12 and 13). It is further shown herein that immunomodulation of the choroid plexus epithelium of mSOD mice with a Th1- adjuvant, CpG, causes upregulation of all trafficking molecules tested (Example 14). The upregulation of the trafficking molecules enables the transfer of the beneficial T cells from the circulation to the CSF and the CNS parenchyma. Indeed, induction of monophasic (self-resolving mild inflammatory) EAE in wild type mice by immunization with a myelin oligodendrocyte glycoprotein (MOG) peptide (together with low levels of *M. tuberculosis*) induced the expression of the trafficking molecule ICAM-1 by the choroid plexus epithelium and infiltration of CD4⁺ T cells to the CSF (Example 15) and the ventral horn gray matter, the area where motor neurons reside (Example 16).

The level of Treg cells in the choroid plexus is a function of the level of this cell type in the circulating immune system, such as the spleen and is critical for its function. For example, depletion of Fox3P⁺ Treg cells in mice causes the activation of the choroid plexus for trafficking (Example 10). It is also shown herein (Example 18) that daily injection of the immune-modulator Copolymer 1 to 5XFAD mice (a mouse model for human Alzheimer's disease), causes enrichment of FoxP3+ Treg cells in the spleen and does not improve the cognitive function of the mice. The reason seems to be that the high level of Treg cells in the circulation and at the choroid plexus (inferred by the high level in the spleen) suppresses the expression of trafficking molecules and denies the entry of Th1 cells to the damaged brain. Copolymer 1 is a random non-pathogenic synthetic copolymer, a heterogeneous mix of polypeptides containing the four amino acids L-glutamic acid (E), L-alanine (A), L-tyrosine (Y) and L-lysine (K) in an approximate ratio of 1.5:4.8:1:3.6, but with no uniform sequence. Copolymer 1 acts as a low-affinity antigen that activates a wide range of self-reacting T cells, resulting, when given at low frequency, in neuroprotective autoimmunity that is effective against both CNS white matter and grey matter degeneration.

This shows that there is room for therapeutic intervention aimed at activating the choroid plexus (characterized by expression of trafficking molecules) by for example immunization with a CNS-specific antigen (to direct the leukocytes to the area of damage in the CNS) and a Th1-adjuvant (to induce the proliferation of Th1-type IFN-γ producing CD4⁺ cells and thus steer the immune-response towards a beneficial Th1 phenotype) to enable Th1-type CD4⁺ cells and other beneficial leukocytes to infiltrate the damaged CNS and promote healing.

In accordance with the above and the findings of the present invention, there are several ways to restore/rejuvenate the choroid plexus; either by restoring the Th1/Th2 balance (and reducing the level of immunosuppression at the choroid plexus) or by restoring the epithelium tissue itself. Restoration of the cytokine milieu may be accomplished, for example by induction of homeostatic-driven proliferation of T cells (Example 4) or T-cell vaccination with a CNS-specific antigen or a peptide derived from a CNS-specific antigen or from an analog thereof, or an analog or derivative of said peptide, using Th1 biased adjuvant.
Thus, in one aspect, the present invention provides a vaccine for use in the treatment of ALS comprising a non-encephalitogenic Th1 adjuvant as a sole active ingredient, wherein the vaccine is to be administered according to a regimen to thereby confer reduction and maintenance of the level of immunosuppression in the circulation of said mammal relative to a reference and induction towards a Th1-type immune response, wherein said reduced level of immunosuppression and Th1-type immune response in the circulation indicates and ensures activation of the choroid plexus of said mammal and maintenance of said activation thus allowing either anti-inflammatory immune cells or immune cells which acquire a healing phenotype at the cerebrospinal fluid to pass through the choroid plexus and accumulate at a site of damage in the CNS caused by said ALS, and the non-encephalitogenic Th1 adjuvant is selected from an agonist of TLR3, 4, 5, 7, 8 and 9.

It is known that intense stimulation of the immune system by repeated immunization may, after a brief activation towards Th1 response and lowering of the level of regulatory T cells, raise the level of regulatory T cells and impose immunosuppression (Lalive et al., 2011), which leads to deactivation of the choroid plexus and reduction or stop of cell trafficking from the circulation via the choroid plexus and into the CNS.

Furthermore, it has been found herein that daily administration of Copolymer 1 to 5XFAD mice causes increase in the level of Treg cells in the spleen and has no beneficial effect on mental cognitive performance, while administration of Copolymer 1 on a weekly basis resulted in at least a transient decrease in the level of Treg cells in the spleen and marked improvement in mental cognitive performance (Example 18). It is therefore expected that the immunization that activates the choroid plexus, as reflected for example by the decreased ratio of Treg cells to IFN-γ producing Th1 cells relative to the ratio before immunization, will fade upon repeated immunization and the choroid plexus will become immunosuppressed as evidenced by an increased ratio. It is therefore important to ensure maintenance of the activation of the choroid plexus in order to provide continuing cell trafficking and accumulation of immune cells with a healing phenotype in the damaged or senescent CNS.

The combined findings of the present invention leads to the understanding that a regimen of administration of the active ingredient as defined herein can be established on an individual basis, i.e. a decision whether to repeat administration of the active ingredient can be reached based on the information gathered from monitoring one or more parameters reflecting the degree of immunosuppression and the Th1/Th2 balance in the choroid plexus in an individual being treated. The level of the immunosuppression measured in the individual can be compared with a reference that may be, but is not limited to, the level of immunosuppression in the individual before the last administration of the active ingredient, or it may be a predetermined reference based on the level of immunosuppression in a population of individuals in need of treatment according to the present invention that are responding well to the treatment, or the level of immunosuppression in a population of healthy individuals. In this respect, depending on the change in the level of immunosuppression and/or Th-phenotype relative to the reference, a decision can be made whether to repeat the administration of the active ingredient or wait and continue the monitoring.

Thus, in another aspect, the present invention provides a vaccine for use in the treatment of ALS comprising a non-encephalitogenic Th1 adjuvant as a sole active ingredient, wherein the vaccine is to be administered according to a regimen to thereby confer reduction and maintenance of the level of immunosuppression in the circulation of said mammal relative to a reference and induction towards a Th1-type immune response, wherein said reduced level of immunosuppression and Th1-type immune response in the circulation indicates and ensures activation of the choroid plexus of said mammal and maintenance of said activation thus allowing either anti-inflammatory immune cells or immune cells which acquire a healing phenotype at the cerebrospinal fluid to pass through the choroid plexus and accumulate at a site of damage in the CNS caused by said ALS, and the non-encephalitogenic Th1 adjuvant is selected from an agonist of TLR3, 4, 5, 7, 8 and 9.

In one embodiment, the method comprises: (a) administering the non-encephalitogenic Th1 adjuvant to the subject in need; (b) determining said regimen by: (i) monitoring immunosuppression and/or Th1/Th2 balance in the subject by measuring in a blood sample obtained from the subject, within a predetermined time-period following the administering, one or more parameters reflecting a degree of immunosuppression and/or Th1/Th2 balance in the choroid plexus in the subject; and (ii) comparing the one or more parameters measured in (b)(i) with the reference and determining whether the one or more parameters is different from the reference; and (c) deciding, based on the relation of the one or more parameters measured in (b)(i) to the reference, whether to repeat treatment and monitoring by repeating steps (a) and (b) or to continue monitoring by repeating only step (b).

In certain embodiments, the level of immunosuppression in the choroid plexus is reflected in the level of Treg cells in the circulation of the subject. This level is understood to undergo variations in response to events that affect the immune system. For example, disease may increase the level of Treg cells in the circulation and activation of the immune system by immunization may reduce the level of immunosuppression relative to the situation in disease. The level of Treg cells in the circulation may easily be measured by determining the level of CD4⁺CD25⁺FoxP3⁺ cells or CD4⁺CD25⁺ FoxP3⁻ cells by conventional methods such as, but not limited to fluorescence activated cell sorting (FACS), flow cytometry or real-time PCR.

In certain embodiments, the Treg cells inhabiting the choroid plexus, the CNS or the circulation are CD4⁺CD25⁺FoxP3⁺ cells or CD4⁺CD25⁺ FoxP3⁻ cells.

In certain embodiments, "the anti-inflammatory immune cells or immune cells which acquire a healing phenotype" are IL-10 producing cells, such as activated M2-machrophages, CD4⁺CD25⁺Foxp3⁺ Treg cells or CD4⁺CD25⁺ FoxP3⁻ Treg cells (see Example 17, **Fig. 17D**).

The terms "Th1/Th2 balance", "Th-phenotype" and "Th1/Th2 status", are used interchangeably herein, and refer to the predominant immune response or immune status in terms of Th1-type cells (characterized by production of cytokines typical for Th1 response, such as IFN-γ) or cytokine milieu (IFN-γ) or Th2-type cells (characterized by production of cytokines typical for Th2 response, such as IL-4) or cytokine milieu (IL-4). In addition to cytokine production profiles, there are a number of cell surface markers proposed to differentiate Th1 vs. Th2 subtypes. For example, Th1 cells express both components of IL-12 receptor chains (beta 1 and alpha. Only Th2 cells appear to express a fully functional IL-1 receptor, and ST2L/T1, a newly discovered IL-1 RI-like molecule, is found on Th2 cells only. Chemokine receptors CXCR3 and CCR5 are characteristic of Th1 cells, while CXCR4, CCR3, CCR4, CCR7 and CCR8 are associated with Th2 cells. CD30, a member of the TNF superfamily, is associated with Th2 cells.

The term "circulation" as used herein refers to the circulating immune system, i.e. the blood, spleen and lymph nodes. It is a well-known fact in the field of immunology that the cell population profile in the spleen is reflected in the cell population profile in the blood (Zhao et al., 2007). Furthermore, daily administration of the immune-modulating drug Copolymer 1 raises the level of Treg cells in the blood of the subjects receiving the daily injections (Lalive et al., 2011). It has also been shown by the inventors of the present invention that daily administration of Copolymer 1 to mice raises the level of Treg cells in the spleen of the mice receiving the daily injections (Example 18). Therefore, data showing changes in the immune cell population in the spleen in response to immunization or disease reflect the changes occurring in the immune cell population in the blood, which in turn indicates the changes taking place in the choroid plexus.

In one embodiment, the parameter measured in a blood sample from the subject is: (i) a ratio of CD4⁺ Treg cells to CD4⁺ effector T cells; (ii) a level of CD4⁺ Treg cells; (iii) a level of IFN-γ producing CD4⁺cells; (iv) a ratio of CD4⁺Treg cells to IFN-γ producing CD4⁺ T cells; (v) a proliferative response of peripheral mononuclear cells to a CNS-specific antigen, a peptide derived from a CNS-specific antigen or from an analog thereof, or an analog or derivative of said peptide; or (vi) any combination of (i), (ii), (iii), (iv) and (v).

The effector T cells measured are CD4⁺ cells that may be T-Bet⁺ thus producing IFN-γ or they may be T-Bet⁻ that do not produce IFN-γ.

The IFN-γ producing CD4⁺ cells may be CNS-specific as it has been shown in accordance with the present invention that the CD4+ cells at the choroid plexus are predominantly CNS-specific (Example 2, **Fig. 2****;** Example 4, **Figs. 4** **and** **5**), and that leukocyte trafficking through the choroid plexus is achieved following immunization with a CNS-specific antigen, but is not achieved following immunization with an irrelevant antigen, thereby identifying the choroid plexus as a novel target for immunomodulation with CNS-specific antigen.

As mentioned above, the parameter indicating a Th1-type immune response may be a predominating level of IFN-γ producing CD4⁺ cells, while the parameter indicating a Th2-type immune response may be a predominating level of IL-4 producing CD4⁺ cells.

In one embodiment, the reference is selected from the group consisting of (a) a parameter measured in the most recent blood sample obtained from said subject before said administering, said parameter indicating a degree of immunosuppression and/or Th1/Th2 status in the choroid plexus in said subject. In certain cases, the reference is the parameter measured before initiation of treatment of the subject, i.e. a base-line degree of immunosuppression and/or Th1/Th2 status in the subject that is in need of treatment and before the treatment starts; (b) a parameter indicating the degree of immunosuppression and/or Th1/Th2 status in the choroid plexus characteristic of a population of subjects afflicted with a disease, disorder, condition or injury of the CNS responding well to said administering, wherein said parameter is measured in blood samples obtained from said subjects; or (c) a parameter indicating the degree of immunosuppression and/or Th1/Th2 status in the choroid plexus characteristic of a population of healthy subjects, wherein said parameter is measured in blood samples obtained from said subjects. In particular, the reference is measured in a blood sample obtained from said subject before said administering in (a).

The monitoring is thus used to determine whether treatment and monitoring should be repeated or only monitoring should be continued/repeated. For example, but limited thereby, if the reference is the base-line level of ratio of Treg to effector T cells of the subject in need of treatment before treatment has been initiated (measured at the first instance of monitoring), and the subject has been administered the active ingredient once (the first administration), then treatment and monitoring will be repeated if the parameter measured at the second instant of monitoring (measured after the first administering of the active ingredient) is similar to or higher than the base-line reference. The subsequent monitoring will compare the parameter measured after the second administration of the active ingredient with the last parameter measured, which is now the new reference. This cycle is repeated until the treatment is discontinued. If the parameter measured after the first administration, on the other hand, is lower than this base-line reference, then the subject will not receive an additional dose of the active ingredient but will only be monitored a second time, and then a new decision is made. In this case, the subsequent monitoring will compare the parameter with the base-line reference. It is also contemplated that the subsequent monitoring will compare the parameter measured at the second monitoring with the parameter measured at the previous monitoring (that was made without treatment), which is now the new reference. The aim is to keep the parameter below the base-line reference.

If the reference used is a parameter indicating the degree of immunosuppression/Th-phenotype in the choroid plexus characteristic of a population of subjects afflicted with a disease, disorder, condition or injury of the CNS responding well to said administering, wherein said parameter is measured in blood samples obtained from said subjects; or a parameter indicating the degree of immunosuppression, Th-phenotype, or both, in the choroid plexus characteristic of a population of healthy subjects, wherein said parameter is measured in blood samples obtained from said subjects, then treatment and monitoring will be repeated if the parameter measured subsequent to treatment in a blood sample obtained from the subject in need of treatment is above said reference. If, on the other hand, the parameter measured after administration of the active ingredient to the subject is equal to or lower than this reference, then only monitoring is repeated and no administration of the active ingredient is performed.

In certain embodiments, the parameter measured is the ratio of Treg cells to effector CD4⁺ T cells in a blood sample obtained from said subject, said reference is the most recent ratio measured in said subject before said administering the active ingredient, and (i) said treatment and monitoring is repeated when the ratio is substantially similar to or higher than the reference; or (ii) said monitoring is repeated when the parameter is lower than the reference value.

In certain embodiments, the predetermined time-period between monitoring and administration of the active ingredient is between 1 and 16 weeks. In particular, the predetermined time-period may be 2, 3, 4, 5 or six days; or 1, 2, 3, 4, 5, 6, 7, 8, 9, 10, 11, 12, 13, 14, 15 or 16 weeks.

The term "substantially similar" as used herein refers to a difference between two values that is not larger than 5% of larger value or a difference that is not statistically significant when analyzed using accepted statistical methods (for example, but not limited to, Student's t-test, with α=0.05).

It has also been found in accordance with the present invention that injection of CpG into normal healthy mice resulted in that the choroid plexus upregulated both genes that are required for immune cell trafficking in this compartment (IFNgR, ICAM-1, MadCAM-1) and neurotropic factors needed for central nervous system (CNS) repair (IGF-1, BDNF). Injection of CpG to animals 1 day before performing spinal cord injury (SCI) resulted in that CpG significantly upregulated both trafficking and neurotropic molecules. Thus, the Th1 adjuvant augments the choroid plexus's ability to let beneficial Th1 type CD4+ T cells and other anti-inflammatory immune cells or immune cells which acquire a healing phenotype at the cerebrospinal fluid pass through and enter the CNS parenchyma, home to the injured site and assist in the healing of the injured tissue.

Thus, in certain embodiments, the Th1 adjuvant comprises an agonist of TLR3, 4, 5, 7, 8 or 9. For example, the TLR agonist may be an agonist of TLR9, such as a CpG or stabilized immune modulatory RNA (SIMRA). In particular, as shown below in the Examples, the CpG may be of class B, for example ODN1826. The Th1 adjuvant of the present invention may also be a CpG in combination with a cationic peptide of the sequence KLKL₅KLK (SEQ ID NO: 2). The TLR agonist may be an agonistic antibody, i.e. an antibody that binds to and activates the TLR.

The terms "CpG", "CpG oligodeoxynucleotides" and "CpG ODN" are used interchangeably herein and refer to short single-stranded synthetic DNA molecules that contain a cytosine triphosphate deoxynucleotide ("C)" followed by a guanine triphosphate deoxynucleotide ("G"). The "p" refers to the phosphodiester link between consecutive nucleotides, although some ODN have a modified phosphorothioate (PS) backbone instead.

The TLR3 agonists may be selected from e.g. double-stranded RNA (dsRNA), a molecular pattern associated with viral infection, or a synthetic analog thereof, such as polyinosine-polycytidylic acid (poly(I:C)).

The agonist of TLR 4 may be MPL (3-O-desacyl-4'-monophosphoryl lipid) optionally in combination with alum (AS04).

The TLR5 agonist may be flagellin, e.g. flagellin purified from B.subtilis (Gram+) or S.typhimurium (Gram-) bacteria.

The TLR 7/8 agonist may be a single stranded RNA or a small synthetic molecule such as imidazoquinoline or a nucleoside analog, e.g. Gardiquimod, Imiquimod and R848 (resiquimod).

In certain embodiments, the Th1 adjuvant comprises a combination of an agonist of TLR 3 with an agonist of TLR 8, or a combination of an agonist of TLR 4 with an agonist of TLR 8. In addition, the Th1 adjuvant of the present invention may be an agonist of retinoic acid inducible gene I (RIGI) or an agonist of dectin-1.

The term "central nervous system (CNS)-specific antigen" as used herein refers to an antigen of the central nervous system that specifically activates T cells such that following activation the activated T cells accumulate at a site of injury or disease in the CNS of a patient, or at the choroid plexus. The CNS-specific antigens and analogs thereof, the peptides derived from CNS-specific antigens or from analogs thereof, and analogs or derivatives of said peptides, include, but are not limited to, the agents described in US Patent No. 7,560,102 and PCT Publications WO 99/60021 and WO 02/055010 of the applicant.

In certain embodiments, the active ingredient is a non-encephalitogenic or weakly encephalitogenic combination of a Th1 adjuvant selected from an agonist of TLR3, 4, 5, 7, 8 and 9 and an agent from a CNS-specific antigen of mammal, preferably human, origin, selected from the group consisting of myelin basic protein (MBP; SEQ ID NO: 3); myelin oligodendrocyte glycoprotein (MOG; SEQ ID NO: 5); proteolipid protein (PLP; SEQ ID NO: 4); myelin-associated glycoprotein (MAG); S-100; β-amyloid; Thy-1; a peripheral myelin protein including P0, P2 and PMP22; neurotransmitter receptors including acetylcholine receptor; and the protein Nogo including Nogo-A, Nogo-B and Nogo-C and the Nogo receptor.

As mentioned above, the induction of monophasic EAE in wild type mice with a MOG peptide activates the choroid plexus, as indicated by the increase in infiltrating T cells in the CSF (Example 15), and to the ventral horn gray matter, the area where motor neurons reside (Example 16), relative to naive or ovalbumin (OVA)-immunized mice.

In certain embodiments, the Th1 adjuvant is a CpG.

It is a well-established fact in the field of immunology that active and passive immunization, wherein an antigen or antigen-specific immune-cells are administered, respectively, results in the same kind of immune response. For example, active immunization of a mammal with the CNS-specific antigenic peptide MOG₃₅₋₅₅ will induce a similar response as the immunization with MOG₃₅₋₅₅-specific T cells.

The term non-encephalitogenic Th1 adjuvant as used herein refers to an adjuvant that stimulates the immune system and directs the differentiation of the immune cells towards the Th1 phenotype, but excluding the Th17 phenotype. Thus, this agent stimulates the cellular immune response without causing encephalitis. As the use of this term is somewhat unorthodox, and since the term actually refers to the adjuvant's encephalitogenic activity only when given in combination with a CNS-specific antigen, the term is used interchangeably herein with the term "a non-encephalitogenic or weakly encephalitogenic combination of a Th1 adjuvant and an agent selected from..." The term "weakly encephalitogenic" refers to encephalitogenic activity that causes monophasic encephalomyelitis, i.e. transient encephalitis that spontaneously resolves, or that causes weakly symptomatic disease as measured by time of onset or mean maximum disease score. An agent may easily be shown to be weakly encephalitogenic by showing that the induced encephalomyelitis is monophasic, or by measuring time of onset or mean maximum disease score by using methods well known in the art (e.g. Oliver et al., 2003). In mice for example, time of onset may be assessed by comparing the time of onset of EAE caused by the agent suspected as "weakly encephalitogenic" with the time of onset of EAE caused by agents known to be highly encephalitogenic or weakly encephalitogenic and showing that the time of onset is after that of the highly encephalitogenic agent and approximately simultaneously with that of the weakly encephalitogenic agent. For example, a weakly encephalitogenic agent may cause the onset of EAE to occur on day 18 on average with a range of 11 to 28 days after initial immunization with the agent. Furthermore, the mean maximum disease score of the allegedly weakly encephalitogenic agent can similarly be assessed by comparing it with mean maximum disease score of known highly encephalitogenic or weakly encephalitogenic agents and showing that the mean maximum disease score of the allegedly weakly encephalitogenic agent is lower than that of the known highly encephalitogenic agent and similar to the known weakly encephalitogenic agent. Thus, the mean maximum disease score of the allegedly weakly encephalitogenic agent may be around 1.7 with a range of 0.5-2.5.

As mentioned above, it has been found in accordance with the present invention that reestablishment of a Th1/Th2 phenotype typical for young animals at the choroid plexus by injection of a non-encephalitogenic Th1 adjuvant (in this case CpG) into aging healthy animals improves their cognitive abilities. It has also been found herein that administration of CpG augments the immune system's natural capability of treating injury and neurodegeneration in the CNS by upregulating both trafficking and neurotropic molecule at the choroid plexus and thus increasing leukocyte recruitment to the injured spinal cord or neurodegenerative brain in ALS. It seems as if the neurodegenerative disease is not capable in itself of activating the choroid plexus and therefore a therapeutic window of opportunity exists that can be realized by the administration of a non-encephalitogenic Th1 adjuvant such as CpG that efficiently activates the choroid plexus and facilitates access of beneficial immune cells to the diseased CNS.

It has further been found, in accordance with the present invention, that vaccinating mSOD1 mice with the NS-specific antigen MOG₃₅₋₅₅ causes elevation in the number of regulatory T cells in the spinal cord of the treated mice relative to untreated mice. Further, these mice had a longer life span compared to untreated mice (Example 17).

The vaccination with the NS-specific antigen elicited a Th1 immune response that induced the expression of trafficking molecules by the CP (Example 17) enabling infiltration of leukocytes to the cerebrospinal fluid (CSF), and initiated an active programmed immune response that began with inflammation, and was followed by an anti-inflammatory resolution process. This response was manifested by transient symptoms of encephalomyelitis that were correlated spatially and temporally with an increase in regulatory T cells in the spinal cord of the immunized mSOD1 mice, leading to a significant increase in survival of these mice compared with unimmunized mSOD1 mice.

The method of the present invention may increase the life span of a subject with amyotrophic lateral sclerosis.

In certain embodiments, the method of the present invention is beneficial for improving CNS motor and/or cognitive function, for example for alleviating age-associate loss of cognitive function, either in diseased subjects or subjects free of a diagnosed disease. The method is also efficacious for alleviating loss of cognitive function resulting from acute stress.

In certain embodiments, the cognitive function is learning, memory or both. In other embodiments, the memory is hippocampal-dependent memory, explicit memory, episodic free recall, recollection of source of remembered fact, memory of details of context in which an event occurred, or working memory.

The term "CNS function" as used herein refers to CNS motor functions, such as controlling motor function and auditory and visual responses, maintaining balance and equilibrium, movement coordination, the conduction of sensory information and controlling such autonomic functions as breathing, heart rate, and digestion, and to CNS cognitive functions, *inter alia,* receiving and processing sensory information, thinking, learning, memorizing, perceiving, producing and understanding language.

The term "treating" as used herein refers to means of obtaining a desired physiological effect. The effect may be therapeutic in terms of partially or completely curing a disease and/or symptoms attributed to the disease. The term refers to inhibiting the disease, i.e. arresting its development; or ameliorating the disease, i.e. causing regression of the disease.

Methods of administration include, but are not limited to, parenteral, e.g., intravenous, intraperitoneal, intramuscular, subcutaneous, mucosal (e.g., oral, intranasal, buccal, vaginal, rectal, intraocular), intrathecal, topical and intradermal routes. Administration can be systemic or local.

The term "carrier" refers to a diluent, adjuvant, excipient, or vehicle with which the active agent is administered. The carriers in the pharmaceutical composition may comprise a binder, such as microcrystalline cellulose, polyvinylpyrrolidone (polyvidone or povidone), gum tragacanth, gelatin, starch, lactose or lactose monohydrate; a disintegrating agent, such as alginic acid, maize starch and the like; a lubricant or surfactant, such as magnesium stearate, or sodium lauryl sulphate; and a glidant, such as colloidal silicon dioxide.

The determination of the doses of the active ingredient to be used for human use is based on commonly used practices in the art, and will be finally determined by physicians in clinical trials. An expected approximate equivalent dose for administration to a human can be calculated based on the *in vivo* experimental evidence disclosed herein below, using known formulas (e.g. Reagan-Show et al. (2007) Dose translation from animal to human studies revisited. The FASEB Journal 22:659-661). According to this paradigm, the adult human equivalent dose (mg/kg body weight) equals a dose given to a mouse (mg/kg body weight) multiplied with 0.081.

The invention will now be illustrated by the following non-limiting examples.

### EXAMPLES

### Materials and Methods

**Animals.** Inbred male 3 month old C57BL/6 mice were supplied by the Animal Breeding Center of the Weizmann Institute of Science. Inbred male 17-24 months old C57BL/6 mice were supplied by the National Institute on Aging (NIA; Baltimore, MD). IFNγR1-KO mice on the B6 background were purchased from the Jackson Laboratory (Bar Harbor, Maine). Aged mice were allowed a 1-month adaptation period following shipment from the NIA to our laboratory. The cages were placed in a light- and temperature-controlled room, and all behavioural tests were conducted during the dark hours. All animals were handled according to the regulations formulated by the Weizmann Institute's Animal Care and Use Committee, and maintained in a pathogen free environment.

Adult male and female wild-type (WT) and CX₃CR1^{GFP/+} mice on a C57BL/6J background, and IL-4-IRES-eGFP reporter mouse model on a NOD background were supplied by Harlan Biotech (Jerusalem, Israel), and the Animal Breeding Center of The Weizmann Institute of Science. IFN-γR1-KO mice on a C57BL/6J background were purchased from the Jackson Laboratory (Bar Harbor, Maine). All experiments conformed to the regulations formulated by the Institutional Animal Care and Use Committee of the Weizmann Institute of Science.

**Preparation of bone marrow chimeras.** Bone marrow (BM) chimeras were prepared by subjecting gender-matched recipient mice to lethal whole-body γ-irradiation (950 rad) while shielding the head. The mice were then reconstituted with 5×10⁶ BM cells. In this method, 70% chimerism is achieved. In order to attain full chimerism (99%), mice were subjected to a split-dose irradiation, with sub-lethal whole-body γ-irradiation (300 rad) without head shielding 3 days prior to the lethal (950 rad) irradiation. Chimeric mice were used 6-10 weeks after BM transplantation.

Adult male and female wild-type (WT) and mSOD1^{G93A} (mSOD1) mice on a C57BL/6J background were supplied by Harlan Biotech (Jerusalem, Israel), and the Animal Breeding Center of The Weizmann Institute of Science. All experiments conformed to the regulations formulated by the Institutional Animal Care and Use Committee of the Weizmann Institute of Science.

**Flow cytometric cell sample preparation and analysis.** Prior to tissue collection, mice were intracardially perfused with phosphate buffered saline (PBS), and their blood was collected into heparin-containing tubes. Spleens were mashed with the plunger of a syringe and treated with ACK lysing buffer to remove erythrocytes. Choroid plexus tissues were isolated from the lateral, third and fourth ventricles of the brain, incubated at 37°C for 45 minutes in PBS (with Ca²⁺/Mg²⁺) containing 400u/ml collagenase type IV (Worthington Biochemical Corporation), and then manually homogenised by pipetation. Lymph nodes were mashed with the plunger of a syringe. Spinal cords were homogenized using a software controlled sealed homogenization system (Dispomix; http://www.biocellisolation.com) followed by separation on a 40% Percoll (GE Healthcare, Sweden) gradient to eliminate residual fat tissue. All samples were stained according to the antibody manufacturers' protocols.

For intracellular staining of IFN-γ or IL-17, the cells were incubated with PMA (10 ng/ml; Sigma-Aldrich) and ionomycin (250 ng/ml; Sigma-Aldrich) for 6 h, and Brefeldin-A (10 µg/ml; Sigma-Aldrich) was added for the last 4 h. Intracellular labeling of cytokines was done with BD Cytofix/Cytoperm Plus Fixation/Permeabilization kit (cat. no. 555028) according to the manufacturer's protocol. Intracellular staining of FoxP3 was performed using the FoxP3 staining kit (eBioscience) according to the manufacturer's protocol. The following fluorochrome-labeled mAbs were used according to the manufacturers' protocols: FITC-conjugated anti-TCRβ, FITC-conjugated anti-CD44, PE-conjugated anti-CD4, PE-conjugated anti-IL-17A, APC-conjugated anti-IFN-γ, FITC-conjugated anti-CD45.2, Alexa-700-conjugated anti-CD45.2, Percp-Cy5.5-conjugated anti-TCRβ (all from BioLegend), and APC-conjugated anti-FoxP3 (eBioscience). Flow cytometry analysis was performed on each sample using a BD Biosciences LSRII flow cytometer, and the acquired data were analyzed using FlowJo software (Tree star). In each experiment, relevant negative control groups and single stained samples for each tissue were used to determine the populations of interest and to exclude others.

**Active immunization and cell isolation.** Mice were immunized subcutaneously at their flanks with either spinal cord homogenate (SCH) or ovalbumin (OVA), each emulsified in an equal volume of CFA containing 2 mg/ml *M. tuberculosis.* SCH was prepared by manually homogenizing the spinal cord of young C57BL/6 mice in PBS. Mice were intracardially perfused with PBS 7 days after immunization, and their spleens, bone marrow and choroid plexus (CP) were extracted and processed to single cell suspensions as described above. CD4⁺ cells were isolated by magnetic depletion of CD4⁺ T cells on a MACS column (Milteney Biotec), according to the manufacturer's protocol.

**Active immunizations with MOG peptide and evaluation of EAE clinical signs.** 200 µg of either MOG₃₅₋₅₅ (amino acid sequence - MEVGWYRSPFSRVVHLYRNGK; SEQ ID NO: 1) or ovalbumin peptides₃₂₃₋₃₃₉ (GL Biochem (Shanghai) Ltd.) were emulsified in incomplete Freund's adjuvant containing 0.5 mg/ml *M. tuberculosis* (strain H37Ra; BD Diagnostics) and 200 µl were injected subcutaneously to the mice. To induce chronic EAE, mice were subcutaneously injected with 200 µg (300 µl) of MOG₃₅₋₅₅ (GL Biochem (Shanghai) Ltd.) in incomplete Freund's adjuvant containing 2.5 mg/ml *M. tuberculosis* (strain H37Ra; BD Diagnostics). Pertussis toxin (300 ng; Sigma-Aldrich) was injected on the day of the immunization and again 2 days later. Clinical signs were evaluated in a blinded fashion by at least 2 investigators and recorded daily (0, healthy; 1, limb tail paralysis; 2, ataxia and/or paresis of hind limbs; 3, paralysis of hind limbs and/or paresis of forelimbs; 4, tetraparalysis; 5, moribund state or death).

**CSF collection.** CSF was collected by the cisterna magna puncture technique. In brief, mice were anesthetized and placed on a stereotactic instrument so that the head formed a 135° angle with the body. A sagittal incision of the skin was made inferior to the occiput and the subcutaneous tissue and muscle were separated, and a capillary was inserted into the cisterna magna through the dura matter lateral to the arteria dorsalis spinalis. Approximately 15µl CSF could be aspirated from an individual mouse. The collected CSF was taken for analysis by flow cytometry.

**cDNA library preparation for TCRβ sequencing.** Total RNA was extracted from CD4⁺ T-cells, derived from naive spleen and choroid plexus tissues of C57BL/6 mice, as well as from spleens of OVA-immunized and SCH-immunized mice. RNA was extracted using RNeasy Mini Kit (Qiagen) and reverse transcribed with SuperScript™ II reverse transcriptase (Invitrogen), using a primer specific for the TCRβ constant region, linked to the Solexa 3' adapter (Cβ-3'adp). cDNA was then used as template for high fidelity PCR amplification (Phusion, Finnzymes) using a pool of 23 Vβ-specific primers, divided into five primer groups, to minimize potential for cross hybridization. Each Vβ primer was linked to a restriction site sequence for the ACUI restriction enzyme (NEB). PCR reactions were performed in duplicates, and PCR products were then pooled and cleaned using QIAquick PCR purification kit (Qiagen), followed by enzymatic digestion, in accordance with the ACUI protocol (NEB). The ACUI enzyme was used to cleave the 14 bp downstream of its binding site, enabling positioning of the Illumina sequencing primer in close proximity to the junction region and assuring sequencing of the entire variable CDR3 region. Digestion produced a 2 bp overhang for the ligation of the Illumina 5' adapter, which was linked to a 3 bp barcode sequence at its 3' end. Overnight ligation was performed using the T4 ligase (Fermetas) at 16°C in accordance with the manufacturer's protocol. A second round of PCR amplification was performed (24 cycles), using primers for the 5' and 3' Illumina adapters. Final PCR products were run on a agarose gel (2%) and purified using the Wizard SV Gel and PCR clean-up System (Promega). Final library concentrations were measured using a nanodrop spectrophotometer. The libraries were sequenced using the Illumina instrument (Genome Analyzer II).

**Computational analysis of the TCRβ sequencing data.** For preprocessing of the data, we used the Smith-Waterman alignment algorithm (Smith TF & Waterman MS (1981)) to assign to each sequencing read its variable (Vβ) and joining (Jβ) gene, using germline Vβ/Jβ gene segment sequences downloaded from the IMGT database (Lefranc MP, et al. (2009)). Reads that were not assigned either a Vβ or Jβ, and other erroneous reads were eliminated. We then clustered the library-derived reads using a version of the quality threshold clustering algorithm (Heyer LJ et al. (1999)), in order to correct for nucleotide copying errors (up to 2 errors for each read). The clustering procedure identified unique CDR3β clonotypes - defined as the most prevalent read found in each cluster. The clonotype sequences were then translated, and those clonotypes that lacked a stop codon in frame with the V/D/J sequences were considered for further analysis. This analysis computed statistics of V/D/J usage, statistical properties of the number of deletions and insertions of nucleotides at both VD and DJ junctions, as well as distributions of CDR3 lengths. The analysis was done using the R statistical software package (R Development Core Team, R). Frequencies of Vβ and Jβ segment usage were measured for all samples in each treatment group. Correlation coefficients were calculated, based on the sample mean of combined Vβ and Jβ usage in each group, using the data analysis program *MATLAB*™ (Mathworks, Natick, MA). Hierarchical clustering was performed, based on combined Vβ and Jβ usage, in all groups (clustergram, *MATLAB*™).

**Primary culture of choroid plexus cells.** Mice were perfused from the left ventricle of the heart with PBS, and their CPs were removed under a dissecting microscope (Stemi DV4; Zeiss) in PBS into tubes containing 0.25% trypsin, and kept on ice. The tubes were shaken for 20 minutes at 37°C and the tissue was dissociated by pipetting. The cell suspension was washed in culture medium for epithelial cells (DMEM/HAM's F12 (Invitrogen Corp)) supplemented with 10% FCS (Sigma-Aldrich), 1 mM 1-glutamine, 1 mM sodium pyruvate, 100 U/ml penicillin, 100 mg/ml streptomycin, 5µg/ml Insulin, 20 µM Ara-C, 5ng/ml sodium selenite, 10ng/ml EGF, and cultured (-250,000 cells/well) at 37°C, 5% CO2 in 24-well plates (Corning Incorporated) coated with poly-L-lysine (PLL; Sigma-Aldrich). After 24 hours, the medium was changed, and the cells were either left untreated, or treated with the indicated cytokines (all purchased from PeproTech): IFN-γ, IL-4, IL-10, IL-17A, GM-CSF, IL-6, IL-1β or TNF-α, or their combinations for 24 h. Cell viability was quantified by Trypan blue staining after detachment of the cells with 0.25% trypsin for 10 minutes at 37°C. Neutralizing antibodies to TNF-R1 or TNF-R2 (20µg/ml, BioLegend) were added 1 h prior to the addition of TNF-α, (100 ng/ml). RNA was isolated using the ZR RNA microprep kit (Zymo Research) according to the manufacturer's protocol.

**Transepithelial migration assay.** For transepithelial migration assay, CP cells were isolated as above and plated on 5-µm pore size, 6.5 mm diameter polycarbonate filters in 24-well Transwell chambers (Costar, Corning) and grown for 7 days. The medium of the upper chamber was then replaced with 100µl of freshly isolated CD115⁺ monocytes (1.5 x 10⁶ cells/ml in RPMI 1640 supplemented with 0.5% BSA) and the inserts were transferred to new wells containing RPMI 1640 supplemented with 0.5% BSA and 100ng/ml CCL2 (PeproTech). The Transwells were then incubated at 37°C, 5% CO₂. After 24 h the migrating cells at the lower chamber were removed using cell scraper and CD11b⁺ cells were quantified by flow cytometry.

**Immunohistochemistry and immunocytochemistry.** For whole mount staining of the choroid plexus, isolated tissues were fixed with 2.5% paraformaldehyde (PFA) for several hours, and subsequently transferred to PBS containing 0.05% sodium azide. Prior to staining, the dissected tissues were washed and blocked (20% horse serum, 0.3% Triton X-100, and PBS) for 1h at room temperature with shaking. Whole mount staining with primary (in PBS containing 2% horse serum and 0.3% Triton X-100) and secondary antibodies (in PBS), was performed for 1h at room temperature, with shaking. Each step was followed by three washes in PBS. The tissues were mounted onto slides, using Immu-mount (9990402, from Thermo), and sealed with cover-slips. For staining of sectioned brains, two different tissue preparation protocols (paraffin embedded and microtomed frozen sections) were applied, as previously described (Ziv Y et al (2006b)). For immunocytochemistry, CP cells were isolated as described above and were grown on PLL coated cover slips for 7 days, replacing the medium every 3 days. Cytokines were added for the last 3 days of culture. Following 7 days of culture, the wells were washed with PBS and the cells were fixed either with 2.5% paraformaldehyde for 30 min or methanol-acetone (1:1) for 10 min at -20°C, following by 2 washing steps with PBS. For CCL11 staining, cells were treated with Brefeldin-A 2 hours prior to fixation in order to arrest protein secretion. The cover slips of the cultured CP cells were incubated with the indicated antibodies and mounted with Glycerol Vinyl Alcohol (GVA) mounting solution (Invitrogen). The following primary antibodies were used: rat anti-CD3 (Abcam); rat anti-ICAM-1 (1:100; Abcam), chicken anti-vimentin (1:500, Millipore), rabbit anti-Ibal (1:300; Wako), goat anti-IL-10 (1:20; R&D), mouse anti-MHC-II (Abcam); mouse anti-Arginase-1 (BD Biosciences); mouse anti-Cytokeratin (Covance); rat anti-CCL11 (R&D Systems); rabbit anti-ZO-1 (Invitrogen); and rabbit anti-Claudin-1 (Invitrogen). Secondary antibodies included: Cy2/Cy3-conjugated donkey anti-rat, mouse or rabbit antibody and Cy3 conjugated donkey anti-mouse, goat, rat, chicken or rabbit (1:200; all from Jackson Immuno Research). The slides were exposed to Hoechst stain (1:2000; Invitrogen) for 1 min. Two negative controls were routinely used in immunostaining procedures, staining with isotype control antibody followed by secondary antibody, and staining with secondary antibody alone. For morphological quantification, average cross-sectional area of epithelial cells was measured following Nissl staining. Cell borders (20-25 cells per picture) were marked and the area was quantified using Image-Pro Plus software in a blinded fashion.

**Mutiplex cytokine analysis system.** CP were isolated from lateral, 3rd and 4th ventricles and pooled in groups of four, due to the limited amount of protein extracted from a single CP. The excised tissues were homogenized in PBS containing protease inhibitors (1:100; P8340, Sigma). Four freeze-thaw cycles (3 minutes each) were performed to break the cell membranes. Homogenates were then centrifuged for 10 min at 500g, and the total protein quantities in supernatants were determined by Bradford reagent. Frozen supernatants were assayed in duplicate using *Multiplex* bead-based *Luminex* Assay (MILLIPLEX mouse cytokine/chemokine panel; Millipore), performed by outsourcing (American Medical Laboratories) according to the manufacturer's instructions. Results are expressed as picograms of protein per milligram of total tissue protein.

**RNA purification, cDNA synthesis and real-time quantitative PCR.** Total RNA of the hippocampus was extracted with TRI reagent (MRC, Cincinnati, OH) and was purified from the lysates and spinal cord using the RNeasy kit (Qiagen, Hilden, Germany). Total RNA of the choroid plexus was extracted using the RNA MicroPrep kit (Zymo Research). mRNA (1 µg) was converted to cDNA using High Capacity cDNA Reverse Transcription Kit (Applied Biosystems). The expression of specific mRNAs was assayed using fluorescence based real-time quantitative PCR (qPCR). qPCR reactions were performed using Power SYBR Green PCR Master Mix (Applied Biosystems). Quantification reactions were performed in triplicates for each sample using the standard curve method. Glyceraldehyde 3-phosphate dehydrogenase (GAPDH), Peptidylprolyl isomerase A (PPIA), and hypoxanthine guanine phosphoribosyltransferase (HPRT) were chosen as reference genes according to their stability in the target tissue. The amplification cycles were 95°C for 5 sec, 60°C for 20 sec, and 72°C for 15 sec. At the end of the assay, a melting curve was constructed to evaluate the specificity of the reaction. For some genes, the cDNA was pre-amplified in 14 PCR cycles with non-random PCR primers, thus increasing the sensitivity of the subsequent real-time PCR analysis (PreAmp Master Mix Kit, Applied Biosystems). For these genes, expression was determined using TaqMan Real-Time PCR, according to manufacturer's instructions (Applied Biosystems). All quantitative real-time PCR reactions were performed and analyzed using the 7500 Real-Time PCR System (Applied Biosystems). The following TaqMan probes were used: Mm02342430_g1 (*ppia*), Mm00446968_m1 (*hprt*)*,* Mm00445260_m1 (*il-4*)*,* Mm01168134_m1 (*ifn-γ*), Mm00441238_m1 (*ccl11*), Mm00475988_m1 (*arg-1*). In addition, the following primers were used:
*arg-1* forward, 5'-AAGACAGGGCTCCTTTCAG-3' (SEQ ID NO: 6) and reverse 5'-TGTTCACAGTACTCTTCACCT-3' (SEQ ID NO: 7);
*bdnf1* forward, 5'-CCTGCATCTGTTGGGGAGAC-3' (SEQ ID NO: 8) and reverse 5'-GCCTTGTCCGTGGACGTTTA-3' (SEQ ID NO: 9);
*bdnf-5* forward 5'- GCGCCCATGAAAGAAGTAAA-3' (SEQ ID NO: 10) and reverse 5'- TCGTCAGACCTCTCGAACCT-3' (SEQ ID NO: 11);
*ccl2* forward 5'-CATCCACGTGTTGGCTCA-3' (SEQ ID NO: 12) and reverse 5'-GATCATCTTGCTGGTGAATGAGT-3' (SEQ ID NO: 13);
*ccl5* forward 5'-GTGCTCCAATCTTGCAGTCGTGTT-3' (SEQ ID NO: 14) and reverse 5'-ACTTCTTCTCTGGGTTGGCACACA-3' (SEQ ID NO: 15);
*ccl11* forward, 5'-CATGACCAGTAAGAAGATCCC-3' (SEQ ID NO: 16) and reverse 5'-CTTGAAGACTATGGCTTTCAGG-3' (SEQ ID NO: 17);
*ccl20* forward 5'-TGCTATCATCTTTCACACGA-3' (SEQ ID NO: 18) and reverse 5'-CATCTTCTTGACTCTTAGGCTG -3' (SEQ ID NO: 19);
*ccl22* forward 5'-CTCTGATGCAGGTCCCTATG-3' (SEQ ID NO: 20) and reverse 5'-TTTGAGGTCCAGAGAAGAACTCC -3' (SEQ ID NO: 21);
*cxcl9* forward 5'-GAGTTCGAGGAACCCTAGTG-3' (SEQ ID NO: 22) and reverse 5'-AACTGTTTGAGGTCTTTGAGG-3' (SEQ ID NO: 23);
*cxcl10* forward 5'-AACTGCATCCATATCGATGAC-3' (SEQ ID NO: 24) and reverse 5'-GTGGCAATGATCTCAACAC-3' (SEQ ID NO: 25);
*cxcl11* forward 5'-CTTCTGTAATTTACCCGAGTAACG-3' (SEQ ID NO: 26) and reverse 5'- TTCTATTGCCTGCATTATGAGG -3' (SEQ ID NO: 27);
*Fractalkine* (*Cx₃cl1*) forward 5'-ATGTGCGACAAGATGACCTCACGA-3' (SEQ ID NO: 28) and reverse 5'-TTTCTCCTTCGGGTCAGCACAGAA-3' (SEQ ID NO: 29);
*gapdh* forward, 5'-AATGTGTCCGTCGTGGATCTGA-3' (SEQ ID NO: 30) and reverse 5'-GATGCCTGCTTCACCACCTTCT-3' (SEQ ID NO: 31);
*h2-aα (MHC-II)* forward, 5'-ACCGTGACTATTCCTTCCA-3' (SEQ ID NO: 32) and reverse 5'-CAGGTTCCCAGTGTTTCAG-3' (SEQ ID NO: 33);
*ho1* forward, 5'-AGATGACACCTGAGGTCAAGCACA-3' (SEQ ID NO: 34) and reverse 5'-GCAGCTCCTCAAACAGCTCAATGT-3' (SEQ ID NO: 35);
*icam1* forward 5'-AGATCACATTCACGGTGCTGGCTA-3' (SEQ ID NO: 36) and reverse 5'-AGCTTTGGGATGGTAGCTGGAAGA-3' (SEQ ID NO: 37);
*igf2* forward 5'- CTGGAGGTGATGAGTGTAGCTCTGGC-3' (SEQ ID NO: 38) and reverse 5'- GAGTGACGAGCCAACACAGACAGGTC-3' (SEQ ID NO: 39);
*igf1* forward 5'- TGAGCTGGTGGATGCTCTTCAGTT -3' (SEQ ID NO: 40) and reverse 5'-TCATCCACAATGCCTGTCTGAGGT-3' (SEQ ID NO: 41);
*ifn-γr2* forward, 5'-TCCCACACCCATTCACAG-3' (SEQ ID NO: 42) and reverse 5'-AGGTCCAACAGTAACATTCTC-3' (SEQ ID NO: 43);
*il-1β* forward 5'- CCAAAAGATGAAGGGCTGCTT-3' (SEQ ID NO: 44) and reverse 5'-TGCTGCTGCGAGATTTGAAG-3' (SEQ ID NO: 45);
*Il-6* forward 5'-TGCAAGAGACTTCCATCCAGTTG-3' (SEQ ID NO: 46) and reverse 5'- TAAGCCTCCGACTTGTGAAGTGGT-3' (SEQ ID NO: 47);
*pgc1α* forward, 5'-GCCAAACCAACAACTTTATCTC-3' (SEQ ID NO: 48) and reverse 5'-GTTCGCTCAATAGTCTTGTTCTC-3' (SEQ ID NO: 49);
*ppia* forward, 5'-AGCATACAGGTCCTGGCATCTTGT-3' (SEQ ID NO: 50) and reverse 5'-CAAAGACCACATGCTTGCCATCCA-3' (SEQ ID NO: 51);
*Mcp-1* forward 5'-CATCCACGTGTTGGCTCA-3' (SEQ ID NO: 52) and reverse 5'-GATCATCTTGCTGGTGAATGAGT-3' (SEQ ID NO: 53);
*m-csf* forward 5'-CCACATGATTGGGAATGGAC-3' (SEQ ID NO: 54) and reverse 5'-GTAGCAAACAGGATCATCCA-3' (SEQ ID NO: 55);
*Madcam1* forward 5'-AGCACTCCGTGAAGATCCTTGTGT-3' (SEQ ID NO: 56) and reverse 5'-TAGCAGGGCAAAGGAGA GACTGTT-3' (SEQ ID NO: 57);
*tgf-β1* forward 5'-TACCATGCCAACTTCTGTCTGGG-3' (SEQ ID NO: 58) and reverse 5'-TGTGTTGGTTGTAGAGGGCAAGG-3' (SEQ ID NO: 59);
*tgfβ2* forward 5'-AATTGCTGCCTTCGCCCTCTTTAC-3' (SEQ ID NO: 60) and reverse 5'-TGTACAGGCTGAGGACTTTGGTGT-3' (SEQ ID NO: 61);
*tnf-α* forward 5'- ACAAGGCTGCCCCGACTAT -3' (SEQ ID NO: 62) and reverse 5'-CTCCTGGTATGAAGTGGCAAATC-3' (SEQ ID NO: 63);
*tnf-r1* forward 5'- CTTCATCAGTTTAATGTGCCGA-3' (SEQ ID NO: 64) and reverse 5'-AGCCTTCTCCTCTTTGACAG-3' (SEQ ID NO: 65);
*vcam1* forward 5'-TGTGAAGGGATTAACGAGGCTGGA-3' (SEQ ID NO: 66) and reverse 5'-CCATGTTTCGGGCACATTTCCACA-3' (SEQ ID NO: 67);

**Irradiation and bone-marrow transplantation.** Aged C57B1/6 wild-type mice were subjected to total body γ-irradiation from a cobalt source (a single dose of 950 rad), while protecting their heads to avoid radiation-induced brain damage. Transplanted BM was comprised of either whole BM, or BM depleted of T-cells, which was prepared by magnetic depletion of CD3⁺ cells on a MACS column (Milteney Biotec), according to the manufacturer's protocol. BM transplantation was performed on the following day, by i.v. injection of 5x10⁶ bone-marrow cells suspended in PBS (total volume 0.15 ml). For detection of BM cells following transplantation, cells were prelabeled with carboxyfluorescein succinimidyl ester (CFSE, Molecular Probes) prior to transplantation. For labelling, 5x10⁶ BM cells (20x10⁶cells/mL) were incubated in PBS (without Ca²⁺/Mg²⁺) supplemented with 5 µM CFSE (Molecular Probes) for 8 min at 25°C. Following incubation, the cells were washed with RPMI containing 8% FBS.

**Radial arm water maze (RAWM).** Mice were tested for two days on 6 radial arm water maze paradigm in a water pool, as described in Alamed et al., Nat Protoc. 2006, 1(4), 1671-1679. Briefly, mice received an injection of GA as indicated in the examples. On the first day, 15 trials were performed, in trials 1, 3, 5, 7, 9 and 11 the platform was visible, and in all other trials the platform was hidden. On the second day, 12 trials were performed, all of them with hidden platform. The time required to reach the platform and the number of errors (incorrect arm entries) in 1-min time period was recorded.

**Morris water maze.** Acquisition and probe trial were performed as previously described (Ron-Harel N, et al. (2008)). Following the probe trial, mice were given three additional trials without the platform to extinguish their initial memory of the platform's position. In the reversal phase, the platform was placed in a new location in the pool (opposite to where it was located in the acquisition phase) and the mice were given three trials per day on 2 consecutive days, conducted in a similar manner to the initial acquisition. Position and movement of the mice were recorded using an EthoVision automated tracking system (Noldus).

**Isolation of monocytes.** CD115⁺ monocytes were isolated as previously reported (Shechter *et al.* , 2009). Briefly, BM cells were harvested from the femur and tibiae of naive mice, and enriched for mononuclear cells on a Ficoll density gradient. The CD115⁺ BM monocyte population was isolated by MACS enrichment using biotinylated anti-CD115 antibodies and streptavidin-coupled magnetic beads (Miltenyi Biotec) according to the manufacturer's protocols. Monocyte purity was checked by flow cytometry based on CD11b reactivity and was 90%.

**Statistical Analysis.** The specific tests used to analyze each set of experiments are indicated in the figure legends. Data are expressed as mean±SEM. In the graphs, *y*-axes error bars represent the SEM. Statistical analysis was performed using Prism 5.0 software (GraphPad Software). Means between two groups were compared with two-tailed, unpaired *Student's t-test.* One-way ANOVA was used to compare several groups, and the Newman-Keuls test for pairwise comparisons was used for follow-up *post hoc* comparison of groups. Data from behavioral tests were analyzed using two-way repeated measured ANOVA with group treatment or age and number of days as the between subject factors. All histology and behavioral experiments were conducted in a randomized and blinded fashion. Fisher's LSD or Tukey-Kramer procedure was used for follow up pairwise comparison of groups after the null hypothesis had been rejected (F < 0.05). Kaplan-Meier survival curves were analyzed by Logrank test to generate an χ2 value for significance. Repeated-measures ANOVA was used for EAE scoring, with follow-up by Student's t test.

**Spinal cord injury and assessment of functional recovery.** The spinal cords of deeply anesthetized mice were exposed by laminectomy at T12, and contusive (200 kdynes) centralized injury was performed using the Infinite Horizon spinal cord impactor (Precision Systems), as previously described (Hauben *et al.* , 2000, Ziv *et al.* , 2006a), causing bilateral degeneration without completely severing the spinal cord. The animals were maintained on twice-daily bladder expression. Recovery was evaluated by hind-limb locomotor performance, assessed according to the open-field Basso Mouse Scale (BMS) (Basso et al., J. neurotrauma 23(5):635-59, 2006). In this scale, a nonlinear score ranging from 0 (complete paralysis) to 9 (normal mobility) is assigned, in which each possible score represents a distinct motor functional state. Blinded scoring ensured that observers were not aware of the identity of tested animals.

### Example 1: The choroid plexus is populated by effector memory CD4⁺ T cells

Using flow cytometry, we began by characterizing the T cell populations within the choroid plexus (CP). We found that, similarly to the lymph nodes, the majority (67±2.76%) of T cells found in the CPs were CD4⁺ cells (data not shown). Since CD4⁺ T cells (rather than CD8⁺ cells) were previously implicated in supporting CNS plasticity (Wolf SA, et al. (2009), Derecki NC, et al. (2010), Cao C, et al. (2009)) we further characterized this sub-population. We focused our interest on memory T cells, commonly divided into two subsets that express high levels of CD44, yet differ in their expression of CD62L; CD44^{high}/CD62L^{high} are central memory T cells (T_{CM}) that home to secondary lymphoid organs and generally have little to no effector function, while CD44^{high}/CD62L^{-/low} are effector memory T cells (T_{EM}) that survey peripheral tissues, and can be locally activated to an immediate effector function upon exposure to their cognate antigen. To examine the peripheral circulating immune cell population in comparison to that within the CP, we examined the blood, lymph nodes and CPs of young (3 month old) and aged (22 month old) mice. We found that while both T_{EM} and T_{CM} were present in the blood and lymph nodes, in the CP, the vast majority (>95%) of memory T cells expressed T_{EM} markers **(****Fig. 1****).** In aged mice, we observed an increase in frequency of T_{EM} cells in both the blood circulation and the lymph nodes, while their levels in the CP were unchanged **(****Fig. 1****).** Thus, examining the CP compartment revealed that unlike the cerebrospinal-fluid (CSF), previously demonstrated to be dominated by CD4+ T_{CM} cells (de Graaf MT, et al. (2011)), the CP specifically retains CD4+ T_{EM} cells. In addition, this compartment appears to maintain this strong T_{EM} bias throughout life. T cells were co-localized with the CP epithelium and adjacent to MHC-II expressing APCs (data not shown), indicating their interactions with APCs in the tissue. We therefore assumed that the T_{EM} cells that reside in the CP are constitutively activated by the APCs that present their cognate antigens. If this is the case, these T cells are likely to be specific for brain antigens. Thus, we next focused on identifying the specificity of the T cells that reside in the healthy CP and whether they undergo changes with age that could, to some extent, explain brain senescence.

### Example 2: The choroid plexus CD4⁺ TCR repertoire is enriched with CNS-specific clones.

Sequencing of the TCR by itself cannot identify its antigenic specificity. Therefore, to identify the specificity of the T cells that reside in the CP, we developed a novel tool that allowed us to identify clonotypic enrichment of CNS-specific T cells. To this end, we created a library representing the TCRβ repertoire of CNS-specific antigens. Mice were immunized either with spinal cord homogenate (SCH), containing a wide array of CNS proteins, or, as a control, with a non-self peptide antigen derived from ovalbumin (OVA). After 7 days, RNA isolated from splenic CD4⁺ T cells of both groups of immunized mice, as well as from a group of non-immunized mice, was analyzed using TCR-seq, a high throughput sequencing procedure of the TCRβ CDR3 region (Freeman JD et al. (2009); Robins HS, et al. (2009); Venturi V, et al. (2011)). We also used TCR-seq to analyze the TCRβ repertoire of CP-residing T cells. Using bioinformatics tools developed in-house (see the ***materials and methods*** section for a detailed description), full characterization of each CDR3 region was achieved from individual sequencing reads, identifying V-D-J usage as well as fully characterizing the junction regions, including random nucleotide insertions and deletions. Data obtained were further analyzed by a specially designed analysis pipeline, enabling extraction of reliable quantitative information on the TCRβ repertoire composition. This pipeline provided us with a list of annotated TCRβ sequences (nucleotide and amino-acid sequences), and their relative abundance, for each sample. Once we had established the TCRβ repertoire of the spleen of animals immunized with CNS antigens, we could compare it to the repertoire of T cells isolated from CP of non-immunized animals.

The TCRβ repertoire composition of the above groups was further compared using different parameters. We observed a high level of similarity in Vβ usage between the TCRβ repertoire found in CP of naive animals and that found in the spleens of the animals immunized with CNS antigens (SCH) (data not shown). In contrast, the CP repertoire had a much lower correlation with the repertoire found in spleens of naive mice or from animals immunized with OVA. The Jβ usage in the repertoire of the naive CP was also very similar to that of the SCH-immunized spleen cells, and less similar to the OVA-immunized or naive spleen (data not shown).

Next, we applied average linkage clustering on the different groups, based on the group average relative frequencies of all Vβ and Jβ segments (39 parameters overall). The naive CP formed a distinct cluster with the SCH-immunized spleen, while the OVA-immunized spleen clustered with the naive spleen (data not shown). Each cluster had specific enriched Vβ and Jβ segments associated with it, forming organ and immune state-specific repertoire signatures. Both correlation and clustering analyses revealed high levels of similarity in Vβ and Jβ segment usage between the repertoires of T cells residing in the naive CP and those of T cells isolated from the spleen after immunization with CNS antigens, yet a low similarity of the CP repertoire to that of the naive spleen, or the spleen after immunization with OVA. Finally, we examined the CP repertoire in terms of specific clones. From our TCR-seq data, we compiled a library of 2,756 TCRβ amino-acid sequences that were significantly induced in the SCH-immunized spleens compared with non-immunized spleens, and are therefore associated with SCH-specific clonal expansion. We then determined how many of the most highly expressed clones from each non-immunized or immunized tissue were found in the SCH-immunized spleen TCR library. The TCRβ repertoire of non-immunized young CP was found to be significantly enriched with CNS-specific T cells compared with the repertories found in OVA-immunized and in naive spleens **(****Fig. 2A****).**

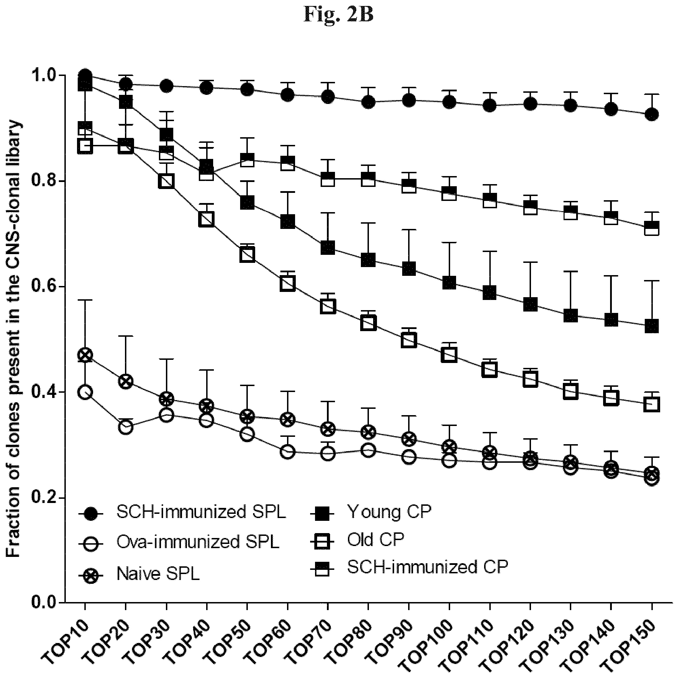

Having established a tool for identification of CNS-specific clonotypic enrichment, we next tested whether clonal specificity changes with age. We isolated T cells from CP and spleens of non-immunized aged mice and compared their repertoire to that of young non-immunized mice **(****Fig. 2B****).** Overall, the CP of non-immunized old animals maintained its clonotypic specificity to CNS antigens. However, the spleens of these aged mice were found to be more highly enriched with CNS-specific clones compared with spleens of non-immunized young mice **(****Fig. 2A****),** possibly reflecting the lifelong exposure to auto-antigens, believed to result in accumulation of auto-reactive clones with age (Linton PJ & Dorshkind K (2004)). Yet, comparing the relative numbers of CNS specific clones in the CP of aged mice relative to young mice, we found that although specificity to CNS antigens seemed to be maintained with aging, the abundance of CNS-specific clones was somewhat lower in the aged-CP relative to young CP **(****Fig. 2B****),** suggesting that overall CNS clonal abundance in the CP declines with age. Interestingly, the abundance of CNS-specific clones was elevated in the CP of young animals that were immunized with SCH in comparison to the non-immunized young CP **(****Fig. 2B****),** suggesting that the antigen specificity of the CP CNS-specific T cells has a dynamic range and is thus amenable to immunomodulation.

### Example 3: Th2-mediated inflammation of the CP epithelium during the aging process.

Our demonstration that the overall TCR specificity of the CD4⁺ T cells residing in the CP is maintained with age, led us to consider the possibility that aging of the CP may be associated not with changes in immune specificity, but rather with phenotype bias, such as changes in the cytokine milieu, a phenomenon established outside the CNS (Alberti S, et al. (2006); Shearer GM (1997); Rink L et al. (1998)). We therefore measured mRNA expression levels of the cytokines IFN-γ and IL-4 in the CP, representing Th1 and Th2 effector milieus, respectively. We found preferential elevation of IL-4 expression, and a decline in IFN-γ expression with aging **(****Fig. 3A****).** Outside the CNS, IL-4 was shown to induce the elevation of CCL11 (Bloemen K, et al. (2007)), a chemokine recently shown to play a part in age-related cognitive decline and to be elevated in the CSF and plasma of aged mice and humans (Villeda SA, et al. (2011)). Accordingly, we hypothesized that the age-related elevation of CCL11 found in the CSF, might be a product of the CP epithelium, resulting from Th2-mediated inflammation that develops in this compartment with aging.

To test our working hypothesis, we first examined whether the CP could serve as a source of CCL11 in aged animals. We found that CCL11 is expressed by the CP epithelium, and that its mRNA and protein levels in aged (18-20 months) mice were significantly higher than in CP of young (3 months) animals (**Fig. 3B and 3C**). Immunohistochemical analysis of the CP revealed that while CCL11-immunoreactivity was barely detected in young animals, it was highly abundant in the aged CP tissue (data not shown). To determine how IL-4 could affect CCL11 upregulation in the presence and absence of IFN-γ, we cultured CP epithelial cells from young mice with IL-4, IFN-γ, or their combination, and examined mRNA expression. When subjected to IL-4, the cultured young CP epithelium expressed high levels of CCL11 **(****Fig. 3E****).** Direct treatment of young CP epithelial cells with IFN-γ alone had no effect on CCL11 expression; however, addition of IFN-γ together with IL-4 reversed the effect of IL-4 on CCL11 production. CCL11 immunoreactivity, following Brefeldin-A treatment to block protein secretion and to thereby allow identification of intracellular cytokines, showed that the CP epithelium produced CCL11 in response to IL-4, and co-incubation with IFN-γ reversed this induction (data not shown). These results confirmed that although young CP epithelial cells hardly produce CCL11, their exposure to IL-4 upregulates CCL11 production, while IFN-γ can mitigate this effect. Accordingly, we tested whether CCL11 production by aged CP is amenable to down-regulation by IFN-γ. To this end, we cultured CP epithelial cells of young and old mice, and monitored their CCL11 expression in the presence or absence of IFN-γ. Similarly to our *in vivo* results (**Figs. 3B and C**), the basal levels of CCL11 in CP cultures from aged mice were higher than those in young animals **(****Fig. 3F****),** yet, the addition of IFN-γ decreased basal CCL11 expression in the cultured aged CP. In order to verify this IL-4/IFN-γ interplay at the CP *in vivo,* we examined this compartment in young IFN-γ receptor knock-out animals (IFNγR-KO) and found that when the receptor for this cytokine is absent, the basal expression levels of CCL11 are significantly higher (data not shown), further demonstrating the role of IFN-γ in balancing the Th2 response at the CP. Thus, it appeared that IL-4-induced CCL11 expression could be controlled by IFN-γ, both *in vivo* and *in vitro,* and that this regulation might be applicable to the aged CP, characterized here by a shift in its IL-4/IFN-γ ratio **(****Fig. 3A****).**

To further substantiate the dominance of IL-4 in the aged CP, we measured levels of arginase-1, an enzyme that is strongly linked to epithelial Th2-mediated inflammation; in cases of asthma, chronic obstructive pulmonary disease (COPD) and cystic fibrosis, increased arginase activity in the airways was shown to have detrimental effects on the airway epithelium. Moreover, chronic Th2-mediated inflammation of the airways can induce remodeling of the epithelium and contribute to a progressive decline in lung function. We found arginase-1 (arg1) mRNA and protein levels to be strongly upregulated in the aged CP **(****Fig. 3H****).** In addition, CP epithelial cell cultures upregulated arginase-1 in response to IL-4, mimicking the situation in the aged CP compartment, and the epithelial tight junctions were dysregulated (data not shown), as observed here by staining for ZO-1 (not shown), and as previously reported in the case of human lung epithelial cells. Histological examination of the CP revealed signs of hypertrophism in the aged CP epithelium **(****Fig. 3I****),** a phenomenon described in the murine lung epithelium in response to IL-4 (Rankin JA, et al. (1996)). Together, these data indicate that the changes in IL-4/IFNγ ratio in the CP of aged mice critically affect gene expression and morphology of the BCSFB, and may potentially explain the age-related cognitive decline that was observed in correlation with elevated CCL11 levels (Villeda SA, et al. (2011)). Since brain aging and impaired hippocampal plasticity have been associated with elevation of parenchymal proinflammatory cytokines (Dantzer R et al. (2008); Monje ML et al. (2003); Ekdahl CT et al. (2003)) such as IL-1β, IL-6 and TNF-α, we assumed that the CP of aged mice is exposed to such proinflammatory cytokines. Testing the aged CP for these proinflammatory cytokines revealed the elevated protein levels of IL-1β and IL-6 (data not shown), suggesting that the cytokine milieu of the aged parenchyma is signalling to the CP, thereby possibly contributing to its dysfunction.

### Example 4: Effects of syngeneic homeostatic-driven proliferation on the CP and hippocampus.

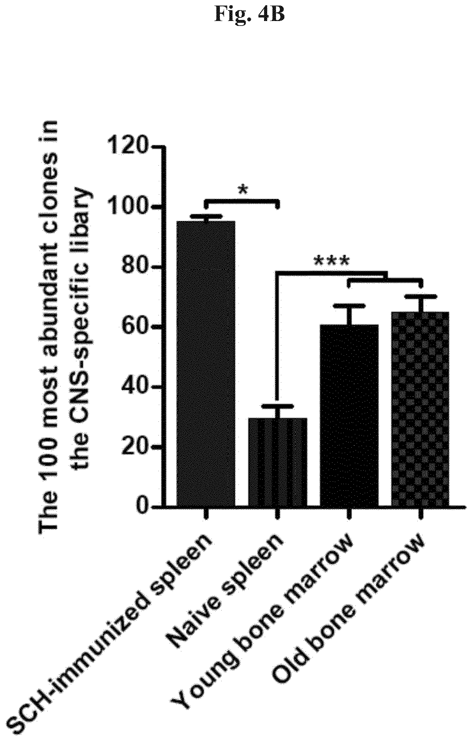

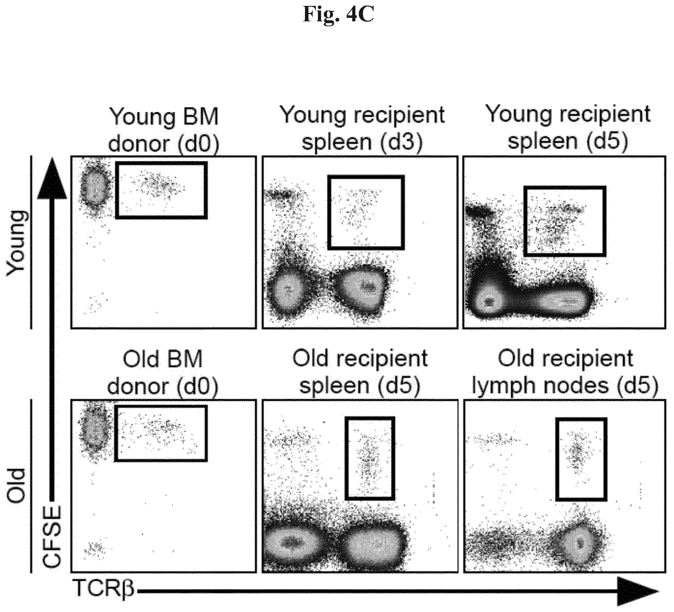

The increased levels of IL-4 in the CP of aged mice without proper balance by IFN-γ could reflect the well-characterized alternations in circulating immune cells during aging (Alberti S, et al. (2006); Shearer GM (1997); Rink L,et al. (1998)). One way by which immunesenescence can be alleviated and memory T cells can be expanded, is the induction of lymphopenia, which is followed by homeostatic-driven proliferation (HDP) of the residual T cell population. We therefore induced HDP by irradiation of the peripheral organs while shielding the head. Since we used high-dose irradiation and aimed to induce homeostatic-driven proliferation of memory T cells that already exist in the aged mice, the hematopoietic cell lineages were restored with syngeneic (pseudo-autologous) bone marrow (BM) derived from genetically identical donors of the same age. We expected that memory T cells that are present in the aged BM would provide an additional source of memory cells, for their expansion in the aged mice under these lymphopenic conditions. To this end, we first confirmed that aged BM contains memory T cells, and determined whether their specificity resembles that of the cells found in the SCH-immunized spleen, in old spleen, and in the naive CP. We found that, indeed, BM from aged mice contained significantly higher levels of CD4⁺ and CD4⁺/CD44^{high} cells relative to young animals **(****Fig. 4A****).** Testing the clonotypic repertoire revealed that T cells in the BM were significantly enriched for CNS specificity **(****Fig. 4B****).** We next verified that indeed, following BM transplantation, T cells from the aged BM repopulated the lymphoid organs. To enable follow-up of the transplanted T cells, the donor BM cells were labeled with the intracellular fluorescent dye, CFSE, prior to transplantation. In both young and old recipients of age-matched BM, BM-derived T cells migrated to the spleen where they proliferated over time **(****Fig. 4C****);** these T cells could be detected in both the spleen and cervical lymph nodes, and demonstrated dilution of the CFSE label (indicating proliferation) on day 5 after transplantation. In previous experiments, using this procedure, we demonstrated cognitive improvement in aged animals (Ron-Harel N, et al. (2008)). Yet, as our protocol involved BM as our source for additional memory T cells beyond the irradiation remnants that undergo proliferation following the irradiation-induced lymphopenia, we verified that the cognitive improvement previously reported is attributable to these transferred pseudo-autologous T cells. To this end, HDP was induced in cognitively impaired aged mice that were either transplanted with aged-matched BM, or with the same BM depleted of T cells. The mice were tested again, 8 weeks later, in the Morris Water Maze (MWM) for their hippocampus-dependent spatial learning/memory, relative to young mice. As seen from Fig. 5, In both the acquisition **(****Fig. 5A****)** and the reversal phases of the test **(****Figs. 5B****,** C), the performance of aged mice transplanted with aged-BM depleted of T-cells (aged-BMT-T) was significantly impaired compared to young mice, whereas the performance of aged mice transplanted with whole BM (aged-BMT) was similar to that of the young mice. The conclusion is that old mice receiving BM depleted of T cells failed to re-establish learning/memory skills.

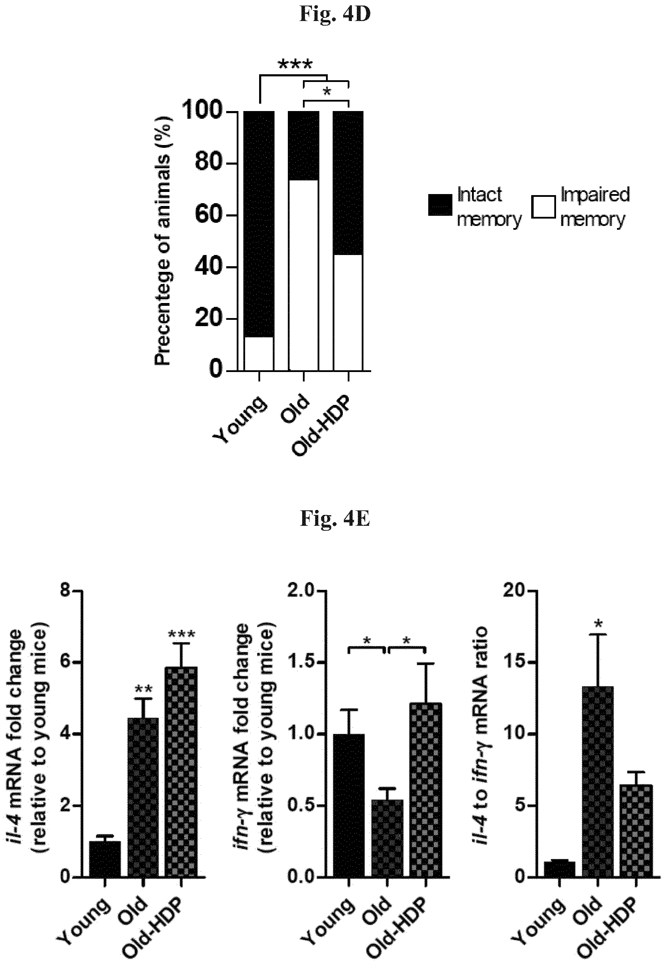

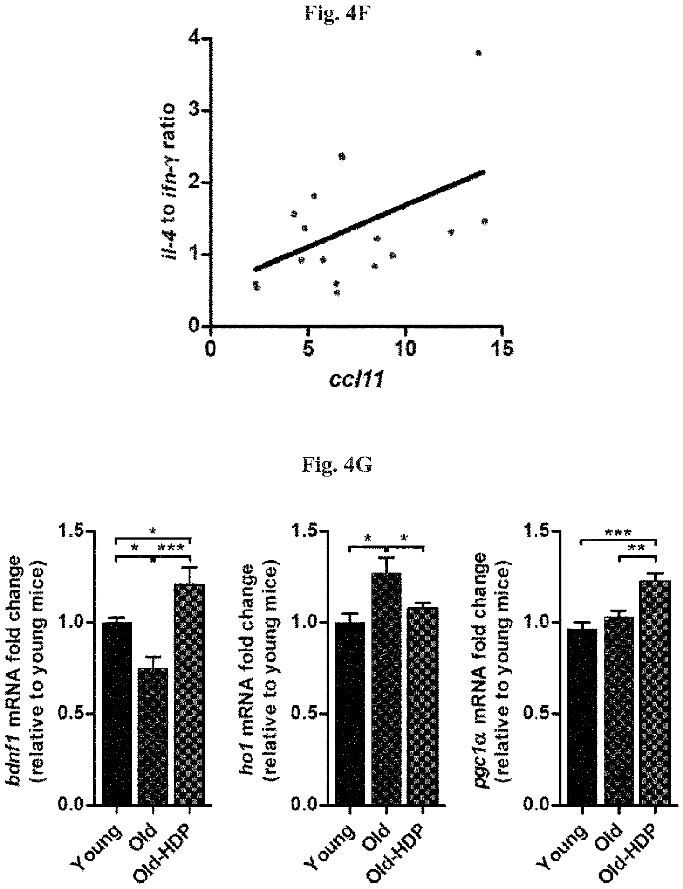

Based on the above results, we then adopted the same HDP procedure using pseudo-autologous aged BM (without depletion of T cells) to examine whether the peripheral immunomodulatory effects of this protocol, which leads to cognitive improvement, are related to the restoration of the IL-4/IFN-γ ratio at the CP. Aged mice were separated to two groups, one group was left untreated, whereas in the other HDP was induced using the procedure of pseudo-autologous aged BM. Before excising the CP and the hippocampi of the animals, we verified using MWM that the treatment was indeed beneficial at 8 weeks following transplantation. Similar to the reported observation that aging is associated with memory loss in -70% of aged mice (Whalley LJ et al. (2004)), in our experiments the proportion of old animals with impaired spatial learning/memory was 74%; this deficit was significantly lowered to 45% of the animals following HDP **(****Fig. 4D****).** The CP was subsequently excised and tested for IL-4 and IFN-γ expression. Indeed, the IL-4/IFN-γ ratio in the old animals following HDP was more similar to that found in young animals **(****Fig. 4E****).** The causal connection between IL-4/IFN-γ ratio and CCL11 levels, and thus the impact on cognitive ability, was further demonstrated by the positive correlation found between the IL-4/IFN-γ ratio and CCL11 levels in the CP of the old animals (treated and untreated) **(****Fig. 4F****).**

Since spatial learning/memory is hippocampus dependent, we next tested whether the HDP-induced changes in the CP cytokine balance were also reflected in expression of plasticity-related molecules in the hippocampi of these mice. We found that hippocampal brain-derived neurotrophic factor-1 (*bdnf1*) levels were down-regulated with age, and restored following HDP **(****Fig. 4G****).** BDNF is an important neurotrophin, and its deficiency has been repeatedly linked with age-associated loss of cognitive function (Hattiangady B et al. (2005); Kesslak JPet al. (1998)). In addition, we measured the expression of heme oxygenase-1 (ho1), previously linked to aging of the hippocampus (Nicolle MM, et al. (2001)), and found its upregulation in the aged group compared to the young animals. HDP reduced *ho1* in aged animals to its expression levels in young animals **(****Fig. 4G****).** We also examined whether rejuvenation of the CP cytokine balance was associated with elevation of PPARγ coactivator-la (PGC-1α), a key regulator in coping with reactive oxygen species (ROS) and a hippocampal neuroprotective mediator. Old animals after HDP had significantly higher levels of hippocampal PGC-1α, **(****Fig. 4G****),** possibly correlating with their improved cognitive ability.

### Example 5. Characterization of the naïve CP for its resident T-cell populations and epithelial cytokine receptors.

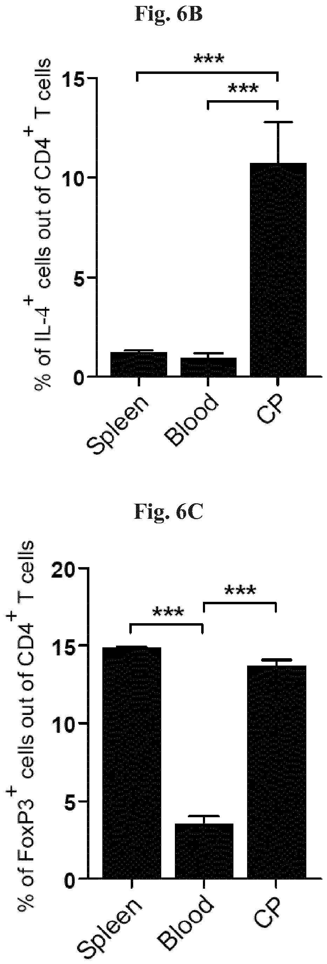

First, we localized the T cell populations that reside in the choroid plexus under normal conditions in wild type (WT) mice. As previously reported, CD3⁺ T-cells are found at the stroma of the CP epithelium. Using flow cytometry, we determined the effector potential of these T cells to secrete various cytokines, in comparison with these T cells from the spleen and the circulation. Intracellular staining of the CD4⁺ T cell populations showed that interferon (IFN)-γ producing T helper (Th)1 cells were present in the naive CP **(****Fig. 6A****).** To quantify the population of interleukin (IL)-4 producing Th2 cells, we took advantage of mice that express green fluorescent protein (GFP) under the IL-4 promoter (Mohrs et al., 2001), and found a population of CD4⁺ T cells in the CP that express IL-4 **(****Fig. 6B****).** We also stained the CD4⁺ T cells for FoxP3, a marker for regulatory T cells (Tregs) (Hori et al., 2003) and found that about 15% of the CD4⁺ cells in the CP were FoxP3 positive **(****Fig. 6C****).** Notably, the CP showed enrichment in all three populations of CD4⁺ T cells relative to the blood, suggesting the active accumulation of these T cells in this compartment. Of the total CD4⁺ T cells, the percentage of the Th1 and Th2 cell populations was greater in the CP than in the spleen, whereas the Treg population in the CP had the same abundance as in the lymphoid organs. Collectively, this data suggested that the CP epithelium is potentially exposed to a variety of T cell-derived cytokines.

Next, we examined the CP epithelium for the expression of relevant receptors for these cytokines. The CP epithelium abundantly expressed the cytokine receptors for IL-4 and IL-10, whereas the expression of IFN-γ receptor (IFN-γR) was barely detectable (data not shown). This initial observation suggested that while IL-4 activity, through its epithelial receptor on the CP, has a constitutive role in the maintenance of this compartment, the expression of IFN-γR is subjected to regulation. If this is indeed the case, expression of IFN-γR is likely to be induced by a parenchyma-emerging danger signal, possibly as the first step in the activation of the CP epithelium in response to CNS stress or damage.

### Example 6. Choroid plexus epithelial cells upregulate trafficking molecules in response to a specific cytokine milieu.

Since we found that the CP is constitutively exposed to T cell-derived cytokines, we hypothesized that such cytokines might have the potential to regulate the expression of trafficking molecules by the CP epithelium. We therefore established an *in vitro* model using primary cultures of murine CP cells. To verify the purity of the epithelial cultures, we isolated CP cells from mice that express GFP on their myeloid cells (CX₃CR1^{GFP/+} mice (Jung *et al. ,* 2000)), enabling us to detect any residual myeloid cell population within the epithelial cell cultures. Immunostaining of the CP cell cultures for the epithelial cytoskeletal marker, cytokeratin, revealed their uniformity (data not shown), and quantitative analysis of the immunostained cells showed that 97.8±0.4% of the cells expressed cytokeratin, and only 2.15±0.3% of the cells were of myeloid origin, as determined by GFP expression (measured by direct counting). Following 1 week in culture, the epithelial cells established tight junctions, as shown by immunostaining for the tight junction molecule, ZO-1 (data not shown).

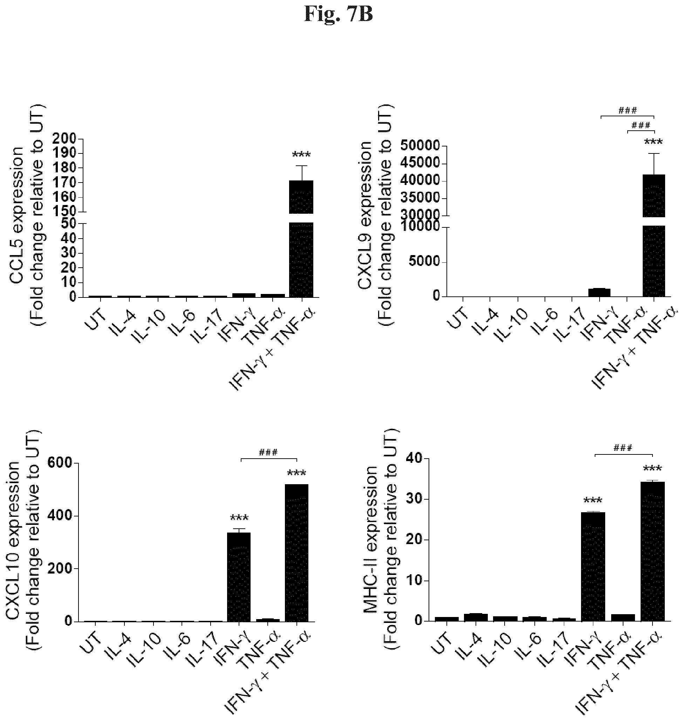

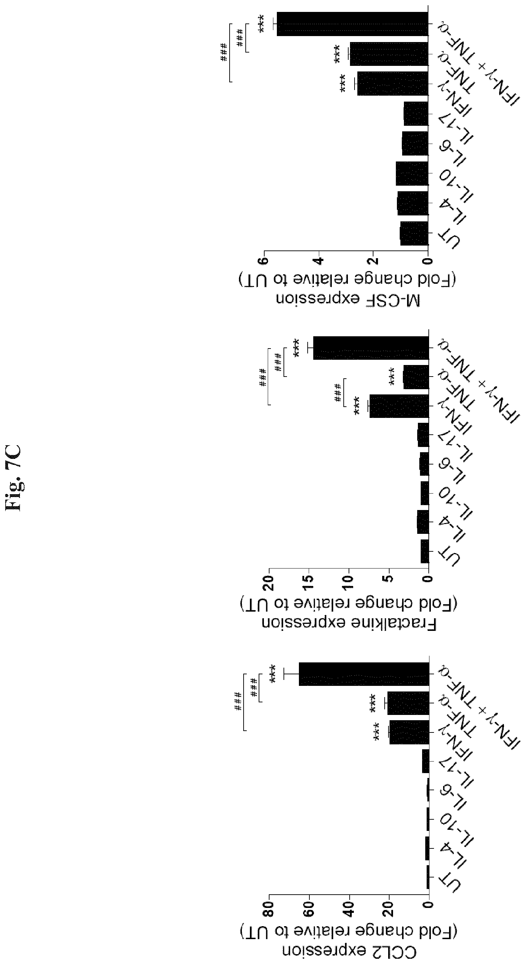

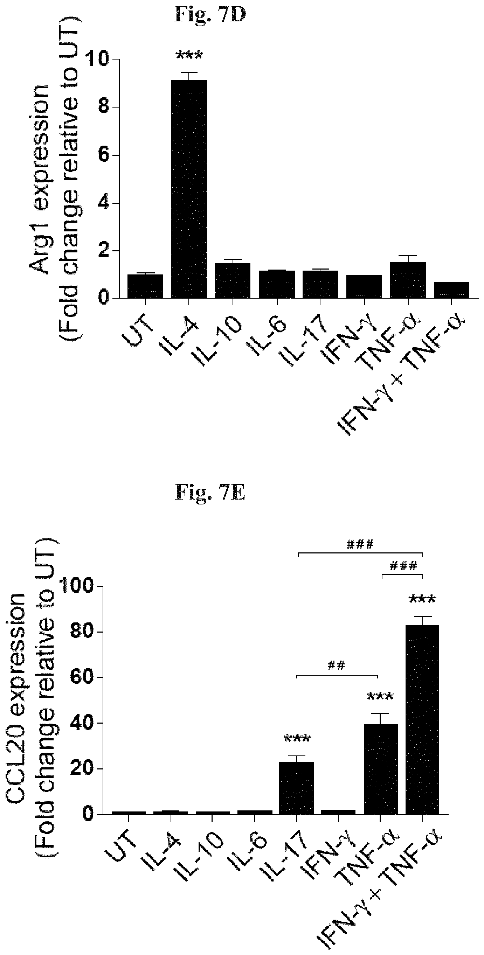

Pro-inflammatory cytokines, such as IL-6, IL-1β, and tumor necrosis factor (TNF)-α, that are elevated at the injured spinal cord (Pineau and Lacroix, 2007, Harrington *et al.,* 2005) can potentially affect the CP via the CSF circulation. We therefore tested whether cytokines released at the injury site would affect the CP in addition to the T-cell derived cytokines. We exposed the cultured CP cells to the characteristic Th1-, Th17-, Th2- and Treg-derived cytokines, IFN-γ, IL-17, IL-4, and IL-10, respectively, and to TNF-α, and IL-6. After 24 hours in culture, we examined the effect of these cytokines on the gene expression levels of specific trafficking molecules. Treatment with IFN-γ induced the upregulation of a wide array of trafficking molecules, such as intercellular adhesion molecule (ICAM)-1, vascular cell adhesion molecule (VCAM)-1 **(****Fig. 7A****),** the chemokines CCL5, CXCL9 and CXCL10, and major histocompatibility complex (MHC)-II **(****Fig. 7B****),** which can contribute to T cell trafficking across epithelial barriers and to T cell activation. The chemokines CCL2, Fractalkine (CX₃CL1) and macrophage colony-stimulating factor (M-CSF) were also upregulated by the CP in response to IFN-γ **(****Fig. 7C****).** When IFN-γ was combined with TNF-α, a strong synergistic effect was observed with respect to most of the tested genes. Notably, TNF-α, was the only cytokine that upregulated, in an IFN-γ independent manner, mucosal vascular addressin cell adhesion molecule (MADCAM)-1 expression **(****Fig. 7A****),** as previously reported. To further verify that the observed ability to induce trafficking molecules by the CP was unique to IFN-γ, we repeated the *in vitro* experiments using escalating dosing of the cytokines IL-4, IL-6, IL-10, GM-CSF and IFN-γ. None of the tested cytokines, other than IFN-γ, had any effect, while IFN-γ activated the CP in a dose dependent manner to express trafficking-related genes (data not shown). Although IL-4 had no effect on trafficking molecules, it had a dramatic effect on the upregulation of arginase-1 (Arg1) **(****Fig. 7D****),** an enzyme classically associated with anti-inflammatory activity.

Since inflammatory autoimmune diseases are associated with IL-17 producing cells (Th17) (Korn et al., 2009) this prompted us to test their potential effect on inducing trafficking molecules by the CP. Interestingly, we found that IL-17, but not IFN-γ, elevated the expression of the chemokine CCL20 by the CP epithelial cells **(****Fig. 7E****);** CCL20 was proposed to participate in CCR6⁺ cell trafficking through the CP in the initiation of EAE.

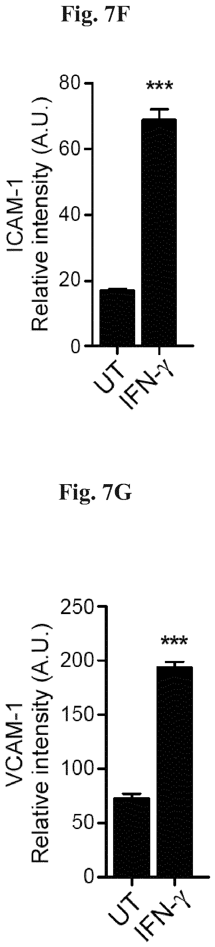

The localization of the induced integrin receptors, ICAM-1 and VCAM-1, was tested by co-immunostaining the cultured cells for these molecules together with epithelial markers; the integrin receptors were co-localized with the CP epithelial cells, and were elevated following treatment with IFN-γ **(****Fig. 7F, G****).** We also immunostained IL-4-treated CP epithelial cells for Arg1, and found it to be higher relative to untreated cells **(****Fig. 7H****).**

### Example 7. TNF-α and INF-y reciprocally control the expression of their receptors by the CP.

To gain insight regarding the mechanism underlying the synergism, described above, between TNF-α, and IFN-γ, we examined their reciprocal effects on the levels of their corresponding receptors *in vitro.* We found that the inflammatory cytokines TNF-α, and IL-1β, but not IL-6, elevated the expression of IFN-γ-receptor (IFN-γR) by the CP epithelial cells **(****Fig. 8A****).** Immunostaining of the cultured cells also showed elevation of IFN-γR following TNF-α, treatment (data not shown). Notably, TNF-α-producing CD4⁺ T cells were not found in the naive CP (data not shown).

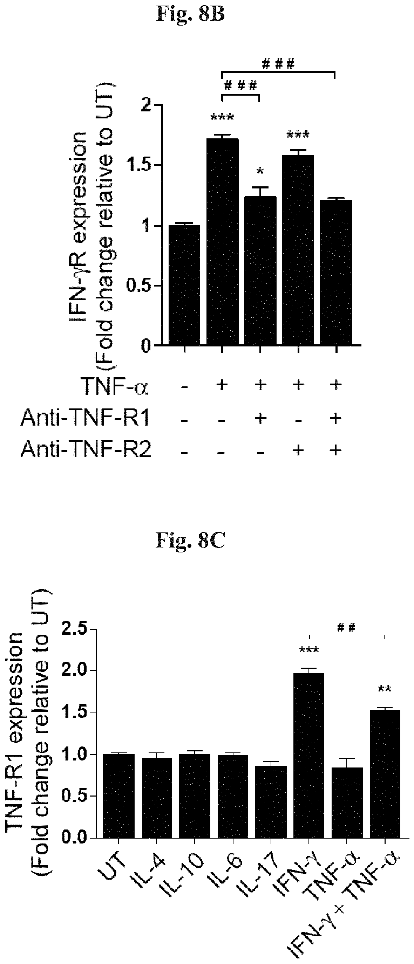

TNF-α, receptors (TNF-R) are differentially expressed in various tissues, and were shown to mediate both inflammation and repair of the CNS. We therefore used neutralizing antibodies directed against TNF-R1 or TNF-R2 to assess which TNF-R mediates the observed elevation of IFN-γR by the CP. We found that the induction of IFN-γR by the CP was mediated by TNF-R1 signaling, as only neutralizing antibodies directed against TNF-R1 impaired IFN-γR expression **(****Fig. 8B****).** Indeed, immunostaining confirmed the expression of TNF-R1 on the epithelial cells (cytokeratin⁺) of the CP of naive mice (data not shown). In addition to the elevated expression of IFN-γR following treatment with TNF-α, IFN-γ elevated the expression of TNF-R1 **(****Fig. 8C****),** suggesting that the synergistic effect between TNF-α, and IFN-γ by the CP epithelium is bidirectional, with each cytokine inducing the expression of its reciprocal cytokine receptor.

### Example 8. IFN-γ signaling is needed to maintain immune surveillance of the healthy CNS

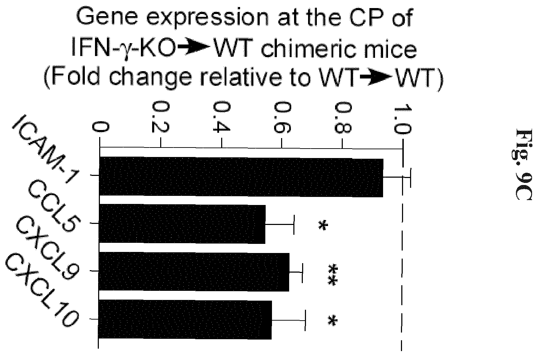

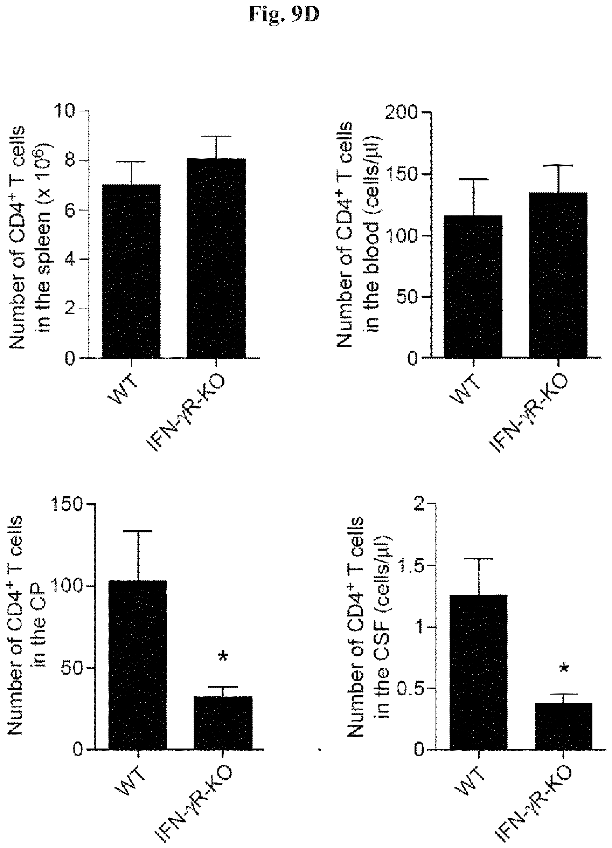

To further substantiate the role of IFN-γ signaling in the expression of trafficking molecules by the CP epithelium *in vivo,* we examined the CP of IFN-γR knockout (IFN-γR-KO) mice. We found the basal expression levels of a wide array of trafficking molecules to be lower in the CP of IFN-γR deficient mice, relative to WT animals **(****Fig. 9A****).** Notably, the basal expression level of Arg1, which was shown above to be induced by the CP epithelium in response to IL-4 **(****Fig. 7D****,** **H****),** was significantly elevated in the CP of IFN-γR-KO mice. To confirm that the observed results were not due to the deficiency of IFN-γR on circulating immune cells, we created chimeric mice following total body irradiation while protecting the head (Shechter *et al.,* 2009); in these mice, the bone marrow (BM) of the IFN-γR-KO mice was replaced by WT BM. The same pattern of down-regulation of trafficking molecules by the CP was noticed **(****Fig. 9B****).** We also created chimeric mice lacking expression of the cytokine IFN-γ in circulating immune cells. These chimeric mice were created by replacing the BM of WT mice with IFN-γ knockout (IFN-γ-KO) BM, following a split-dose irradiation protocol, to allow total depletion of the host hematopoietic cells (achieving 99% chimerism). Strikingly, after 2 months of BM-chimerism, the CP of these animals showed reduced levels of CP-expressed trafficking molecules, relative to their controls that received WT BM **(****Fig. 9C****),** further supporting the need for IFN-γ signaling for the expression of trafficking molecules by the CP. These results prompted us to examine whether the outcome of poor trafficking molecule expression by the CP of IFN-γR-KO mice might result in reduced immune surveillance of the CNS. We therefore examined the numbers of CD4⁺ T cells in the CP and CSF of IFN-γR-KO mice, and found a significant reduction in their numbers relative to age matched WT controls **(****Fig. 9D****).** This reduction was specific for the CP and CSF and did not result from a systemic reduction in CD4⁺ T cells, as the numbers of these cells in the circulation and lymphoid organs were similar to the WT mice **(****Fig. 9D****).** These results demonstrated a pivotal role of IFN-γ signaling in maintaining CNS immune surveillance via the CP, and prompted us to examine the role of IFN-γ signaling in CNS repair.

### Example 9. IFN-yR-KO mice have impaired recovery following SCI, which is associated with failure of CP activation for leukocyte trafficking

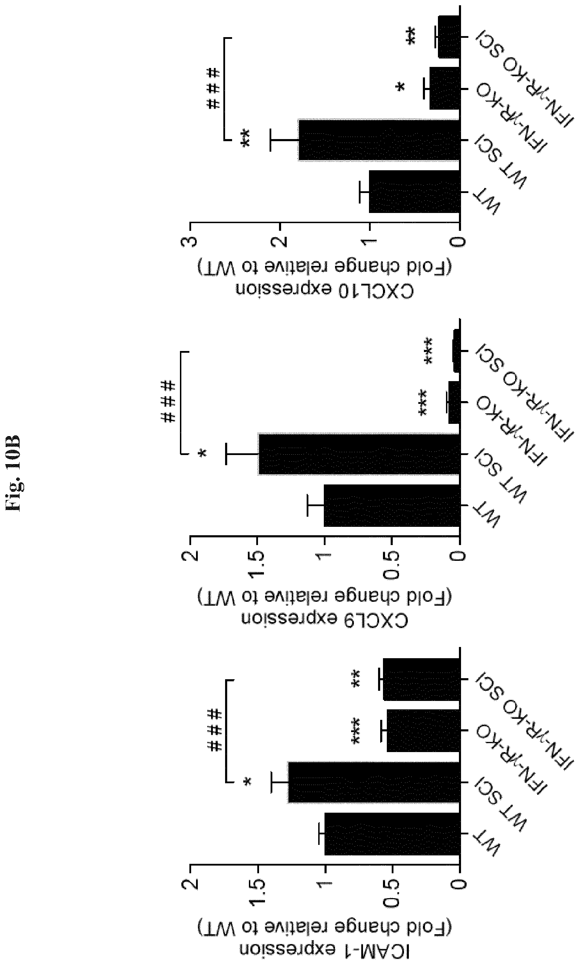

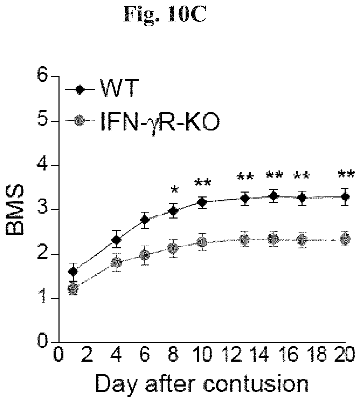

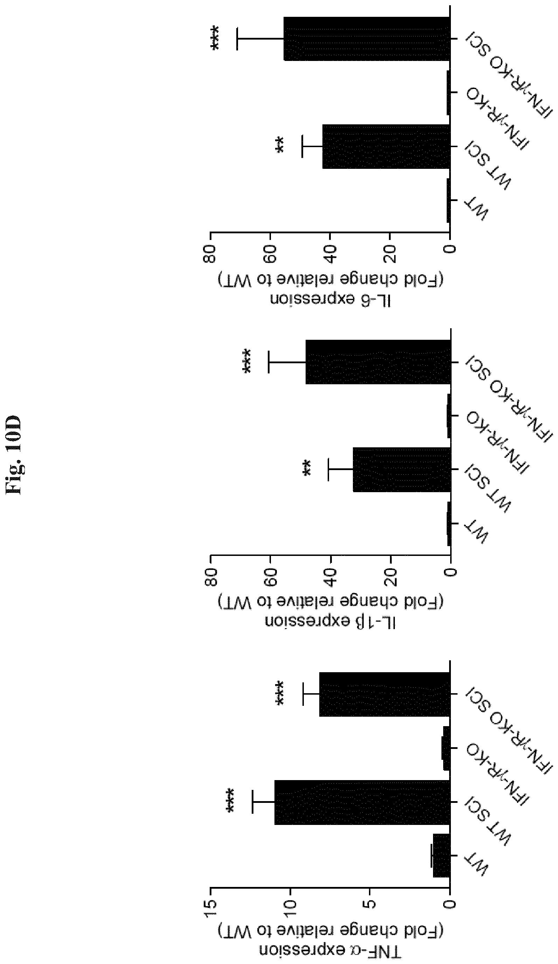

The reduced trafficking of leukocytes across the CP to the CSF in IFN-γR-KO animals under physiological conditions, led us to consider that such a defect might have a much more pronounced effect following injury. We subjected WT mice to a severe, well-calibrated contusive SCI at the level of T-12 (Ziv *et al.* , 2006b), and first tested whether such injury affected the expression of IFN-γR by the CP. We found IFN-γR expression to be upregulated by the CP epithelium on day 1 and day 7 post SCI **(****Fig. 10A****),** corresponding to previously described waves of immune cell trafficking to the CNS following acute injury to the spinal cord. Notably, while the expression of IFN-γR peaked in two waves, immunostaining for the tight junction molecule E-cadherin revealed its reduction in the CP epithelium for several days following the injury (data not shown). Next we determined whether in the absence of IFN-γR, the CP would lose its capacity to be activated for expression of trafficking molecules following injury. We inflicted SCI in WT and IFN-γR-KO mice and found that while in the WT animals the CP was activated to enable leukocyte trafficking, IFN-γR-KO mice failed to elevate expression of the adhesion molecule ICAM-1, and the chemokines CXCL9 and CXCL10 **(****Fig. 10B**). The lack of activation of the CP of the IFN-γR-KO mice for trafficking prompted us to test their functional recovery from the injury. A follow-up of hind-limb locomotor performance, assessed according to the open-field Basso Mouse Scale (BMS; in which a score of 0 represents complete paralysis and of 9 complete mobility), revealed that IFN-γR-KO mice had a worse recovery following the injury relative to WT mice **(****Fig. 10C****).** To rule out the possibility that the different activation of the CP for expression of trafficking molecules resulted from differences in the injury-induced inflammatory response at the lesion site, we subjected WT and IFN-γR-KO mice to SCI, and examined the lesion site for levels of the pro-inflammatory cytokines, TNF-α, IL-1β, and IL-6. Both WT and IFN-γR-KO mice showed similar increase of local levels of the tested pro-inflammatory cytokines 24 h following the injury (the hyper-acute phase) **(****Fig. 10D****).** In addition, immunohistochemical analysis revealed that microglia and astrocytes were activated at the site of injury, in both WT and IFN-γR-KO mice (data not shown).

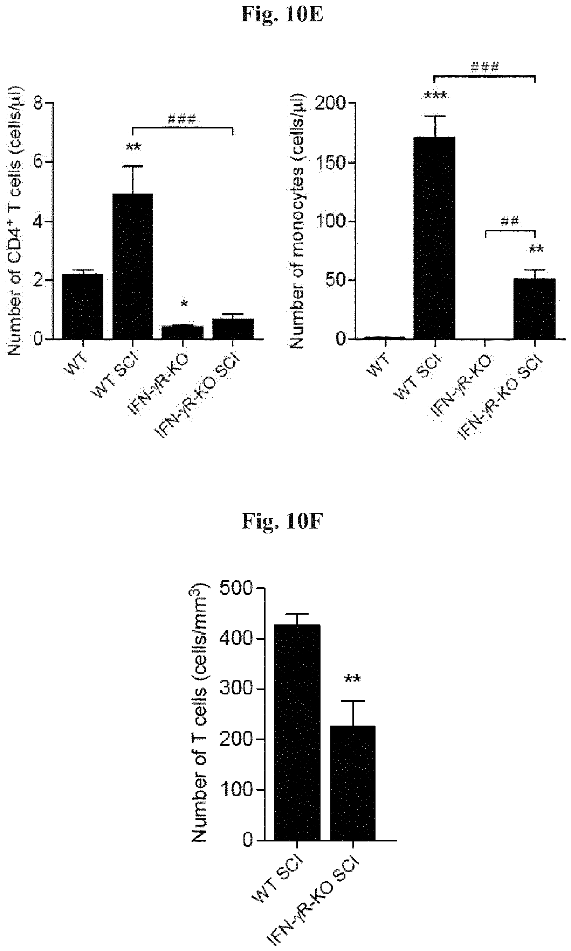

Importantly, the lack of activation of the CP was correlated with impaired trafficking of both CD4⁺ T cells and monocytes to the CSF of the injured IFN-γR-KO mice relative to WT mice after SCI **(****Fig. 10E****).** The IFN-γ-dependent trafficking of monocytes through the CP was further verified *in vitro* using a transepithelial migration assay; TNF-α, in combination with IFN-γ stimulated the CP to mediate monocyte trafficking towards CCL2 (data not shown).

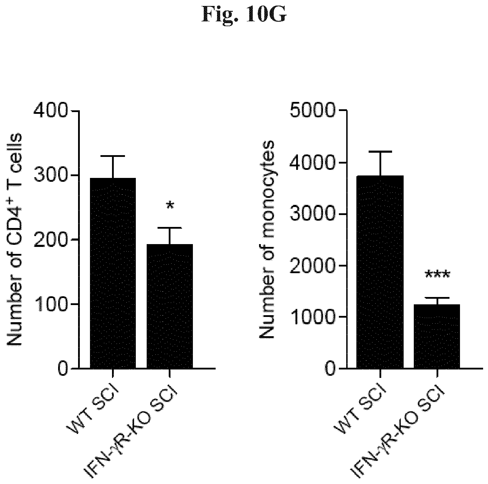

Leukocyte trafficking across the CP to the CNS parenchyma, was further supported by the appearance of T cells in the ependymal layer of the central canal, rostral to the lesion site (data not shown), indicating their migration to the injury site through this route. Their numbers were significantly reduced in the lesion site epicenter in IFN-γR-KO mice following the injury, relative to WT mice **(****Fig. 10F****).** In addition, flow cytometry analysis of the spinal cord of IFN-γR-KO mice on day 7 post injury showed reduced numbers of CNS-recruited CD4⁺ T cells and monocytes relative to their numbers in injured WT mice **(****Fig. 10G**). Taken together, these results demonstrate the role of IFN-γ-signaling in CNS repair process, via its essential role in regulating leukocyte trafficking through the CP.

### Example 10. Foxp3⁺ Tregs depletion activates the CP for trafficking.

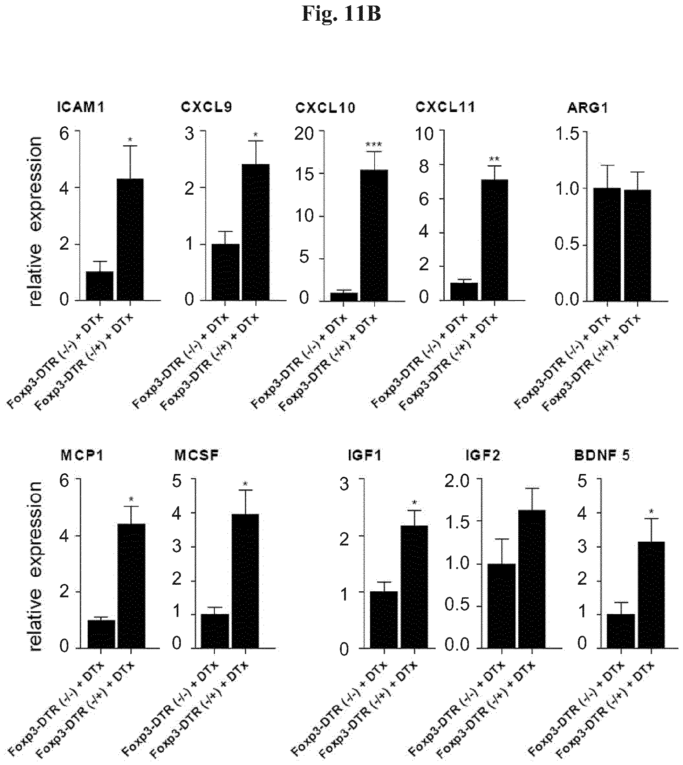

DEREG mice carry a DTR-eGFP transgene under the control of an additional Foxp3 promoter, thereby allowing specific depletion of Treg by application of diphtheria toxin (DTX), as described in Lahl and Sparwasser, 2011, Methods Mol. Biol. 707:157-72.. Foxp3-DTR^{-/-} and Foxp3-DTR^{-/+} were injected i.p. with 8ng/g animal weight DTX per day for 4 constitutive days. On the fifth day mice were perfused with PBS and their spleen, CP and CSF were analyzed by flow cytometry for their cellular composition. As can be seen from **Fig. 11A****,** Foxp3⁺ T cells were depleted from the spleen, the CP, and the CSF in the DTR⁺ mice (n=6 per group). Following the same depletion protocol, mRNA expression levels of selected genes was examined in the CP of both Foxp3-DTR^{-/-} and Foxp3-DTR^{-/+} mice. Expression of the integrin receptor ICAM-1 and the chemokines CXCL9, CXCL10, CXCL11, MCP-1 and MCSF, and the neurotropic factors BDNF and IGF-1, was significantly upregulated in the CP following Foxp3⁺ T cells depletion **(****Fig. 11B****)**

### Example 11. Immunomodulation of the choroid plexus epithelium with CpG.

The aim of this experiment was to characterize the immune system present at the CP in healthy adult mice under normal physiological conditions and its response to activation by the Th1 adjuvant CpG. C57B1/6J male mice were injected intraperitoneally (i.p.) with CpG (20ug). One or three days after the injection, mice were intracardially perfused with PBS and choroid plexus tissues were isolated from the lateral, third and fourth ventricles of the brain. Tissues were immediately placed on dry ice, and were later processed for RNA isolation. Total RNA of the choroid plexus was extracted and converted to cDNA. The expression of specific mRNAs was assayed using fluorescence based real-time quantitative PCR (qPCR). As seen in **Fig. 12****,** following CpG injection, the CP upregulated both genes that are required for immune cells trafficking in this compartment (IFNγR, ICAM-1, MadCAM-1) and neurotropic factors needed for central nervous system (CNS) repair (IGF-1, BDNF).

Using the same paradigm of CpG immunomodulation, we injected CpG to animals 1 day before (day -1) preforming spinal cord injury (SCI). The animals were intracardially perfused with PBS and choroid plexus tissues were isolated one day after the spinal cord injury. CpG significantly upregulated both trafficking and neurotropic molecule, pointing to beneficial activation of this compartment in an effort to increase leukocyte recruitment to the injured spinal cord **(****Fig. 13****).**

### Example 12. The choroid plexus (CP) of mutant superoxide dismutase 1 G93A (mSOD1) mice is not spontaneously activated to enable leukocyte trafficking.

Amyotrophic lateral sclerosis (ALS) is a fatal neurodegenerative disease in which motor neurons progressively die, leading to paralysis and death within 1 to 5 years after diagnosis. While the etiology of motor neuron degeneration in ALS remains elusive, it is considered a non-cell-autonomous disease. Like many other neurodegenerative diseases, ALS is associated with a local innate-based inflammatory response, the nature of which is not fully understood, and the mechanism underlying its effect on disease onset and progression has not been fully elucidated.

In the context of ALS, accumulated data suggest that life expectancy is shorter, and disease emerges earlier, in animals lacking CD4⁺ T cells. Accordingly, mutant superoxide dismutase 1 G93A (mSOD1) mice, a murine model of ALS, spontaneously develop immune deficiency along disease progression (Banerjee *et al.,* 2008). Increased survival of mSOD1 mice has been correlated with enhanced infiltration of CD4⁺ T cells to the spinal cord, suggesting that the spontaneous infiltration of CD4⁺ T cells to the spinal cord of mSOD1 mice at early stages of the disease is not sufficient, and that boosting this process may delay disease progression. Among the CD4⁺ T cell subsets that were shown to mitigate this disease are effector T cells and Tregs (Banerjee *et al.,* 2008; Beers 2011). The findings that the CP orchestrates recruitment of inflammation-resolving leukocytes to the injured CNS, together with the observation that lymphocytes are beneficial in ALS, and immune deficiency develops along disease progression, created the basis for our current hypothesis that activation of the CP for leukocyte trafficking might be dysregulated in ALS as a results of peripheral immunity dysregulation, and is amenable to immunomodulation as a therapeutic strategy.

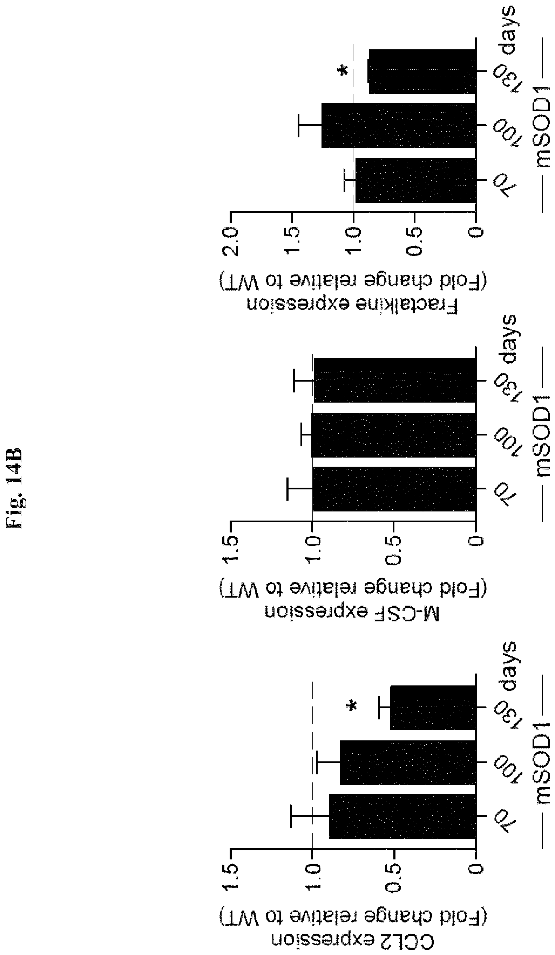

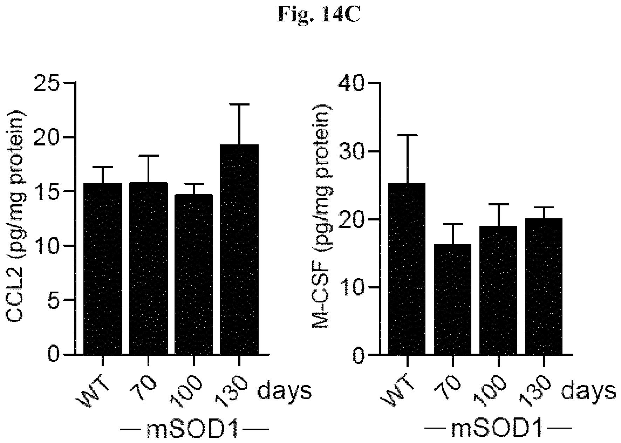

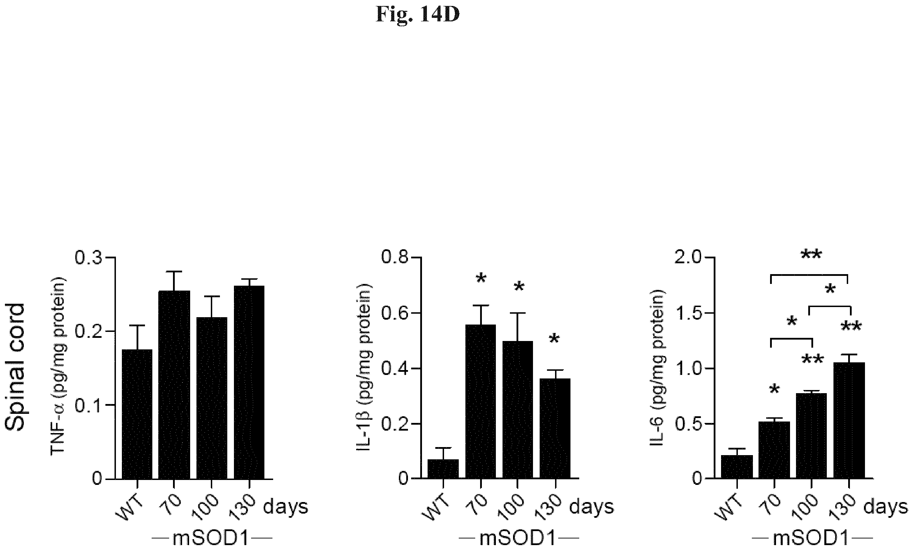

First, we tested by quantitative real-time PCR the expression of the intercellular adhesion molecule 1 (ICAM-1) and vascular cell adhesion molecule 1 (VCAM-1). We found that neither adhesion molecule was elevated in the CP of mSOD1 mice relative to wild type (WT) mice, and that ICAM-1 was down-regulated over the disease course **(****Fig. 14A****).** Immunohistochemistry revealed that ICAM-1 was co-localized with the CP epithelium (visualized by cytokeratin staining) in WT, and was reduced on the epithelium at the progressive stage of the disease in mSOD1 mice (day 130) (data not shown). Analysis of the trafficking molecules CCL2, M-CSF, and Fractalkine, showed a similar pattern at both the mRNA **(****Fig. 14B****)** and protein **(****Fig. 14C****)** levels. Thus, no elevation of the tested trafficking-associated molecules was detected upon disease onset (day 100) or during its progression. Importantly, measurement of the local inflammatory response in the spinal cord parenchyma revealed the elevation of both IL-1β and IL-6 **(****Fig. 14D****);** these cytokines are elevated following acute spinal cord injury, and are involved in activation of the CP to induce expression of trafficking molecules. Taken together, these results suggested that although the CNS parenchyma of mSOD1 mice shows signs of local inflammation similar to those found under acute spinal cord injury, their CP is not activated to enable immune cell trafficking.

### Example 13: IFN-γ-dependent activation of the choroid plexus in mSOD1 mice.

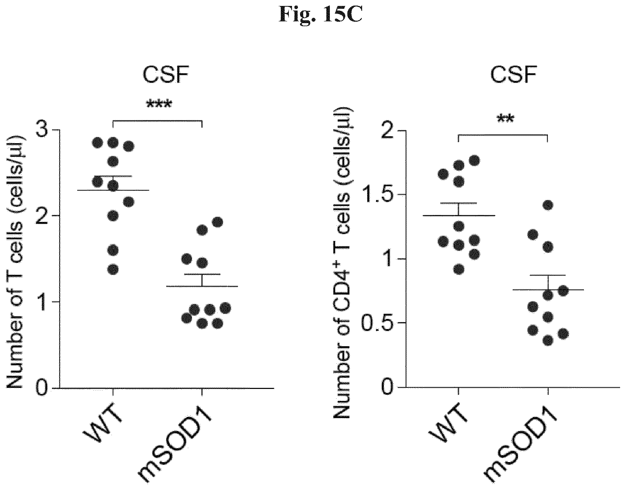

The CP of healthy WT animals is populated by effector memory CD4⁺ T cells, capable of producing IFN-γ and IL-4; of these cytokines, IFN-γ was found to be essential to promote trafficking molecule expression by the CP epithelium. We therefore quantitated the levels of these cytokines in the CP of mSOD1 mice. We found a general decrease, relative to WT, in the levels of both cytokines; IFN-γ levels showed an earlier reduction starting from day 70 **(****Fig. 15A****).** Examination of the CP of mSOD1 mice at different stages of the disease by flow cytometry revealed a reduction in T cell numbers, including CD4⁺ T cells, and specifically, IFN-γ producing CD4⁺ T cells, as early as day 70, an asymptomatic stage in this animal model **(****Fig. 15B****).** Moreover, numbers of both total T cells and of CD4⁺ T cells were lower in the cerebrospinal fluid (CSF) of 70-day old mSOD1 mice **(****Fig. 15C****),** indicating a general reduction in CNS immune surveillance.

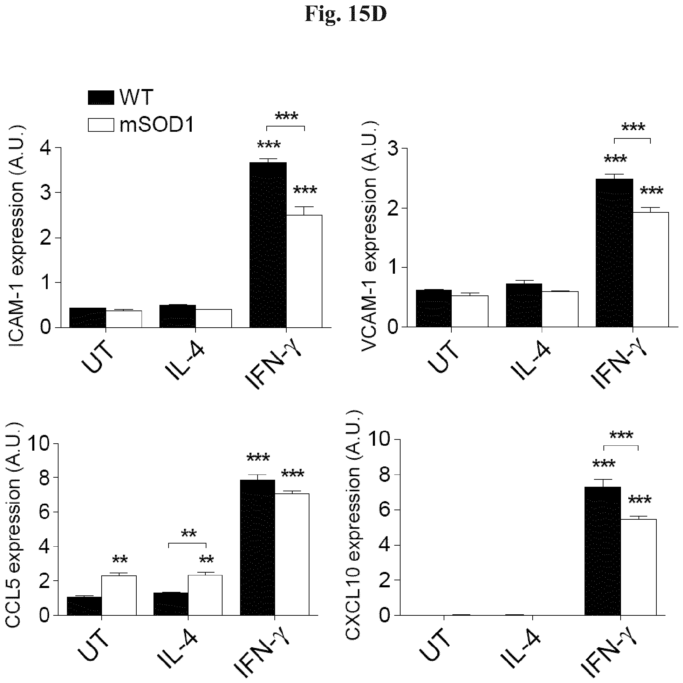

To test whether the CP of the mSOD1 mice did not lose the capacity to respond to these cytokines, we cultured CP epithelial cells from WT and mSOD1 mice, and treated them with IFN-γ or IL-4. mRNA levels of various trafficking molecules were measured 24h after the addition of cytokines, and were compared to untreated cells. IFN-γ treatment elevated the expression of trafficking molecules by both WT and mSOD1 CP cultures to a similar degree **(****Fig 15D****),** suggesting that the CP of mSOD1 mice has the ability to respond to effector T cell-derived cytokines. Our finding that the CP of mSOD1 mice is amenable to activation for leukocyte trafficking, prompted us to search for a way to stimulate trafficking *in vivo.*

### Example 14. Immunomodulation of the choroid plexus epithelium of mSOD mice with CpG.

We examined the effects of CpG immunomodulation of the choroid plexus in mSOD1 mice. Mice were injected intraperitoneally (i.p.) with CpG (20ug) at age 80 and 83 days, the time period of the beginning of clinical signs. Seven days following the first injection, mice were intracardially perfused with PBS and choroid plexus tissues were isolated from the lateral, third and fourth ventricles of the brain. Tissues were immediately placed on dry ice, and were later processed for RNA isolation. Total RNA of the choroid plexus was extracted and converted to cDNA. The expression of specific mRNAs was assayed using fluorescence based real-time quantitative PCR (qPCR). As seen in **Fig. 16****,** all trafficking molecules tested were upregulated following CpG injection. These results point to CpG immunomodulation to be potentially beneficial in treating ALS.

### Example 15: Induction of transient autoimmune encephalomyelitis activates the choroid plexus of WT mice for leukocytes trafficking.

Monophasic experimental autoimmune encephalomyelitis (EAE) creates favorable conditions for accumulation of Foxp3⁺ regulatory T cells in the CNS parenchyma. In addition, it was previously shown that the induction of chronic EAE activates the CP to induce leukocyte trafficking (Murugesan *et al.,* 2012). Together, these observations prompted us to examine whether the induction of monophasic autoimmune encephalomyelitis would similarly activate trafficking of leukocytes through the CP, and if so, whether this treatment would promote entry of Tregs to the diseased CNS in ALS. We first addressed this question in WT mice by immunizing the mice with myelin oligodendrocyte glycoprotein (MOG) peptide together with low levels of *M. tuberculosis;* the resulting inflammation was significantly milder compared to chronic EAE induced by MOG with *pertussis toxin* (MOG+PTX), and spontaneously resolved after 21 days **(****Fig. 17A****).** This monophasic EAE induced the expression of ICAM-1 by the CP epithelium compared to naive WT mice (PBS) (data not shown). Examination of the CSF of the mice 14 days post immunization, revealed the increase of infiltrating T cells, comprised mostly of CD4⁺ T cells, relative to naive or to ovalbumin (OVA)-immunized mice **(****Fig. 17B****).**

### Example 16: Induction of transient autoimmune encephalomyelitis in mSOD1 mice activates the choroid plexus for trafficking of leukocytes to the ventral horn gray matter.

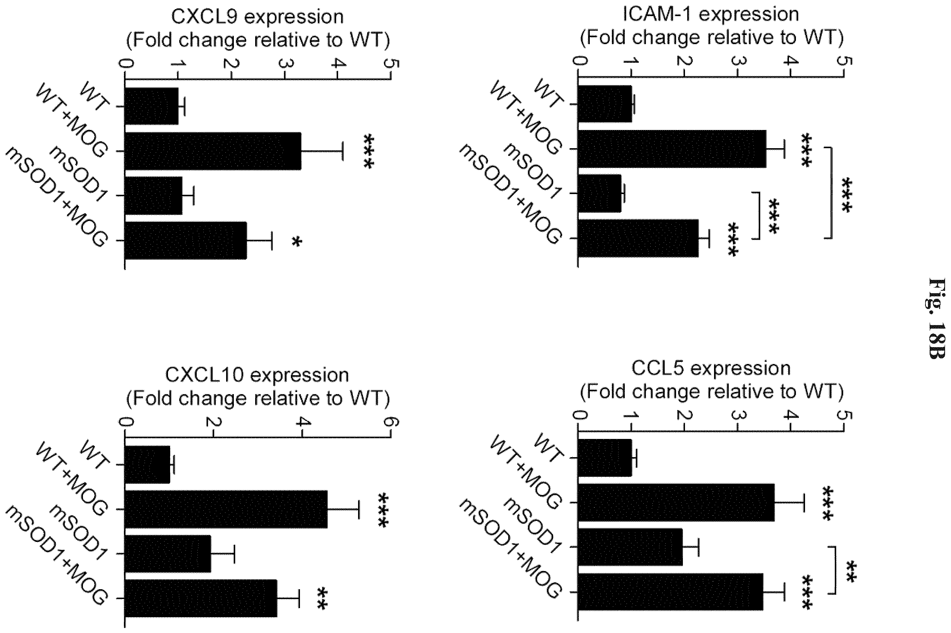

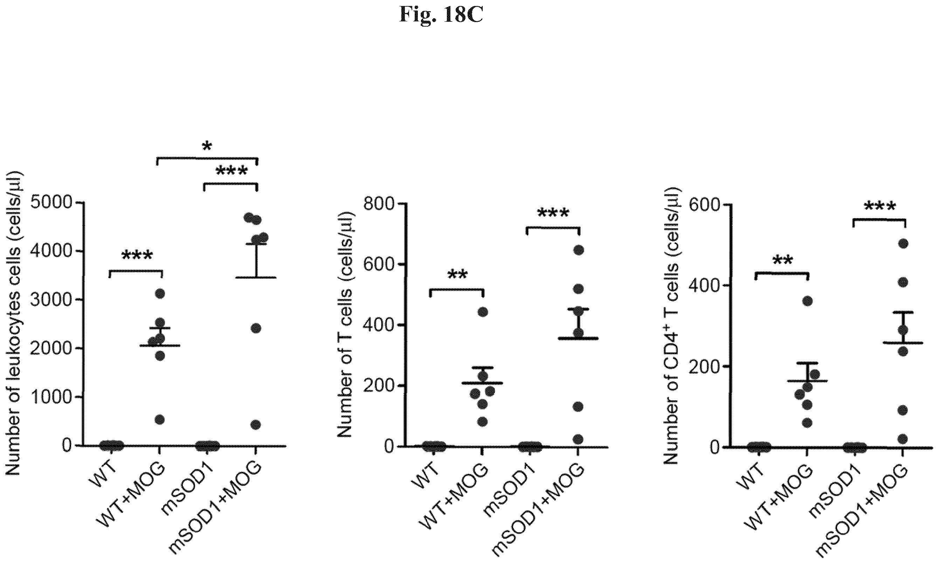

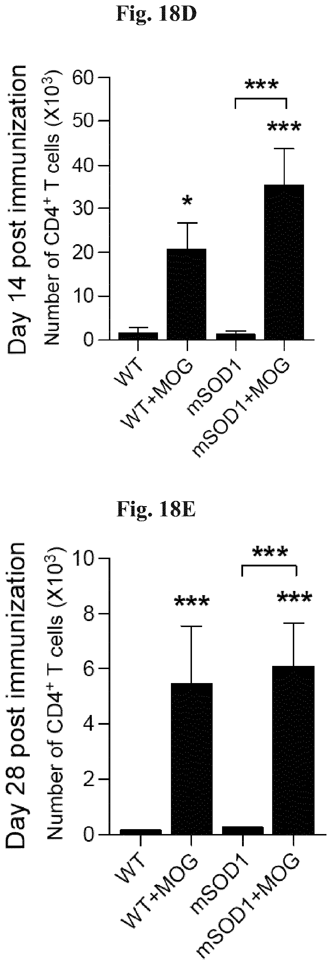

Based on the results above, we immunized mSOD1 mice at the age of 60 days, the asymptomatic phase, to allow the self-resolution of the autoimmune encephalomyelitis-associated inflammation to take place before the onset of the clinical symptoms of ALS. As we observed in the WT mice, immunization of mSOD1 mice caused mild and transient clinical symptoms of autoimmune encephalomyelitis **(****Fig. 18A****),** which spontaneously resolved by day 21-28 post-vaccination. Examination of the CP 14 days post immunization, showed a significant increase in the expression of trafficking molecules compared to non-immunized mice **(****Fig. 18B****).** The activation of the CP was accompanied by an increase in total leukocyte numbers, including T cells and CD4⁺ T cells, in the CSF of the immunized animals **(****Fig. 18C****).** Flow cytometry analysis of the excised spinal cord parenchyma 14 days post immunization, revealed that the immunization caused increased infiltration of CD4⁺ T cells to the spinal cord of WT and mSOD1 mice compared to non-immunized mice **(****Fig. 18D****).** At this time point, infiltrating T cells were found in the spinal cord parenchyma in association with central canal ependymal cells (data not shown), previously suggested to provide a migratory pathway for leukocytes that traffic via the CP to the parenchyma. In addition, T cells were found in association with parenchymal blood vessels (data not shown).

CD4⁺ T cell numbers remained elevated in the spinal cord parenchyma at 28 days post immunization, after the autoimmune encephalomyelitis symptoms were resolved **(****Fig. 18E****).** Examination of the spatial distribution of the infiltrating T cells at this time point revealed that in the immunized mSOD1 mice, T cells were found in the gray matter of the spinal cord ventral horn, the area where motor neurons reside (data not shown); in ALS, these diseased parenchymal regions were shown to express chemoattractive molecules associated with local innate neuroinflammation (Appel *et al.,* 2010). Importantly, in the absence of immunization, T cells were hardly detectable in the gray matter of either WT or mSOD1 mice.

### Example 17: Induction of transient autoimmune encephalomyelitis in mSOD1 mice facilitates accumulation of Foxp3⁺ regulatory T cells in the spinal cord and increases life expectancy.

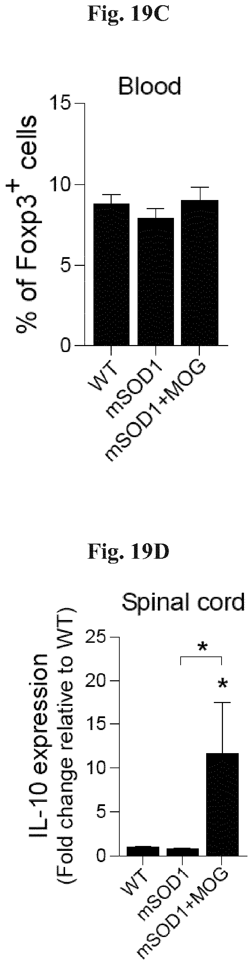

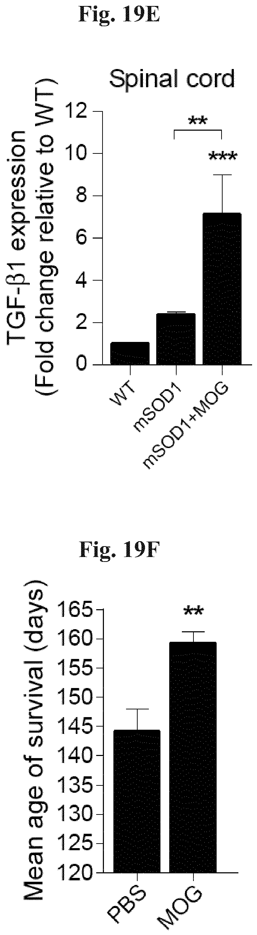

As it was previously shown that in monophasic EAE, the CNS inflammation drives the rapid activation and proliferation of Foxp3⁺ Tregs (O'Connor *et al.,* 2007), we examined by flow cytometry whether Foxp3⁺ Tregs accumulated in the spinal cord of mSOD1 mice at day 28 post immunization, when the symptoms of the transient autoimmune encephalomyelitis were resolved. At this time point, we found a significant elevation of Tregs in both the parenchyma **(****Fig. 19A****)** and in the CSF **(****Fig. 19B****),** yet not in the blood **(****Fig. 19C****),** indicating their selective elevation in the CNS; this elevation following monophasic EAE was previously shown to be derived from local proliferation within the CNS. The accumulated Tregs in the spinal cord were associated with increased local expression of IL-10 **(****Fig 19D****),** the hallmark cytokine of anti-inflammatory cells. Assessment of tissue distribution of Tregs and cells expressing IL-10, revealed accumulation of both IL-10⁺ and of Foxp3⁺ cells at the site of motor neuron loss in mSOD1 mice, in association with inflammatory Iba1⁺ microglia, known to be activated as part of the microglial toxicity in ALS (data not shown). The CNS-specific accumulation of Tregs during the resolution of EAE was reported to be TGF-β1 dependent (Liu, 2006); we therefore examined the expression of this cytokine in the spinal cord parenchyma of the immunized mice and found it to be elevated relative to non-immunized WT or mSOD1 mice **(****Fig. 19E****).** Finally, we determined whether such immunization, which led to the accumulation of IL-10-producing cells in the spinal cord parenchyma, would increase life expectancy in the mSOD1 mice. The immunized mice showed increased average survival of 15 days from 144.2±3.8 to 159.5±2.2 days **(****Fig. 19F****).** The effect of the vaccination was most strongly apparent at the age of 154 days, when 90% of the immunized mice were still alive, compared to only 10% of the untreated animals **(****Fig. 19G****).**

### Example 18. Infrequent vaccination with Copolymer 1 (GA) rescues cognitive decline and reduces the incidence of peripheral FoxP3⁺ T cells in 5XFAD mice.

Alzheimer's disease (AD) is an irreversible, progressive brain disorder that occurs gradually and results in memory loss, behavioral and personality changes, and a decline in mental abilities. These losses are related to the death of brain cells and the breakdown of the connections between them. The course of this disease varies from person to person, as does the rate of decline. On average, AD patients live for 8 to 10 years after they are diagnosed, though the disease can last up to 20 years.

AD advances by stages, from early, mild forgetfulness to a severe loss of mental function. At first, AD destroys neurons in parts of the brain that control memory, especially in the hippocampus and related structures. As nerve cells in the hippocampus stop functioning properly, short-term memory fails. AD also attacks the cerebral cortex, particularly the areas responsible for language and reasoning. Eventually, many other areas of the brain are involved.

Copolymer 1, also called Cop 1, GA or Glatiramer Acetate, is a random non-pathogenic synthetic copolymer, a heterogeneous mix of polypeptides containing the four amino acids L-glutamic acid (E), L-alanine (A), L-tyrosine (Y) and L-lysine (K) in an approximate ratio of 1.5:4.8:1:3.6, but with no uniform sequence. Although its mode of action remains controversial, Cop 1 clearly helps retard the progression of human multiple sclerosis (MS) and of the related autoimmune condition studied in mice, EAE. One form of Cop 1, known as glatiramer acetate, has been approved in several countries for the treatment of multiple sclerosis under the trademark Copaxone® (Teva Pharmaceutical Industries Ltd., Petach Tikva, Israel).

Vaccination with Cop 1 or with Cop 1-activated T cells have been shown by the present inventors to boost the protective autoimmunity, after traumatic CNS insult, thereby reducing further injury-induced damage, and can further protect CNS cells from glutamate toxicity. Reference is made to Applicant's previous US Patent Applications Nos. 09/765,301 and 09/765,644 and corresponding published International Application Nos. WO 01/52878 and WO 01/93893, which disclose that Cop 1, Cop 1-related peptides and polypeptides and T cells activated therewith prevent or inhibit neuronal degeneration and promote nerve regeneration in the CNS or peripheral nervous system (PNS), and protect CNS cells from glutamate toxicity.

Prof. Schwartz and colleagues have shown that Cop 1 acts as a low-affinity antigen that activates a wide range of self-reacting T cells, resulting in neuroprotective autoimmunity that is effective against both CNS white matter and grey matter degeneration. The neuroprotective effect of Cop 1 vaccination was demonstrated by the inventors in animal models of acute and chronic neurological disorders such as optic nerve injury, glaucoma, and amyotrophic lateral sclerosis as disclosed in the applicant's patent applications WO 01/52878, WO 01/93893 and WO 03/047500.

The use of Copolymer 1 for treatment of prion-related diseases is disclosed in WO 01/97785. Gendelman and co-workers disclose that passive immunization with splenocytes of mice immunized with Cop 1 confers dopaminergic neuroprotection in MPTP-treated mice (Benner et al., 2004).

The generation of the 5XFAD mice, a mouse model for AD has been described previously (Oakley, et al., 2006, JNeurosci 26(40):10129-40, 2006).

In this study we were interested in clarifying whether the beneficial effect of Cop 1 on mouse models of AD is mediated by the activation of the CP and whether the frequency of immunization affect the activation.

The level of FoxP3⁺ cells (Tregs) gated from total CD4⁺TCRβ⁺ T cells in spleen from 4 and 8 months old 5XFAD mice as compared to non-transgene (WT) controls was analyzed by FACS. As can be seen from fig **19A****,** FoxP3⁺ T cells were enriched in the spleens of 5XFAD mice as compared to their non-transgenic WT littermates.

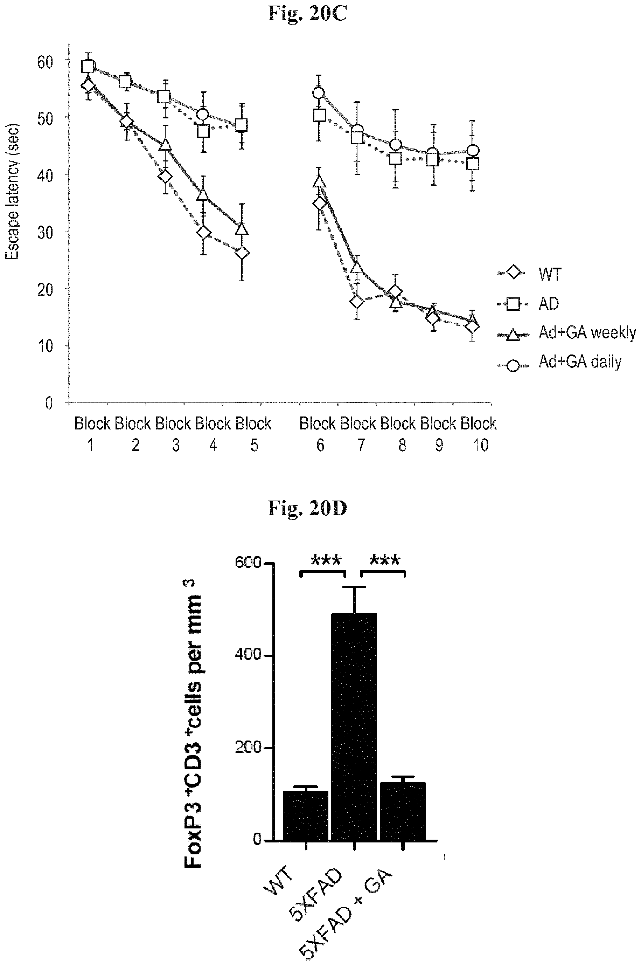

To test the effect of different regimens of administration of Cop 1, 5XFAD females were vaccinated subcutaneously (S.C.) with 100 µg Cop 1 dissolved in in 200 µl PBS. Three different regimens were compared: 5XFAD+2GA were vaccinated twice, with the second injection given three days after the first; 5XFAD+5GA were vaccinated five times, twice in the first week and once a week for another three weeks, and 5XFAD+daily GA were vaccinated every day for 28 days **(19B).** One day after the last GA injection, mice were sacrificed and their spleens were analyzed by FACS and compared to WT and untreated 5XFAD littermates. Note the significant decrease in the fold change of FoxP3⁺ T cells following 2GA injections as compared to untreated 5XFAD. However, when administered daily, GA promoted the enrichment of FoxP3⁺ T cells in the spleens of 5XFAD mice. Radial arm water maze (RAWM) was performed to 4 groups of 8 months old mice to test mental cognitive performance. As can be seen from **Fig. 20C****,** performance was significantly improved in 5XFAD mice vaccinated with weekly GA (twice in the first week and once a week for another 3 weeks) compared to the unvaccinated 5XFAD mice, but not with daily administration of GA, which performed as badly as the unvaccinated 5XFAD mice. CP sections from six months old untreated non-transgenic (WT), and 5XFAD mice, and 5XFAD+5GA mice were stained for CD3 and FoxP3. As can be seen in **Fig. 20D****,** the incidence of FoxP3⁺CD3⁺ T cells in the CP of untreated 5XFAD mice as compared to WT is significantly increased. However, the incidence of these cells was decreased in the 5XFAD+5GA (indicated as 5XFAD+GA in the figure) mice as compared to untreated 5XFAD littermates.

### Example 19. Active immunization with MOG and monitoring levels of T regulatory and T helper 1 (Th1) cells in the blood.

200 µg of MOG₃₅₋₅₅ (GL Biochem Ltd., Shanghai) are emulsified in 200 µl incomplete Freund's adjuvant (IFA) containing 0.5 mg/ml *M. tuberculosis* (strain H37Ra; BD Diagnostics), and 200 µl is injected subcutaneously to three groups of mice: WT, mSOD and 5XFAD. Control mice for each group are injected subcutaneously with PBS. At days 7, 14 and 28 post immunization, the mice are anesthetized, blood is collected into heparin containing tubes and 50ml of each sample are treated with ACK lysing buffer (Life Technologies) to remove erythrocytes. For intracellular staining of IFN-γ (corresponding to Th1 cells) the cells are incubated with PMA (10 ng/ml; Sigma-Aldrich) and ionomycin (250 ng/ml; Sigma-Aldrich) for 6 h, and Brefeldin-A (10 mg/ml; Sigma-Aldrich) was added for the last 4 h. Intracellular labeling is done with BD Cytofix/Cytoperm Plus Fixation/Permeabilization kit (cat. no. 555028) according to the manufacturer's protocol. For intracellular staining of Foxp3⁺ T regulatory cells (Tregs), the cells are stained according to manufacturer's protocol (Mouse Regulatory T Cell Staining Kit, eBioscience, cat. No. 88-8111-40). Fluorochrome-labeled mAbs are used according to the manufacturers' protocols. Flow cytometry analysis is performed on each sample using a BD Biosciences LSRII flow cytometer, and the acquired data is analyzed using FlowJo software (Tree star). The ratio between Tregs and Th1 cells is calculated for each sample.

It is expected that the ratio of Tregs to Th1 cells will be lower in treated mice 7 or 14 days following immunization, than in the control group, reflecting the reduction of immunosuppression and activation of the choroid plexus. It is also expected that 28 days following immunization, this ratio will increase relatively to the ratio calculated 7 or 14 days following immunization. The increased ratio would correspond to re-establishment of immunosuppression and closing of the choroid plexus for transfer of healing cells.

Alternatively, instead of staining for IFN-γ and Foxp3 and calculating the ratio of Tregs/Th1, the peripheral blood mononuclear cells isolated at the indicated time points are tested for their proliferative response by standard methods, such as ³H-Thymidine uptake, in the presence or absence of MOG. A positive response (a higher proliferative response in the presence of MOG compared to without MOG) indicates successful vaccination and would correspond to reduction of immunosuppression and activation of the choroid plexus.

### Example 20. Treatment of Alzheimer's disease in a mouse model.

Alzheimer's disease or aged mice are first tested and scored for their cognitive ability by the Moris Water Maze test. Acquisition and probe trial are performed as previously described (Ron-Harel et al., 2008, Rejuvenation research 11(5):903-913). Following the probe trial, mice are given three additional trials without the platform to extinguish their initial memory of the platform's position. In the reversal phase, the platform is placed in a new location in the pool (opposite to where it was located in the acquisition phase) and the mice are given three trials per day on 2 consecutive days, conducted in a similar manner to the initial acquisition. Position and movement of the mice are recorded using an EthoVision automated tracking system (Noldus). According to their cognitive ability, mice are divided into aged mice with "intact" or "impaired" memory. The group of impaired memory is divided into two groups: "treated" vs. untreated.

The treated mice are subjected to induction of homeostatic-driven proliferation of T cells and/or administered a TLR agonist of TLR3, 4, 5, 7 or 9 (but not including CpG), or a neutralizing antibody directed to TLR2, adoptive transfer of CNS+ specific T-cells, optionally activated *ex vivo* by CNS-specific antigenic peptides or altered peptide ligands (APLs) of the peptides, cytokines such as IFN-γ or contact with CNS-specific APCs. Appropriate controls are performed on a group of mice with impaired memory. Following the immunomodulatory treatment, mice are examined again for their cognitive scoring, this time by using a different hippocampal-dependent test (for example: object recognition task) to evaluate the effects of the treatment.

### Example 21. Treatment of other neurogenerative diseases in a mouse models.

Accepted models for human disease comprising mice expressing the phenotype of Parkinson's disease, Huntington's disease, amyotrophic lateral sclerosis, age-related macular degeneration, retinitis pigmentosa, anterior ischemic optic neuropathy, glaucoma, uveitis, depression or stress are used. Diseased or aged mice afflicted with disease affecting cognitive abilities are first tested and scored for their cognitive ability by the Moris Water Maze test. Acquisition and probe trial are performed as previously described (Ron-Harel N, et al., 2008, Rejuvenation research 11(5):903-913). Following the probe trial, mice are given three additional trials without the platform to extinguish their initial memory of the platform's position. In the reversal phase, the platform is placed in a new location in the pool (opposite to where it was located in the acquisition phase) and the mice are given three trials per day on 2 consecutive days, conducted in a similar manner to the initial acquisition. Position and movement of the mice are recorded using an EthoVision automated tracking system (Noldus). According to their cognitive ability, mice are divided into aged mice with "intact" or "impaired" memory.

Mice afflicted with disease affecting motor function are tested for motor function using accepted tests such as the rotarod test.

The group of impaired memory is divided into two groups: "treated" vs. untreated.

The treated mice are subjected to induction of homeostatic-driven proliferation of T cells and/or administered a TLR agonist of TLR3, 4, 5, 7 or 9 (for example CpG), or a neutralizing antibody directed to TLR2, adoptive transfer of CNS+ specific T-cells, optionally activated *ex vivo* by CNS-specific antigenic peptides or APLs of the peptides, cytokines such as IFN-γ or contact with CNS-specific APCs. Appropriate controls are performed on a group of mice with impaired memory. Following the immunomodulatory treatment, mice are examined again for their cognitive scoring and/or motor function, this time by using a different (hippocampal-dependent) test (for example: object recognition task) to evaluate the effects of the treatment.

The present disclosure further relates to the following items:
Item 1: A method for treating a disease, disorder, condition or injury of the Central Nervous System (CNS) in a subject in need thereof, comprising administering to said subject a therapeutically effective amount of an active ingredient selected from the group consisting of:
   (i) a non-encephalitogenic or weakly encephalitogenic combination of a Th1 adjuvant and an agent selected from a CNS-specific antigen, a peptide derived from a CNS-specific antigen or from an analog thereof, or an analog or derivative of said peptide;
   (ii) a non-encephalitogenic or weakly encephalitogenic combination of a Th1 adjuvant and CNS-reactive T cells;
   (iii)CNS-reactive T cells having a Th1 phenotype as sole active ingredient; or
   (iv) a non-encephalitogenic Th1 adjuvant as a sole active ingredient provided that when said subject is afflicted with Alzheimer's disease, said non-encephalitogenic Th1 adjuvant is not CpG,
   thereby activating the choroid plexus of said subject and maintaining said activation by reducing immunosuppression and establishing Th1-type immune response at the choroid plexus thus allowing either anti-inflammatory immune cells or immune cells which acquire a healing phenotype at the cerebrospinal fluid, to pass through the choroid plexus, and accumulate at a site of damage in the CNS caused by said disease, disorder, condition or injury.
Item 2: A method for treating a disease, disorder, condition or injury of the CNS in a subject in need thereof having a certain level of immunosuppression in the circulation, said method comprising administering to said subject a therapeutically effective amount of an active ingredient selected from the group consisting of:
   (i) a non-encephalitogenic or weakly encephalitogenic combination of a Th1 adjuvant and an agent selected from a CNS-specific antigen, a peptide derived from a CNS-specific antigen or from an analog thereof, or an analog or derivative of said peptide;
   (ii) a non-encephalitogenic Th1 adjuvant or a Th1 adjuvant in combination with CNS-reactive T cells; or
   (iii) CNS-reactive T cells having a Th1 phenotype as sole active ingredient; or
   (iv) a non-encephalitogenic Th1 adjuvant as a sole active ingredient,
   wherein said administering is performed according to a regimen causing reduction of the level of said immunosuppression in the circulation of said subject relative to a reference, maintenance of said reduced level, and induction towards a Th1-type immune response, wherein said reduced level of immunosuppression and Th1-type immune response in the circulation indicates and ensures activation of the choroid plexus of said subject and thus allowing either anti-inflammatory immune cells or immune cells which acquire a healing phenotype at the cerebrospinal fluid from the circulation to pass through the choroid plexus and accumulate at a site of damage in the CNS caused by said disease, disorder, condition or injury.
Item 3: The method according to item 2, comprising:
   (i) administering said active ingredient of (i), (ii) or (iii) to said subject in need;
   (ii) determining said regimen by:
      i. monitoring immunosuppression and Th1/Th2 balance in said subject by measuring in a blood sample obtained from said subject, within a predetermined time-period following said administering, one or more parameters reflecting a degree of immunosuppression and the Th1/Th2 balance in the choroid plexus in said subject; and
      ii. comparing the one or more parameters measured in (b)(i) with said reference and determining whether said parameter is different from said reference; and
   (iii) deciding, based on the relation of said one or more parameters measured in (b)(i) to said reference, whether to repeat treatment and monitoring by repeating steps (a) and (b) or to continue monitoring by repeating only step (b).
Item 4: The method according to item 3, wherein said one or more parameters measured in a blood sample from said subject is:
   (i) a ratio of Treg cells to effector T cells;
   (ii) a level of Treg cells;
   (iii) a level of IFN-γ producing cells;
   (iv) a ratio of Treg cells to IFN-γ producing T cells;
   (v) a proliferative response of peripheral mononuclear cells to a CNS-specific antigen, a peptide derived from a CNS-specific antigen or from an analog thereof, or an analog or derivative of said peptide; or
   (vi) any combination of (i), (ii), (iii), (iv) and (v).
Item 5: The method according to item 2, wherein said reference is selected from the group consisting of:
   (a) a parameter measured in the most recent blood sample obtained from said subject before said administering, said parameter indicating a degree of immunosuppression in the choroid plexus in said subject;
   (b) a parameter indicating the degree of immunosuppression in the choroid plexus characteristic of a population of subjects afflicted with a disease, disorder, condition or injury of the CNS responding well to said administering, wherein said parameter is measured in blood samples obtained from said subjects; or
   (c) a parameter indicating the degree of immunosuppression in the choroid plexus characteristic of a population of healthy subjects, wherein said parameter is measured in blood samples obtained from said subjects.
Item 6 The method according to item 3, wherein said reference is measured in a blood sample obtained from said subject before said administering in (a).
Item 7 The method according to item 4 or 5, wherein said parameter is the ratio of Treg cells to effector T cells in a blood sample obtained from said subject, said reference is the most recent ratio measured in said subject before said administering the active ingredient, and
   (i) said treatment and monitoring is repeated when the ratio is substantially similar to or higher than the reference; or
   (ii) said monitoring is repeated when the parameter is lower than the reference value.
Item 8 The method according to any one of items 3 to 7, wherein said predetermined time-period is between 1 and 16 weeks.
Item 9 The method according to any one of items 1 to 7, wherein said non-encephalitogenic Th1 adjuvant comprises an agonist of TLR3, 4, 5, 7, 8 or 9; or an antagonist of TLR2 or neutralizing antibody directed to TLR2.
Item 10 The method according to item 8, wherein said TLR agonist is an agonist of TLR9.
Item 11 The method according to item 9, wherein said agonist of TLR9 is a CpG or stabilized immune modulatory RNA (SIMRA).
Item 12 The method according to item 10, wherein said CpG is of class B, preferably ODN1826.
Item 13 The method according to any one of items 1 to 7, wherein said active ingredient is a non-encephalitogenic Th1 adjuvant or a Th1 adjuvant with a weak encephalitogenic activity in combination with a CNS-specific antigen selected from the group consisting of myelin basic protein (MBP); myelin oligodendrocyte glycoprotein (MOG); proteolipid protein (PLP); myelin-associated glycoprotein (MAG); S-100; β-amyloid; Thy-1; a peripheral myelin protein including P0, P2 and PMP22; neurotransmitter receptors including acetylcholine receptor; and the protein Nogo including Nogo-A, Nogo-B and Nogo-C and the Nogo receptor.
Item 14 The method according to any one of items 1 to 12, wherein said active ingredient is a non-encephalitogenic Th1 adjuvant or a Th1 adjuvant with a weak encephalitogenic activity in combination with a peptide derived from a CNS-specific antigen or from an analog thereof, or an analog or derivative of said peptide.
Item 15 The method according to item 13, wherein said peptide derived from a CNS-specific antigen is selected from MBP₁₁₋₃₀, MBP₅₁₋₇₀, MBP₈₃₋₉₉, MBP₈₇₋₉₉, MBP₉₁₋₁₁₀, MBP₁₃₁₋₁₅₀, MBP₁₅₁₋₁₇₀ or MBP₈₄₋₁₀₄, MOG₃₅₋₅₅ or MOG₉₂₋₁₀₆, or PLP₁₃₉₋₁₅₁, or PLP₁₇₈₋₁₉₁.
Item 16 The method according item 14, wherein said peptide is MOG₃₅₋₅₅.
Item 17 The method according to item 13, wherein said Th1 adjuvant is a CpG.
Item 18 The method according to any one of items 13 to 16, wherein said peptide is MOG₃₅₋₅₅ and said Th1 adjuvant is a CpG.
Item 19 The method according to any one of items 1 to 7 wherein said active ingredient is:
   (iv) a non-encephalitogenic or weakly encephalitogenic combination of a Th1 adjuvant and CNS-reactive T cells generated by *ex vivo* expansion of T cells in the presence of a CNS-specific antigen; or
   (v) CNS-reactive T cells generated by expansion of T cells in the presence of a CNS-specific antigen and activated towards a Th1 phenotype *ex vivo,* as a sole active ingredient.
Item 20 The method according to any one of items 1 to 19, for treating a disease, disorder or condition of the CNS selected from the group consisting of a neurodegenerative disease, disorder or condition selected from the group consisting of amyotrophic lateral sclerosis, Alzheimer's disease, Parkinson's disease and Huntington's disease; primary progressive multiple sclerosis; secondary progressive multiple sclerosis; a retinal degeneration disorder selected from the group consisting of age-related macular degeneration and retinitis pigmentosa; anterior ischemic optic neuropathy; glaucoma; uveitis; depression; stress; and Rett syndrome.
Item 21 The method according to item 20, for treating amyotrophic lateral sclerosis.
Item 22 The method according to any one of items 1 to 19, for treating an injury of the CNS selected from spinal cord injury, closed head injury, blunt trauma, penetrating trauma, hemorrhagic stroke, ischemic stroke, cerebral ischemia, optic nerve injury, myocardial infarction, organophosphate poisoning and injury caused by tumor excision.
Item 23 The method according to any one of items 1 to 19 wherein said treating improves CNS motor and/or cognitive function.
Item 24 The method according to item 23, for alleviating age-associated loss of cognitive function.
Item 25 The method according to item 24, wherein said age-associated loss of cognitive function occurs in subjects free of a diagnosed disease.
Item 26 The method according to item 23, for alleviating loss of cognitive function resulting from acute stress.
Item 27 The method according to any one of items 23 to 26, wherein said cognitive function is learning, memory or both.
Item 28 A vaccine for use in a method of therapeutic immunization of a mammal comprising an active ingredient selected from:
   (i) a non-encephalitogenic or weakly encephalitogenic combination of a Th1 adjuvant and an agent selected from a CNS-specific antigen, a peptide derived from a CNS-specific antigen or from an analog thereof, or an analog or derivative of said peptide;
   (ii) a non-encephalitogenic or weakly encephalitogenic combination of a Th1 adjuvant and CNS-reactive T cells;
   (iii) CNS-reactive T cells having a Th1 phenotype as sole active ingredient; or
   (iv) a non-encephalitogenic Th1 adjuvant as a sole active ingredient provided that when said subject is afflicted with Alzheimer's disease, said non-encephalitogenic Th1 adjuvant is not CpG,
   wherein the vaccine is to be administered to thereby activate the choroid plexus of said mammal and maintain said activation by reducing immunosuppression and establishing Th1-type immune response at the choroid plexus thus allowing either anti-inflammatory immune cells or immune cells which acquire a healing phenotype at the cerebrospinal fluid, to pass through the choroid plexus, and accumulate at a site of damage in the CNS caused by said disease, disorder, condition or injury.
Item 29 A vaccine for use in a method of therapeutic immunization of a mammal comprising an active ingredient selected from:
   (i) a non-encephalitogenic or weakly encephalitogenic combination of a Th1 adjuvant and an agent selected from a CNS-specific antigen, a peptide derived from a CNS-specific antigen or from an analog thereof, or an analog or derivative of said peptide;
   (ii) a non-encephalitogenic or weakly encephalitogenic combination of a Th1 adjuvant and CNS-reactive T cells;
   (iii) CNS-reactive T cells having a Th1 phenotype as sole active ingredient; or
   (iv) a Th1 adjuvant as a sole active ingredient provided that when said subject is afflicted with Alzheimer's disease, said Th1 adjuvant is not CpG,
   wherein the vaccine is to be administered according to a regimen to thereby confer reduction and maintenance of the level of immunosuppression in the circulation of said mammal relative to a reference and induction towards a Th1-type immune response, wherein said reduced level of immunosuppression and Th1-type immune response in the circulation indicates and ensures activation of the choroid plexus of said mammal and maintenance of said activation thus allowing either anti-inflammatory immune cells or immune cells which acquire a healing phenotype at the cerebrospinal fluid to pass through the choroid plexus and accumulate at a site of damage in the CNS caused by disease, disorder, condition or injury.
Item 30 A method for inhibiting neuronal degeneration in the CNS, protecting neurons from glutamate toxicity or promoting nerve regeneration in nerve tissue damaged by injury to the CNS or by a disease, disorder or condition of the CNS, comprising administering to said subject a therapeutically effective amount of an active ingredient selected from the group consisting of:
   (i) a non-encephalitogenic or weakly encephalitogenic combination of a Th1 adjuvant and an agent selected from a CNS-specific antigen, a peptide derived from a CNS-specific antigen or from an analog thereof, or an analog or derivative of said peptide;
   (ii) a non-encephalitogenic or weakly encephalitogenic combination of a Th1 adjuvant and CNS-reactive T cells;
   (iii) CNS-reactive T cells having a Th1 phenotype as sole active ingredient; or
   (iv) a non-encephalitogenic Th1 adjuvant as a sole active ingredient provided that when said subject is afflicted with Alzheimer's disease, said non-encephalitogenic Th1 adjuvant is not CpG,
   thereby activating the choroid plexus of said subject and maintaining said activation by reducing immunosuppression and establishing Th1-type immune response at the choroid plexus thus allowing either anti-inflammatory immune cells or immune cells which acquire a healing phenotype at the cerebrospinal fluid, to pass through the choroid plexus, and accumulate at a site of damage in the CNS caused by said disease, disorder, condition or injury.
Item 31 A method for inhibiting neuronal degeneration in the CNS, protecting neurons from glutamate toxicity or promoting nerve regeneration in nerve tissue damaged by injury to the CNS or by a disease, disorder or condition of the CNS, said method comprising administering to said subject a therapeutically effective amount of an active ingredient selected from the group consisting of:
   (i) a non-encephalitogenic or weakly encephalitogenic combination of a Th1 adjuvant and an agent selected from a CNS-specific antigen, a peptide derived from a CNS-specific antigen or from an analog thereof, or an analog or derivative of said peptide;
   (ii) a non-encephalitogenic or weakly encephalitogenic combination of a Th1 adjuvant and CNS-reactive T cells;
   (iii) CNS-reactive T cells having a Th1 phenotype as sole active ingredient; or
   (iv) a non-encephalitogenic Th1 adjuvant as a sole active ingredient provided that when said subject is afflicted with Alzheimer's disease, said non-encephalitogenic Th1 adjuvant is not CpG,
   wherein said administering is performed according to a regimen causing reduction and maintenance of the level of said immunosuppression in the circulation of said subject relative to a reference and induction towards a Th1-type immune response, wherein said reduced level of immunosuppression and Th1-type immune response in the circulation indicates and ensures activation of the choroid plexus of said subject and thus allowing either anti-inflammatory immune cells or immune cells which acquire a healing phenotype at the cerebrospinal fluid from the circulation to pass through the choroid plexus and accumulate at a site of damage in the CNS caused by said disease, disorder, condition or injury.
Item 32 A pharmaceutical composition comprising a pharmaceutically acceptable carrier and a combination of agents selected from the group consisting of:
   (i) a non-encephalitogenic or weakly encephalitogenic combination of a Th1 adjuvant and an agent selected from a CNS-specific antigen, a peptide derived from a CNS-specific antigen or from an analog thereof, or an analog or derivative of said peptide;
   (ii) a non-encephalitogenic or weakly encephalitogenic combination of a Th1 adjuvant and CNS-reactive T cells;
   (iii) IFN-γ and a Th1 adjuvant;
   (iv) a combination of (i) with IFN-γ; and
   (v) a combination of (ii) with IFN-γ.

### REFERENCES

Alberti S, et al. (2006) Age-dependent modifications of Type 1 and Type 2 cytokines within virgin and memory CD4+ T cells in humans. Mechanisms of ageing and development 127(6):560-566.
Anandasabapathy N, et al. (2011) Flt3L controls the development of radiosensitive dendritic cells in the meninges and choroid plexus of the steady-state mouse brain. The Journal of experimental medicine 208(8):1695-1705.
Banerjee, R., R.L. Mosley, A.D. Reynolds, A. Dhar, V. Jackson-Lewis, P.H. Gordon, S. Przedborski, and H.E. Gendelman. 2008. Adaptive immune neuroprotection in G93A-SOD1 amyotrophic lateral sclerosis mice. PloS one 3:e2740.
Barrett, E.G., C. Johnston, G. Oberdorster, and J.N. Finkelstein. 1998. Silica-induced chemokine expression in alveolar type II cells is mediated by TNF-alpha. Am J Physiol 275:L1110-1119.
Beers, D.R., J.S. Henkel, W. Zhao, J. Wang, and S.H. Appel. 2008. CD4+ T cells support glial neuroprotection, slow disease progression, and modify glial morphology in an animal model of inherited ALS. Proceedings of the National Academy of Sciences of the United States of America 105:15558-15563.
Beers, D.R., et al. 2011. Endogenous regulatory T lymphocytes ameliorate amyotrophic lateral sclerosis in mice and correlate with disease progression in patients with amyotrophic lateral sclerosis. Brain : a journal of neurology 134, 1293-1314.
Benner, E.J., R.L. Mosley, C.J. Destache, T.B. Lewis, V. Jackson-Lewis, S. Gorantla, C. Nemachek, S.R. Green, S. Przedborski, and H.E. Gendelman. 2004. Therapeutic immunization protects dopaminergic neurons in a mouse model of Parkinson's disease. Proceedings of the National Academy of Sciences of the United States of America 101:9435-9440.
Bloemen K, et al. (2007) The allergic cascade: review of the most important molecules in the asthmatic lung. Immunology letters 113(1):6-18.
Cao C, et al. (2009) Abeta-specific Th2 cells provide cognitive and pathological benefits to Alzheimer's mice without infiltrating the CNS. Neurobiology of disease 34(1):63-70.
Dantzer R, O'Connor JC, Freund GG, Johnson RW, & Kelley KW (2008) From inflammation to sickness and depression: when the immune system subjugates the brain. Nature reviews. Neuroscience 9(1):46-56.
de Graaf MT, et al. (2011) Central memory CD4+ T cells dominate the normal cerebrospinal fluid. Cytometry. Part B, Clinical cytometry 80(1):43-50.
Derecki NC, et al. (2010) Regulation of learning and memory by meningeal immunity: a key role for IL-4. The Journal of experimental medicine 207(5):1067-1080.
Derecki, N.C., J.C. Cronk, Z. Lu, E. Xu, S.B. Abbott, P.G. Guyenet, and J. Kipnis. 2012. Wild-type microglia arrest pathology in a mouse model of Rett syndrome. Nature 484:105-109.
Ekdahl CT, Claasen JH, Bonde S, Kokaia Z, & Lindvall O (2003) Inflammation is detrimental for neurogenesis in adult brain. Proceedings of the National Academy of Sciences of the United States of America 100(23):13632-13637.
Emerich, D.F., S.J. Skinner, C.V. Borlongan, A.V. Vasconcellos, and C.G. Thanos. 2005. The choroid plexus in the rise, fall and repair of the brain. BioEssays : news and reviews in molecular, cellular and developmental biology 27:262-274.
Engelhardt B & Ransohoff RM (2005) The ins and outs of T-lymphocyte trafficking to the CNS: anatomical sites and molecular mechanisms. Trends in immunology 26(9):485-495.
Fish, S.M., R. Proujansky, and W.W. Reenstra. 1999. Synergistic effects of interferon gamma and tumour necrosis factor alpha on T84 cell function. Gut 45:191-198.
Freeman JD, Warren RL, Webb JR, Nelson BH, & Holt RA (2009) Profiling the T-cell receptor beta-chain repertoire by massively parallel sequencing. Genome research 19(10):1817-1824.
Harrington JF, Messier AA, Levine A, Szmydynger-Chodobska J, Chodobski A. Shedding of tumor necrosis factor type 1 receptor after experimental spinal cord injury. Journal of neurotrauma. 2005 Aug;22(8):919-28.
Hattiangady B, Rao MS, Shetty GA, & Shetty AK (2005) Brain-derived neurotrophic factor, phosphorylated cyclic AMP response element binding protein and neuropeptide Y decline as early as middle age in the dentate gyrus and CA1 and CA3 sub fields of the hippocampus. Experimental neurology 195(2):353-371.
Hauben, E., O. Butovsky, U. Nevo, E. Yoles, G. Moalem, E. Agranov, F. Mor, R. Leibowitz-Amit, E. Pevsner, S. Akselrod, M. Neeman, I.R. Cohen, and M. Schwartz. 2000. Passive or active immunization with myelin basic protein promotes recovery from spinal cord contusion. The Journal of neuroscience : the official journal of the Society for Neuroscience 20:6421-6430.
Heyer LJ, Kruglyak S, & Yooseph S (1999) Exploring expression data: identification and analysis of coexpressed genes. Genome research 9(11):1106-1115.
Hori, S., T. Nomura, and S. Sakaguchi. 2003. Control of regulatory T cell development by the transcription factor Foxp3. Science 299:1057-1061.
Jung, S., J. Aliberti, P. Graemmel, M.J. Sunshine, G.W. Kreutzberg, A. Sher, and D.R. Littman. 2000. Analysis of fractalkine receptor CX(3)CR1 function by targeted deletion and green fluorescent protein reporter gene insertion. Molecular and cellular biology 20:4106-4114.
Kesslak JP, So V, Choi J, Cotman CW, & Gomez-Pinilla F (1998) Learning upregulates brain-derived neurotrophic factor messenger ribonucleic acid: a mechanism to facilitate encoding and circuit maintenance? Behavioral neuroscience 112(4):1012-1019.
Kivisakk, P., D.J. Mahad, M.K. Callahan, C. Trebst, B. Tucky, T. Wei, L. Wu, E.S. Baekkevold, H. Lassmann, S.M. Staugaitis, J.J. Campbell, and R.M. Ransohoff. (2003). Human cerebrospinal fluid central memory CD4+ T cells: evidence for trafficking through choroid plexus and meninges via P-selectin. Proceedings of the National Academy of Sciences of the United States of America 100:8389-8394.
Korn, T., E. Bettelli, M. Oukka, and V.K. Kuchroo. 2009. IL-17 and Th17 Cells. Annual review of immunology 27:485-517.
Lalive P.H., Oliver Neuhaus, Mahdia Benkhoucha, Danielle Burger, Reinhard Hohlfeld, Scott S. Zamvil and Martin S. Weber (2011) Glatiramer Acetate in the Treatment of Multiple Sclerosis. Emerging Concepts Regarding its Mechanism of Action CNS Drugs 25 (5): 401-414)
Lees, J.R., P.T. Golumbek, J. Sim, D. Dorsey, and J.H. Russell. 2008. Regional CNS responses to IFN-gamma determine lesion localization patterns during EAE pathogenesis. The Journal of experimental medicine 205:2633-2642.
Lefranc MP, et al. (2009) IMGT, the international ImMunoGeneTics information system. Nucleic acids research 37(Database issue):D1006-1012.
Liang Zhao, Liguang Sun, Hongjun Wang, Haixia Ma, Guangwei Liu, and Yong Zhao1, 2007, Changes of CD4_CD25_Foxp3 regulatory T cells in aged Balb/c mice. Journal of Leukocyte Biology 81:1386-1394
Liu, Y., Teige, I., Birnir, B. & Issazadeh-Navikas, S. 2006. Neuron-mediated generation of regulatory T cells from encephalitogenic T cells suppresses EAE. Nature medicine 12, 518-525.
Linton PJ & Dorshkind K (2004) Age-related changes in lymphocyte development and function. Nature immunology 5(2):133-139.
Miller, L.A., and E.C. Butcher. 1998. Human airway epithelial monolayers promote selective transmigration of memory T cells: a transepithelial model of lymphocyte migration into the airways. Am J Respir Cell Mol Biol 19:892-900.
Moalem, G., R. Leibowitz-Amit, E. Yoles, F. Mor, I.R. Cohen, and M. Schwartz. (1999). Autoimmune T cells protect neurons from secondary degeneration after central nervous system axotomy. Nature medicine 5(1):49-55.
Mohrs, M., K. Shinkai, K. Mohrs, and R.M. Locksley. 2001. Analysis of type 2 immunity in vivo with a bicistronic IL-4 reporter. Immunity 15:303-311.
Monje ML, Toda H, & Palmer TD (2003) Inflammatory blockade restores adult hippocampal neurogenesis. Science 302(5651):1760-1765.
Mosley, R.L., P.H. Gordon, C.M. Hasiak, F.J. Van Wetering, H. Mitsumoto, and H.E. Gendelman. 2007. Glatiramer acetate immunization induces specific antibody and cytokine responses in ALS patients. Amyotrophic lateral sclerosis : official publication of the World Federation of Neurology Research Group on Motor Neuron Diseases 8:235-242.
Murugesan, N., Paul, D., Lemire, Y., Shrestha, B., Ge, S., and Pachter, J.S. 2012. Active induction of experimental autoimmune encephalomyelitis by MOG35-55 peptide immunization is associated with differential responses in separate compartments of the choroid plexus. Fluids Barriers CNS 9:15.
Nicolle MM, et al. (2001) Signatures of hippocampal oxidative stress in aged spatial learning-impaired rodents. Neuroscience 107(3):415-431.
O'Connor, R.A., Malpass, K.H. & Anderton, S.M. 2007. The inflamed central nervous system drives the activation and rapid proliferation of Foxp3+ regulatory T cells. Journal of immunology 179, 958-966.
Oliver A.R., Geoffrey M. Lyon and Nancy H. Ruddle. 2003. Rat and Human Myelin Oligodendrocyte Glycoproteins Induce Experimental Autoimmune Encephalomyelitis by Different Mechanisms in C57BL/6 Mice. J Immunol 2003; 171:462-468]
Olsson T, Lidman O, & Piehl F (2003) Harm or heal--divergent effects of autoimmune neuroinflammation? Trends in immunology 24(1):5-6; author reply 7-8.
Pineau, I., and S. Lacroix. 2007. Proinflammatory cytokine synthesis in the injured mouse spinal cord: multiphasic expression pattern and identification of the cell types involved. The Journal of comparative neurology 500:267-285.
Porter, J.C., and A. Hall. 2009. Epithelial ICAM-1 and ICAM-2 regulate the egression of human T cells across the bronchial epithelium. FASEB J 23:492-502.
Porter, J.C., M. Falzon, and A. Hall. 2008. Polarized localization of epithelial CXCL11 in chronic obstructive pulmonary disease and mechanisms of T cell egression. J Immunol 180:1866-1877.
Rankin JA, et al. (1996) Phenotypic and physiologic characterization of transgenic mice expressing interleukin 4 in the lung: lymphocytic and eosinophilic inflammation without airway hyperreactivity. Proceedings of the National Academy of Sciences of the United States of America 93(15):7821-7825.
Reboldi, A., C. Coisne, D. Baumjohann, F. Benvenuto, D. Bottinelli, S. Lira, A. Uccelli, A. Lanzavecchia, B. Engelhardt, and F. Sallusto. 2009. C-C chemokine receptor 6-regulated entry of TH-17 cells into the CNS through the choroid plexus is required for the initiation of EAE. Nature immunology 10:514-523.
Rink L, Cakman I, & Kirchner H (1998) Altered cytokine production in the elderly. Mechanisms of ageing and development 102(2-3):199-209.
Robins HS, et al. (2009) Comprehensive assessment of T-cell receptor beta-chain diversity in alphabeta T cells. Blood 114(19):4099-4107.
Ron-Harel N, et al. (2008) Age-dependent spatial memory loss can be partially restored by immune activation. Rejuvenation research 11(5):903-913.
Ron-Harel N & Schwartz M (2009) Immune senescence and brain aging: can rejuvenation of immunity reverse memory loss? Trends in neurosciences 32(7):367-375.
Ron-Harel, N., M. Cardon, and M. Schwartz (2011) Brain homeostasis is maintained by "danger" signals stimulating a supportive immune response within the brain's borders. Brain, behavior, and immunity 25:1036-1043.
Schwartz, M., and R. Shechter. 2010. Systemic inflammatory cells fight off neurodegenerative disease. Nature reviews. Neurology 6:405-410.
Shearer GM (1997) Thl/Th2 changes in aging. Mechanisms of ageing and development 94(1-3):1-5.
Shechter, R., A. London, C. Varol, C. Raposo, M. Cusimano, G. Yovel, A. Rolls, M. Mack, S. Pluchino, G. Martino, S. Jung, and M. Schwartz. 2009. Infiltrating blood-derived macrophages are vital cells playing an anti-inflammatory role in recovery from spinal cord injury in mice. PLoS medicine 6:e1000113.
Simard, A.R., D. Soulet, G. Gowing, J.P. Julien, and S. Rivest. 2006. Bone marrow-derived microglia play a critical role in restricting senile plaque formation in Alzheimer's disease. Neuron 49:489-502.
Smith TF & Waterman MS (1981) Identification of common molecular subsequences. Journal of molecular biology 147(1):195-197.
Stevenson NJ, et al. (2009) CCL11 blocks IL-4 and GM-CSF signaling in hematopoietic cells and hinders dendritic cell differentiation via suppressor of cytokine signaling expression. Journal of leukocyte biology 85(2):289-297.
Szmydynger-Chodobska, J., N. Strazielle, J.R. Gandy, T.H. Keefe, B.J. Zink, J.F. Ghersi-Egea, and A. Chodobski. 2012. Posttraumatic invasion of monocytes across the blood-cerebrospinal fluid barrier. J Cereb Blood Flow Metab 32:93-104.
Taguchi, M., D. Sampath, T. Koga, M. Castro, D.C. Look, S. Nakajima, and M.J. Holtzman. 1998. Patterns for RANTES secretion and intercellular adhesion molecule 1 expression mediate transepithelial T cell traffic based on analyses in vitro and in vivo. J Exp Med 187:1927-1940.
Venturi V, et al. (2011) A mechanism for TCR sharing between T cell subsets and individuals revealed by pyrosequencing. Journal of immunology 186(7):4285-4294.
Villeda SA, et al. (2011) The ageing systemic milieu negatively regulates neurogenesis and cognitive function. Nature 477(7362):90-94.
Whalley LJ, Deary IJ, Appleton CL, & Starr JM (2004) Cognitive reserve and the neurobiology of cognitive aging. Ageing research reviews 3(4):369-382.
Wolf, S.A., B. Steiner, A. Akpinarli, T. Kammertoens, C. Nassenstein, A. Braun, T. Blankenstein, and G. Kempermann. (2009). CD4-positive T lymphocytes provide a neuroimmunological link in the control of adult hippocampal neurogenesis. Journal of immunology 182(7):3979-3984.
Zhou, L., J.E. Lopes, M.M. Chong, Ivanov, II, R. Min, G.D. Victora, Y. Shen, J. Du, Y.P. Rubtsov, A.Y. Rudensky, S.F. Ziegler, and D.R. Littman. 2008. TGF-beta-induced Foxp3 inhibits T(H)17 cell differentiation by antagonizing RORgammat function. Nature 453:236-240.
Ziv, Y., N. Ron, O. Butovsky, G. Landa, E. Sudai, N. Greenberg, H. Cohen, J. Kipnis, and M. Schwartz. 2006a. Immune cells contribute to the maintenance of neurogenesis and spatial learning abilities in adulthood. Nature neuroscience 9(2):268-275.
Ziv, Y., H. Avidan, S. Pluchino, G. Martino, and M. Schwartz. (2006b). Synergy between immune cells and adult neural stem/progenitor cells promotes functional recovery from spinal cord injury. Proceedings of the National Academy of Sciences of the United States of America 103(35):13174-13179.

## Claims

1. A vaccine for use in the treatment of amyotrophic lateral sclerosis (ALS) comprising a non-encephalitogenic Th1 adjuvant as a sole active ingredient,
wherein the vaccine is to be administered to thereby activate the choroid plexus of a mammal and maintain said activation by reducing immunosuppression and establishing Th1-type immune response at the choroid plexus thus allowing either anti-inflammatory immune cells or immune cells which acquire a healing phenotype at the cerebrospinal fluid, to pass through the choroid plexus, and accumulate at a site of damage in the CNS caused by said ALS, and the non-encephalitogenic Th1 adjuvant is selected from an agonist of TLR3, 4, 5, 7, 8 and 9.

2. A vaccine for use in the treatment of ALS comprising
a non-encephalitogenic Th1 adjuvant as a sole active ingredient,
wherein the vaccine is to be administered according to a regimen to thereby confer reduction and maintenance of the level of immunosuppression in the circulation of said mammal relative to a reference and induction towards a Th1-type immune response, wherein said reduced level of immunosuppression and Th1-type immune response in the circulation indicates and ensures activation of the choroid plexus of said mammal and maintenance of said activation thus allowing either anti-inflammatory immune cells or immune cells which acquire a healing phenotype at the cerebrospinal fluid to pass through the choroid plexus and accumulate at a site of damage in the CNS caused by said ALS, and the non-encephalitogenic Th1 adjuvant is selected from an agonist of TLR3, 4, 5, 7, 8 and 9.

3. The vaccine for use according to claim 1 or 2, wherein the non-encephalitogenic Th1 adjuvant is a CpG oligodeoxynucleotide, a stabilized immune modulatory RNA (SIMRA), a double-stranded RNA (dsRNA), a single stranded RNA, a polyinosine-polycytidylic acid poly(I:C), a lipopolysaccharide, a flagellin, an imidazoquinoline, or an anti-TLR agonistic antibody.

4. The vaccine for use according to claim 2 or 3, wherein said use comprises:
(a) administering said Th1 adjuvant to said subject in need;
(b) determining said regimen by:
(i) monitoring immunosuppression and Th1/Th2 balance in said subject by measuring in a blood sample obtained from said subject, within a predetermined time-period following said administering, one or more parameters reflecting a degree of immunosuppression and the Thl/Th2 balance in the choroid plexus in said subject; and
(ii) comparing the one or more parameters measured in (b)(i) with said reference and determining whether said parameter is different from said reference; and
(c) deciding, based on the relation of said one or more parameters measured in (b)(i) to said reference, whether to repeat treatment and monitoring by repeating steps (a) and (b) or to continue monitoring by repeating only step (b).

5. The vaccine for use according to claim 4, wherein said parameter is the ratio of Treg cells to effector T cells in a blood sample obtained from said subject, said reference is the most recent ratio measured in said subject before said administering the Th1 adjuvant, and
(i) said treatment and monitoring is repeated when the ratio is substantially similar to or higher than the reference; or
(ii) said monitoring is repeated when the parameter is lower than the reference value.

6. The vaccine for use according to any one of claims 1 to 5 wherein said treating improves CNS motor and/or cognitive function.

7. The vaccine for use according to claim 6, for alleviating age-associated loss of cognitive function.

8. The vaccine for use according to claim 6 or 7, wherein said cognitive function is learning, memory or both.

9. The vaccine for use according to claim 6, wherein the CNS motor function is controlling a motor function, controlling an auditory response, controlling a visual response, maintaining balance, maintaining equilibrium, movement coordination, conduction of sensory information or controlling an autonomic function.

## Patentansprüche

1. Impfstoff zur Verwendung bei der Behandlung von amyotropher Lateralsklerose (ALS), umfassend ein nicht-enzephalitogenes Th1-Adjuvans als einzigen Wirkstoff,
wobei der Impfstoff verabreicht werden soll, um dadurch den Plexus choroideus eines Säugers zu aktivieren und die Aktivierung durch Senken der Immunsuppression und Erzeugen einer Th1-Typ-Immunantwort am Plexus choroideus aufrechtzuerhalten, wodurch ermöglicht wird, dass entweder entzündungshemmende Immunzellen oder Immunzellen, die einen heilenden Phänotyp an der zerebrospinalen Flüssigkeit erwerben, durch den Plexus choroideus durchtreten und sich an einer durch die ALS verursachten Schädigungsstelle im ZNS ansammeln, und wobei das nicht-enzephalitogene Th1-Adjuvans aus einem Agonisten von TLR3, 4, 5, 7, 8 und 9 ausgewählt ist.

2. Impfstoff zur Verwendung bei der Behandlung von ALS, umfassend ein nicht-enzephalitogenes Th1-Adjuvans als einzigen Wirkstoff,
wobei der Impfstoff gemäß einem Regime verabreicht werden soll, um dadurch Senkung und Aufrechterhaltung des Immunsuppressionsspiegels im Kreislauf des Säugers in Bezug auf eine Referenz und Einleitung einer TH1-Typ-Immunantwort zu übertragen, wobei der gesenkte Immunsuppressionsspiegel und die Th1-Typ-Immunantwort im Kreislauf Aktivierung des Plexus choroideus des Säugers und Aufrechterhaltung der Aktivierung anzeigen und sicherstellen, wodurch ermöglicht wird, dass entweder entzündungshemmende Immunzellen oder Immunzellen, die einen heilenden Phänotyp an der zerebrospinalen Flüssigkeit erwerben, durch den Plexus choroideus durchtreten und sich an einer durch die ALS verursachten Schädigungsstelle im ZNS ansammeln, und wobei das nicht-enzephalitogene Th1-Adjuvans aus einem Agonisten von TLR3, 4, 5, 7, 8 und 9 ausgewählt ist.

3. Impfstoff zur Verwendung nach Anspruch 1 oder 2, wobei das nicht-enzephalitogene Th1-Adjuvans ein CpG-Oligodeoxynucleotid, eine stabilisierte immunmodulatorische RNA (SIMRA), eine doppelsträngige RNA (dsRNA), eine einsträngige RNA, eine Polyinosinsäure:Polycytidylsäure (Poly(I:C)), ein Lipopolysaccharid, ein Flagellin, ein Imidazochinolin oder ein agonistischer anti-TLR-Antikörper ist.

4. Impfstoff zur Verwendung nach Anspruch 2 oder 3, wobei die Verwendung umfasst:
(a) Verabreichen des Th1-Adjuvans an ein Individuum mit Bedarf daran;
(b) Bestimmen des Regimes durch:
(i) Überwachen der Immunsuppression und des Th1/Th2-Gleichgewichts im Individuum durch Messen eines oder mehrerer Parameter, die einen Grad der Immunsuppression und des Th1/Th2-GIeichgewichts im Plexus choroideus im Individuum widerspiegeln, in einer dem Individuum entnommenen Blutprobe innerhalb eines vorbestimmten Zeitraums nach der Verabreichung; und
(ii) Vergleichen des einen oder der mehreren in (b)(i) gemessenen Parameter mit der Referenz und Bestimmen, ob der Parameter sich von der Referenz unterscheidet; und
(c) Entscheiden basierend auf der Beziehung des einen oder der mehreren in (b)(i) gemessenen Parameter zur Referenz, ob die Behandlung wiederholt werden soll, und Überwachen durch Wiederholen von Schritt (a) und (b) oder Fortfahren mit dem Überwachen durch Wiederholen nur von Schritt (b).

5. Impfstoff zur Verwendung nach Anspruch 4, wobei der Parameter das Verhältnis von Treg-Zellen zu Effektor-T-Zellen in einer dem Individuum entnommenen Blutprobe ist, die Referenz das letzte Verhältnis ist, das im Individuum vor dem Verabreichen des Th1-Adjuvans gemessen wurde, und
(i) die Behandlung und das Überwachen wiederholt werden, wenn das Verhältnis im Wesentlichen gleich wie oder höher als die Referenz ist; oder
(ii) das Überwachen wiederholt wird, wenn der Parameter niedriger als der Referenzwert ist.

6. Impfstoff zur Verwendung nach einem der Ansprüche 1 bis 5, wobei die Behandlung die motorische und/oder kognitive ZNS-Funktion verbessert.

7. Impfstoff zur Verwendung nach Anspruch 6 zum Lindern eines altersbedingten Verlusts der kognitiven Funktion.

8. Impfstoff zur Verwendung nach Anspruch 6 oder 7, wobei es sich bei der kognitiven Funktion um Lernen, Gedächtnis oder beides handelt.

9. Impfstoff zur Verwendung nach Anspruch 6, wobei es sich bei der motorischen ZNS-Funktion um Steuern einer motorischen Funktion, Steuern einer Gehörreaktion, Steuern einer visuellen Reaktion, Halten der Balance, Halten des Gleichgewichts, Bewegungskoordination, Leitung von sensorischen Informationen oder Steuern einer autonomen Funktion handelt.

## Revendications

1. Vaccin destiné à être utilisé dans le traitement de la sclérose latérale amyotrophique (SLA) comprenant un adjuvant Th1 non encéphalitogène en tant qu'unique ingrédient actif,
où le vaccin doit être administré pour ainsi activer le plexus choroïde d'un mammifère et maintenir ladite activation en réduisant l'immunosuppression et en établissant une réponse immunitaire de type Th1 au niveau du plexus choroïde, en permettant ainsi à des cellules immunitaires anti-inflammatoires ou à des cellules immunitaires qui acquièrent un phénotype de réparation au niveau du liquide céphalorachidien de passer à travers le plexus choroïde, et de s'accumuler au niveau d'un site de lésion dans le SNC provoqué par ladite SLA, et l'adjuvant Th1 non encéphalitogène est sélectionné parmi un agoniste de TLR3, 4, 5, 7, 8 et 9.

2. Vaccin destiné à être utilisé dans le traitement de la SLA comprenant un adjuvant Th1 non encéphalitogène en tant qu'unique ingrédient actif,
où le vaccin doit être administré selon un régime pour ainsi conférer une réduction et un maintien du niveau d'immunosuppression dans la circulation dudit mammifère par rapport à une référence et induire une réponse immunitaire de type Th1, dans lequel ledit niveau d'immunosuppression réduit et la réponse immunitaire de type Th1 dans la circulation indiquent et garantissent l'activation du plexus choroïde dudit mammifère et le maintien de ladite activation, en permettant ainsi à des cellules immunitaires anti-inflammatoires ou à des cellules immunitaires qui acquièrent une phénotype de réparation au niveau du liquide céphalorachidien de passer à travers le plexus choroïde et de s'accumuler au niveau d'un site de lésion dans le SNC provoqué par ladite SLA, et l'adjuvant Th1 non encéphalitogène est sélectionné parmi un agoniste de TLR3, 4, 5, 7, 8 et 9.

3. Vaccin destiné à être utilisé selon la revendication 1 ou 2, dans lequel l'adjuvant Th1 non encéphalitogène est un oligodésoxynucléotide CpG, un ARN immunomodulateur stabilisé (SIMRA), un ARN double brin (ARNdb), un ARN simple brin, l'acide polyinosinique-polycytidylique poly(I:C), un lipopolysaccharide, une flagelline, une imidazoquinoline, ou un anticorps agoniste anti-TLR.

4. Vaccin destiné à être utilisé selon la revendication 2 ou 3, dans lequel ladite utilisation comprend :
(a) l'administration dudit adjuvant Th1 audit sujet en ayant besoin ;
(b) la détermination dudit régime par :
(i) la surveillance de l'immunosuppression et de l'équilibre Th1/Th2 chez ledit sujet en mesurant, dans un échantillon de sang obtenu chez ledit sujet, pendant une période prédéterminée suite à ladite administration, un ou plusieurs paramètres reflétant un degré d'immunosuppression et l'équilibre Th1/Th2 dans le plexus choroïde chez ledit sujet ; et
(ii) la comparaison du un ou plusieurs paramètres mesuré(s) en (b)(i) avec ladite référence et la détermination du fait que ledit paramètre est différent ou non de ladite référence ; et
(c) la décision, basée sur la relation entre ledit un ou plusieurs paramètres mesuré(s) en (b)(i) et ladite référence, de répéter ou non le traitement et la surveillance en répétant les étapes (a) et (b) ou de poursuivre ou non la surveillance en répétant uniquement l'étape (b).

5. Vaccin destiné à être utilisé selon la revendication 4, dans lequel ledit paramètre est le rapport des cellules Treg sur les cellules T effectrices dans un échantillon de sang obtenu chez ledit sujet, ladite référence est le rapport le plus récent mesuré chez ledit sujet avant ladite administration de l'adjuvant Th1, et
(i) ledit traitement et ladite surveillance sont répétés lorsque le rapport est essentiellement similaire à ou supérieur à la référence ; ou
(ii) ladite surveillance est répétée lorsque le paramètre est inférieur à la valeur de référence.

6. Vaccin destiné à être utilisé selon l'une quelconque des revendications 1 à 5, dans lequel ledit traitement améliore la fonction motrice et/ou cognitive du SNC.

7. Vaccin destiné à être utilisé selon la revendication 6, pour soulager la perte de fonction cognitive liée à l'âge.

8. Vaccin destiné à être utilisé selon la revendication 6 ou 7, dans lequel ladite fonction cognitive est l'apprentissage, la mémoire, ou les deux.

9. Vaccin destiné à être utilisé selon la revendication 6, dans lequel la fonction motrice du SNC est le contrôle d'une fonction motrice, le contrôle d'une réponse auditive, le contrôle d'une réponse visuelle, le maintien de la stabilité, le maintien de l'équilibre, la coordination des mouvements, la conduction des informations sensorielles ou le contrôle d'une fonction autonome.


### SEQUENCE LISTING

<110> Yeda Research and Development Co. Ltd
   Schwartz-Eisenbach, Michal
   Friedman, Nir
   Cahalon , Liora
   Deczkowska, Aleksandra
   Rosenzweig, Neta
   Baruch, Kuti
   Kunis, Gilad
<120> INDIVIDUALIZED IMMUNOMODULATION THERAPY FOINDIVIDUALIZED IMMUNOMODULATION THERAPY FOR NEURODEGENERATIVE DISORDERS, CNS INJURY AND AGE-RELATED DEMENTIA
<130> YEDA-120 PCT
<150> 61/699,071
   <151> 2012-09-10
<150> 61/779,440
   <151> 2013-03-13
<160> 67
<170> PatentIn version 3.5
<210> 1
   <211> 21
   <212> PRT
   <213> Artificial Sequence
<220>
   <223> Synthetic peptide
<400> 1 
Met Glu Val Gly Trp Tyr Arg Ser Pro Phe Ser Arg Val Val His Leu
1               5                   10                  15

Tyr Arg Asn Gly Lys
            20
<210> 2
   <211> 11
   <212> PRT
   <213> Artificial Sequence
<220>
   <223> synthetic peptide
<400> 2 
Lys Leu Lys Leu Leu Leu Leu Leu Lys Leu Lys
1               5                   10
<210> 3
   <211> 171
   <212> PRT
   <213> Homo sapiens
<400> 3 
Met Ala Ser Gln Lys Arg Pro Ser Gln Arg His Gly Ser Lys Tyr Leu
1               5                   10                  15

Ala Thr Ala Ser Thr Met Asp His Ala Arg His Gly Phe Leu Pro Arg
            20                  25                  30

His Arg Asp Thr Gly Ile Leu Asp Ser Ile Gly Arg Phe Phe Gly Gly
        35                  40                  45

Asp Arg Gly Ala Pro Lys Arg Gly Ser Gly Lys Asp Ser His His Pro
    50                  55                  60

Ala Arg Thr Ala His Tyr Gly Ser Leu Pro Gln Lys Ser His Gly Arg
65                  70                  75                  80

Thr Gln Asp Glu Asn Pro Val Val His Phe Phe Lys Asn Ile Val Thr
                85                  90                  95

Pro Arg Thr Pro Pro Pro Ser Gln Gly Lys Gly Arg Gly Leu Ser Leu
            100                 105                 110

Ser Arg Phe Ser Trp Gly Ala Glu Gly Gln Arg Pro Gly Phe Gly Tyr
        115                 120                 125

Gly Gly Arg Ala Ser Asp Tyr Lys Ser Ala His Lys Gly Phe Lys Gly
    130                 135                 140

Val Asp Ala Gln Gly Thr Leu Ser Lys Ile Phe Lys Leu Gly Gly Arg
145                 150                 155                 160

Asp Ser Arg Ser Gly Ser Pro Met Ala Arg Arg
                165                 170
<210> 4
   <211> 274
   <212> PRT
   <213> Homo sapiens
<400> 4 
Met Gly Leu Leu Glu Cys Cys Ala Arg Cys Leu Val Gly Ala Pro Phe
1               5                   10                  15

Ala Ser Leu Val Ala Thr Gly Leu Cys Phe Phe Gly Val Ala Leu Phe
            20                  25                  30

Cys Gly Cys Gly His Glu Ala Leu Thr Gly Thr Glu Lys Leu Ile Glu
 
        35                  40                  45

Thr Tyr Phe Ser Lys Asn Tyr Gln Asp Tyr Glu Tyr Leu Ile Asn Val
    50                  55                  60

Ile His Ala Phe Gln Tyr Val Ile Tyr Gly Thr Ala Ser Phe Phe Phe
65                  70                  75                  80

Leu Tyr Gly Ala Leu Leu Leu Ala Glu Gly Phe Tyr Thr Thr Gly Ala
                85                  90                  95

Val Arg Gln Ile Phe Gly Asp Tyr Lys Thr Thr Ile Cys Gly Lys Gly
            100                 105                 110

Leu Ser Ala Thr Val Thr Gly Gly Gln Lys Gly Arg Gly Ser Arg Gly
        115                 120                 125

Gln His Gln Ala His Ser Leu Glu Arg Val Cys His Cys Leu Gly Lys
    130                 135                 140

Trp Leu Gly His Pro Asp Lys Ile Thr Tyr Ala Leu Thr Val Val Trp
145                 150                 155                 160

Leu Leu Val Phe Ala Cys Ser Ala Val Pro Val Tyr Ile Tyr Phe Asn
                165                 170                 175

Thr Trp Thr Thr Cys Gln Ser Ile Ala Phe Pro Ser Lys Thr Ser Ala
            180                 185                 190

Ser Ile Gly Ser Leu Cys Ala Asp Ala Arg Met Tyr Gly Val Leu Pro
        195                 200                 205

Trp Asn Ala Phe Pro Gly Lys Val Cys Gly Ser Asn Leu Leu Ser Ile
    210                 215                 220

Cys Lys Thr Ala Glu Phe Gln Met Thr Phe His Leu Phe Ile Ala Ala
225                 230                 235                 240

Phe Val Gly Ala Ala Ala Thr Leu Val Ser Leu Leu Thr Phe Met Ile
                245                 250                 255

Ala Ala Thr Tyr Asn Phe Ala Val Leu Lys Leu Met Gly Arg Gly Thr
            260                 265                 270

Lys Phe
<210> 5
   <211> 247
   <212> PRT
   <213> Homo sapiens
<400> 5 
Met Ala Ser Leu Ser Arg Pro Ser Leu Pro Ser Cys Leu Cys Ser Phe
1               5                   10                  15

Leu Leu Leu Leu Leu Leu Gln Val Ser Ser Ser Tyr Ala Gly Gln Phe
            20                  25                  30

Arg Val Ile Gly Pro Arg His Pro Ile Arg Ala Leu Val Gly Asp Glu
        35                  40                  45

Val Glu Leu Pro Cys Arg Ile Ser Pro Gly Lys Asn Ala Thr Gly Met
    50                  55                  60

Glu Val Gly Trp Tyr Arg Pro Pro Phe Ser Arg Val Val His Leu Tyr
65                  70                  75                  80

Arg Asn Gly Lys Asp Gln Asp Gly Asp Gln Ala Pro Glu Tyr Arg Gly
                85                  90                  95

Arg Thr Glu Leu Leu Lys Asp Ala Ile Gly Glu Gly Lys Val Thr Leu
            100                 105                 110

Arg Ile Arg Asn Val Arg Phe Ser Asp Glu Gly Gly Phe Thr Cys Phe
        115                 120                 125

Phe Arg Asp His Ser Tyr Gln Glu Glu Ala Ala Met Glu Leu Lys Val
    130                 135                 140

Glu Asp Pro Phe Tyr Trp Val Ser Pro Gly Val Leu Val Leu Leu Ala
145                 150                 155                 160

Val Leu Pro Val Leu Leu Leu Gln Ile Thr Leu Gly Leu Val Phe Leu
                165                 170                 175

Cys Leu Gln Tyr Arg Leu Arg Gly Lys Leu Arg Ala Glu Ile Glu Asn
            180                 185                 190

Leu His Arg Thr Phe Asp Pro His Phe Leu Arg Val Pro Cys Trp Lys
        195                 200                 205

Ile Thr Leu Phe Val Ile Val Pro Val Leu Gly Pro Leu Val Ala Leu
    210                 215                 220
 
Ile Ile Cys Tyr Asn Trp Leu His Arg Arg Leu Ala Gly Gln Phe Leu
225                 230                 235                 240

Glu Glu Leu Arg Asn Pro Phe
                245
<210> 6
   <211> 19
   <212> DNA
   <213> Artificial Sequence
<220>
   <223> synthetic
<400> 6
   aagacagggc tcctttcag 19
<210> 7
   <211> 21
   <212> DNA
   <213> Artificial Sequence
<220>
   <223> synthetic
<400> 7
   tgttcacagt actcttcacc t 21
<210> 8
   <211> 20
   <212> DNA
   <213> Artificial Sequence
<220>
   <223> synthetic
<400> 8
   cctgcatctg ttggggagac 20
<210> 9
   <211> 20
   <212> DNA
   <213> Artificial Sequence
<220>
   <223> synthetic
<400> 9
   gccttgtccg tggacgttta 20
<210> 10
   <211> 20
   <212> DNA
   <213> Artificial Sequence
<220>
   <223> synthetic
<400> 10
   gcgcccatga aagaagtaaa 20
<210> 11
   <211> 20
   <212> DNA
   <213> Artificial Sequence
<220>
   <223> synthetic
<400> 11
   tcgtcagacc tctcgaacct 20
<210> 12
   <211> 18
   <212> DNA
   <213> Artificial Sequence
<220>
   <223> synthetic
<400> 12
   catccacgtg ttggctca 18
<210> 13
   <211> 23
   <212> DNA
   <213> Artificial Sequence
<220>
   <223> synthetic
<400> 13
   gatcatcttg ctggtgaatg agt 23
<210> 14
   <211> 24
   <212> DNA
   <213> Artificial Sequence
<220>
   <223> synthetic
<400> 14
   gtgctccaat cttgcagtcg tgtt 24
<210> 15
   <211> 24
   <212> DNA
   <213> Artificial Sequence
<220>
   <223> synthetic
<400> 15
   acttcttctc tgggttggca caca 24
<210> 16
   <211> 21
   <212> DNA
   <213> Artificial Sequence
<220>
   <223> synthetic
<400> 16
   catgaccagt aagaagatcc c 21
<210> 17
   <211> 22
   <212> DNA
   <213> Artificial Sequence
<220>
   <223> synthetic
<400> 17
   cttgaagact atggctttca gg 22
<210> 18
   <211> 20
   <212> DNA
   <213> Artificial Sequence
<220>
   <223> synthetic
<400> 18
   tgctatcatc tttcacacga 20
<210> 19
   <211> 22
   <212> DNA
   <213> Artificial Sequence
<220>
   <223> synthetic
<400> 19
   catcttcttg actcttaggc tg 22
<210> 20
   <211> 20
   <212> DNA
   <213> Artificial Sequence
<220>
   <223> synthetic
<400> 20
   ctctgatgca ggtccctatg 20
<210> 21
   <211> 23
   <212> DNA
   <213> Artificial Sequence
<220>
   <223> synthetic
<400> 21
   tttgaggtcc agagaagaac tcc 23
<210> 22
   <211> 20
   <212> DNA
   <213> Artificial Sequence
<220>
   <223> synthetic
<400> 22
   gagttcgagg aaccctagtg 20
<210> 23
   <211> 21
   <212> DNA
   <213> Artificial Sequence
<220>
   <223> synthetic
<400> 23
   aactgtttga ggtctttgag g 21
<210> 24
   <211> 21
   <212> DNA
   <213> Artificial Sequence
<220>
   <223> synthetic
<400> 24
   aactgcatcc atatcgatga c 21
<210> 25
   <211> 19
   <212> DNA
   <213> Artificial Sequence
<220>
   <223> synthetic
<400> 25
   gtggcaatga tctcaacac 19
<210> 26
   <211> 24
   <212> DNA
   <213> Artificial Sequence
<220>
   <223> synthetic
<400> 26
   cttctgtaat ttacccgagt aacg 24
<210> 27
   <211> 22
   <212> DNA
   <213> Artificial Sequence
<220>
   <223> synthetic
<400> 27
   ttctattgcc tgcattatga gg 22
<210> 28
   <211> 24
   <212> DNA
   <213> Artificial Sequence
<220>
   <223> synthetic
<400> 28
   atgtgcgaca agatgacctc acga 24
<210> 29
   <211> 24
   <212> DNA
   <213> Artificial Sequence
<220>
   <223> synthetic
<400> 29
   tttctccttc gggtcagcac agaa 24
<210> 30
   <211> 22
   <212> DNA
   <213> Artificial Sequence
<220>
   <223> synthetic
<400> 30
   aatgtgtccg tcgtggatct ga 22
<210> 31
   <211> 22
   <212> DNA
   <213> Artificial Sequence
<220>
   <223> synthetic
<400> 31
   gatgcctgct tcaccacctt ct 22
<210> 32
   <211> 19
   <212> DNA
   <213> Artificial Sequence
<220>
   <223> synthetic
<400> 32
   accgtgacta ttccttcca 19
<210> 33
   <211> 19
   <212> DNA
   <213> Artificial Sequence
<220>
   <223> synthetic
<400> 33
   caggttccca gtgtttcag 19
<210> 34
   <211> 24
   <212> DNA
   <213> Artificial Sequence
<220>
   <223> synthetic
<400> 34
   agatgacacc tgaggtcaag caca 24
<210> 35
   <211> 24
   <212> DNA
   <213> Artificial Sequence
<220>
   <223> synthetic
<400> 35
   gcagctcctc aaacagctca atgt 24
<210> 36
   <211> 24
   <212> DNA
   <213> Artificial Sequence
<220>
   <223> synthetic
<400> 36
   agatcacatt cacggtgctg gcta 24
<210> 37
   <211> 24
   <212> DNA
   <213> Artificial Sequence
<220>
   <223> synthetic
<400> 37
   agctttggga tggtagctgg aaga 24
<210> 38
   <211> 26
   <212> DNA
   <213> Artificial Sequence
<220>
   <223> synthetic
<400> 38
   ctggaggtga tgagtgtagc tctggc 26
<210> 39
   <211> 26
   <212> DNA
   <213> Artificial Sequence
<220>
   <223> synthetic
<400> 39
   gagtgacgag ccaacacaga caggtc 26
<210> 40
   <211> 24
   <212> DNA
   <213> Artificial Sequence
<220>
   <223> synthetic
<400> 40
   tgagctggtg gatgctcttc agtt 24
<210> 41
   <211> 24
   <212> DNA
   <213> Artificial Sequence
<220>
   <223> synthetic
<400> 41
   tcatccacaa tgcctgtctg aggt 24
<210> 42
   <211> 18
   <212> DNA
   <213> Artificial Sequence
<220>
   <223> synthetic
<400> 42
   tcccacaccc attcacag 18
<210> 43
   <211> 21
   <212> DNA
   <213> Artificial Sequence
<220>
   <223> synthetic
<400> 43
   aggtccaaca gtaacattct c 21
<210> 44
   <211> 21
   <212> DNA
   <213> Artificial Sequence
<220>
   <223> synthetic
<400> 44
   ccaaaagatg aagggctgct t 21
<210> 45
   <211> 20
   <212> DNA
   <213> Artificial Sequence
<220>
   <223> synthetic
<400> 45
   tgctgctgcg agatttgaag 20
<210> 46
   <211> 23
   <212> DNA
   <213> Artificial Sequence
<220>
   <223> synthetic
<400> 46
   tgcaagagac ttccatccag ttg 23
<210> 47
   <211> 24
   <212> DNA
   <213> Artificial Sequence
<220>
   <223> synthetic
<400> 47
   taagcctccg acttgtgaag tggt 24
<210> 48
   <211> 22
   <212> DNA
   <213> Artificial Sequence
<220>
   <223> synthetic
<400> 48
   gccaaaccaa caactttatc tc 22
<210> 49
   <211> 23
   <212> DNA
   <213> Artificial Sequence
<220>
   <223> synthetic
<400> 49
   gttcgctcaa tagtcttgtt ctc 23
<210> 50
   <211> 24
   <212> DNA
   <213> Artificial Sequence
<220>
   <223> synthetic
<400> 50
   agcatacagg tcctggcatc ttgt 24
<210> 51
   <211> 24
   <212> DNA
   <213> Artificial Sequence
<220>
   <223> synthetic
<400> 51
   caaagaccac atgcttgcca tcca 24
<210> 52
   <211> 18
   <212> DNA
   <213> Artificial Sequence
<220>
   <223> synthetic
<400> 52
   catccacgtg ttggctca 18
<210> 53
   <211> 23
   <212> DNA
   <213> Artificial Sequence
<220>
   <223> synthetic
<400> 53
   gatcatcttg ctggtgaatg agt 23
<210> 54
   <211> 20
   <212> DNA
   <213> Artificial Sequence
<220>
   <223> synthetic
<400> 54
   ccacatgatt gggaatggac 20
<210> 55
   <211> 20
   <212> DNA
   <213> Artificial Sequence
<220>
   <223> synthetic
<400> 55
   gtagcaaaca ggatcatcca 20
<210> 56
   <211> 24
   <212> DNA
   <213> Artificial Sequence
<220>
   <223> synthetic
<400> 56
   agcactccgt gaagatcctt gtgt 24
<210> 57
   <211> 24
   <212> DNA
   <213> Artificial Sequence
<220>
   <223> synthetic
<400> 57
   tagcagggca aaggagagac tgtt 24
<210> 58
   <211> 23
   <212> DNA
   <213> Artificial Sequence
<220>
   <223> synthetic
<400> 58
   taccatgcca acttctgtct ggg 23
<210> 59
   <211> 23
   <212> DNA
   <213> Artificial Sequence
<220>
   <223> synthetic
<400> 59
   tgtgttggtt gtagagggca agg 23
<210> 60
   <211> 24
   <212> DNA
   <213> Artificial Sequence
<220>
   <223> synthetic
<400> 60
   aattgctgcc ttcgccctct ttac 24
<210> 61
   <211> 24
   <212> DNA
   <213> Artificial Sequence
<220>
   <223> synthetic
<400> 61
   tgtacaggct gaggactttg gtgt 24
<210> 62
   <211> 19
   <212> DNA
   <213> Artificial Sequence
<220>
   <223> synthetic
<400> 62
   acaaggctgc cccgactat 19
<210> 63
   <211> 23
   <212> DNA
   <213> Artificial Sequence
<220>
   <223> synthetic
<400> 63
   ctcctggtat gaagtggcaa atc 23
<210> 64
   <211> 22
   <212> DNA
   <213> Artificial Sequence
<220>
   <223> synthetic
<400> 64
   cttcatcagt ttaatgtgcc ga 22
<210> 65
   <211> 20
   <212> DNA
   <213> Artificial Sequence
<220>
   <223> synthetic
<400> 65
   agccttctcc tctttgacag 20
<210> 66
   <211> 24
   <212> DNA
   <213> Artificial Sequence
<220>
   <223> synthetic
<400> 66
   tgtgaaggga ttaacgaggc tgga 24
<210> 67
   <211> 24
   <212> DNA
   <213> Artificial Sequence
<220>
   <223> synthetic
<400> 67
   ccatgtttcg ggcacatttc caca 24